# EUROPEAN PATENT APPLICATION

(11) **EP 2 116 169 A1**
(43) Date of publication of application: **11.11.2009**
(21) Application number: 08711211.6
(22) Date of filing: 13.02.2008
(51) Int. Cl.: A61B 1/00, A61B 1/04, A61B 5/07

(54) **OPERATING DEVICE, MONITOR DEVICE, AND CAPSULE GUIDING SYSTEM**

(30) Priority: 14.02.2007 JP 2007033844; 31.08.2007 JP 2007226946
(71) Applicant: Olympus Medical Systems Corp., Tokyo 151-0072 (JP)
(72) Inventor: UCHIYAMA, Akio, Tokyo 151-0072 (JP); KIMURA, Atsushi, Tokyo 151-0072 (JP); CHIBA, Atsushi, Tokyo 151-0072 (JP); SATO, Ryoji, Tokyo 151-0072 (JP); KAWANO, Hironao, Tokyo 151-0072 (JP)
(74) Representative: von Hellfeld, Axel
(86) International application number: PCT/JP2008/052353
(87) International publication number: WO 2008/099851

(57) **Abstract**

An object of the present invention is to facilitate at least 3 degrees-of-freedom motion of a capsule medical device inserted into a subject. An operating device (5) according to the present invention operates a capsule endoscope (2) with 6 degrees-of-freedom motion by using a magnetic field generator (3) with respect to the capsule endoscope (2) inserted into the subject. The operating device (5) comprises an operating unit (30) including a fixed unit (31) and a movable unit (32), and a force sensor (35) incorporated in the operating unit (30). The operating unit (30) has a three-dimensional shape substantially identical to the capsule endoscope (2) and is a holdable size. The force sensor detects force information of the movable unit (32) when the movable unit (32) of the operating unit (30) is operated once or continuously. The force information detected by the force sensor (35) is output as instruction information for instructing 6 degrees-of-freedom motion of the capsule endoscope (2).

## Description

### TECHNICAL FIELD

The present invention relates to an operating device that operates magnetic guidance for guiding a capsule medical device inserted into a subject such as a patient by a magnetic force, and to a monitor device and a capsule guiding system.

### BACKGROUND ART

Conventionally, there are capsule medical devices that can be inserted into digestive organs of a subject such as a patient. A capsule medical device is swallowed from a mouth of a subject, acquires in-vivo information such as in-vivo images of the subject while moving in digestive organs with peristaltic movements or the like, and wirelessly transmits acquired in-vivo information to a receiver outside the subject. The capsule medical device sequentially acquires the in-vivo information of the subject after it is inserted into the digestive organs of the subject until it is naturally discharged therefrom.

Further, in recent years, there has been proposed a system that magnetically guides a capsule medical device inserted into a subject (see Patent Documents 1 and 2). For example, in a medical apparatus guiding system disclosed in Patent Document 1, a capsule medical device with a spiral protrusion provided on an outer peripheral surface of a capsule casing having a built-in magnet radially magnetized is inserted into digestive organs of a subject, and the capsule medical device is guided to a desired position inside the subject by applying a rotating magnetic field generated by a rotating magnetic field generator to the capsule medical device in the subject. Meanwhile, in a system disclosed in Patent Document 2, a capsule medical device (that is, an in-vivo robot) including a specimen collecting tool and a magnet inside an elliptic casing is inserted into a subject, and the in-vivo robot is guided to a desired position inside the subject by applying a three-dimensional gradient field generated by a magnetism generator to the in-vivo robot in the subject.

Patent Document 1: Japanese Patent Application Laid-open No. 2004-255174
Patent Document 2: Japanese Patent Application Laid-open No. 2003-111720

### DISCLOSURE OF INVENTION

### PROBLEM TO BE SOLVED BY THE INVENTION

In a capsule guiding system that magnetically guides a capsule medical device to a desired position in a subject, in recent years, it has been desired that not only a forward and backward motion that moves the capsule medical device along digestive organs but also at least three motions of a direction changing motion that changes a direction of the capsule medical device vertically and horizontally, a rotary motion that rotates the capsule medical device centering on a longitudinal axis of the capsule medical device, and a shifting motion that translates the capsule medical device can be controlled by a magnetic field. That is, when a three-axis (XYZ) rectangular coordinates system is defined with respect to such a capsule medical device, it is desired that at least three motions (that is, at least 3 degrees-of-freedom motion) of a displacing motion in a positive or negative direction of an X-axis, a displacing motion in a positive or negative direction of a Y-axis, a displacing motion in a positive or negative direction of a Z-axis, a rotary motion around the X-axis, a rotary motion around the Y-axis, and a rotary motion around the Z-axis (hereinafter, collectively "6 degrees-of-freedom motion") can be controlled by the magnetic field.

However, in the conventional techniques described above, a magnetic field strength and a direction of the magnetic field need to be operated by using a joy stick and an input button, and further a foot pedal or the like to control at least 3 degrees-of-freedom motion of the capsule medical device inserted into the subject by the magnetic field. Therefore, it is difficult to provide at least 3 degrees-of-freedom motion of the capsule medical device without skilled handling techniques.

The present invention has been achieved in view of the above circumstances, and an object of the present invention is to provide an operating device that can easily provide at least 3 degrees-of-freedom motion to a capsule medical device inserted into a subject, a monitor device, and a capsule guiding system.

### MEANS FOR SOLVING PROBLEM

To solve the above problems and achieve the object, an operating device according to the present invention uses a magnetic field generator with respect to a capsule medical device inserted into a subject to for operating the capsule medical device with at least 3 degrees-of-freedom motion. The operating device comprises a casing having directionality; and a detecting unit that detects respective physical values of at least 3 degrees-of-freedom motion of an entirety or a part of the casing, wherein one operation or continuous operations to the entirety or a part of the casing provides the at least 3 degrees-of-freedom motion to the capsule medical device.

In the operating device according to the present invention, the casing has a three-dimensional shape having the directionality.

In the operating device according to the present invention, the casing has a three-dimensional shape substantially identical to the capsule medical device and is a holdable size.

In the operating device according to the present invention, the casing comprises an axis display unit that indicates a specific axial direction of the capsule medical device.

In the operating device according to the present invention, the casing comprises a movable unit that receives one operation or continuous operations of the at least 3 degrees-of-freedom motion; and a fixed unit that supports the movable unit so that at least 3 degrees-of-freedom motion can be performed, wherein the detecting unit comprises a force sensor that is encapsulated in the detecting unit and detects respective pieces of force information of the at least 3 degrees-of-freedom motion of the movable unit.

In the operating device according to the present invention, the detecting unit further comprises a rotation-amount detecting device that detects an amount of rotation around an axis of the at least 3 degrees-of-freedom motion of the movable unit.

The operating device according to the present invention further comprises a supporting unit that supports the casing so that at least 3 degrees-of-freedom motion can be performed, wherein the detecting unit comprises a plurality of rotation-amount detecting devices that detect respective amounts of rotation around three axes orthogonal to each other in the at least 3 degrees-of-freedom motion of the casing; and a plurality of displacement-amount detecting devices that detect respective displacement amounts in three axial directions orthogonal to each other in the at least 3 degrees-of-freedom motion of the casing.

The operating device according to the present invention further comprises a supporting unit that supports the casing so that at least 3 degrees-of-freedom motion can be performed, wherein the detecting unit comprises a plurality of rotation-amount detecting devices that detect respective amounts of rotation around three axes orthogonal to each other in the at least 3 degrees-of-freedom motion of the casing; and a force sensor that is encapsulated in the supporting unit, and detect respective pieces of force information in three axial directions orthogonal to each other in the at least 3 degrees-of-freedom motion of the casing.

The operating device according to the present invention further comprises a magnetic-field generating stage that generates a magnetic field in a space where one operation or continuous operations with respect to an entire casing is performed; and a plurality of sense coils that are fixedly arranged inside the casing, and detect the magnetic field generated by the magnetic-field generating stage, wherein the detecting unit detects respective operating amounts of the at least 3 degrees-of-freedom motion of the casing based on a magnetic field detection result of the sense coils.

The operating device according to the present invention further comprises an acceleration sensor that is arranged in the casing and detects an acceleration of the casing generated by one operation or continuous operations with respect to the entire casing, wherein the detecting unit detects respective operating amounts of the at least 3 degrees-of-freedom motion of the casing based on an acceleration detection result of the acceleration sensor.

The operating device according to the present invention further comprises a transmitting unit that wirelessly transmits the acceleration detection result of the acceleration sensor to outside of the casing; and a receiving unit that receives the acceleration detection result of the acceleration sensor wirelessly transmitted from the transmitting unit, wherein the detecting device detects the respective operating amounts of the at least 3 degrees-of-freedom motion of the casing based on the acceleration detection result of the acceleration sensor acquired via the receiving unit.

The operating device according to the present invention further comprises a switching unit for enabling or disabling a detecting process performed by the detecting unit for detecting respective physical values of the at least 3 degrees-of-freedom motion of the casing.

The operating device according to the present invention further comprises an input unit that inputs instruction information for instructing to hold a detection result of the detecting unit, wherein the detecting unit holds the respective physical values of the at least 3 degrees-of-freedom motion of the casing based on the instruction information input by the input unit.

An operating device according to the present invention uses a magnetic field generator with respect to a capsule medical device inserted into a subject to operate the capsule medical device. The operating device comprises a casing having an axis display unit that indicates a specific axial direction of the capsule medical device; and a detecting unit that detects each physical value of at least 3 degrees-of-freedom motion provided for the casing, wherein directions of the respective physical values detected by the detecting unit match respective axial directions of a coordinate system set with respect to any one of the capsule medical device, the magnetic field generator, or a bed for placing the subject thereon.

In the operating device according to the present invention, the casing has a three-dimensional shape having directionality.

In the operating device according to the present invention, the casing is substantially identical to the capsule medical device.

In the operating device according to the present invention, the specific axial direction is a longitudinal axis direction of the capsule medical device.

In the operating device according to the present invention, the specific axial direction is an imaging direction of the capsule medical device.

In the operating device according to the present invention, the at least 3 degrees-of-freedom motion is 6 degrees-of-freedom motion.

A capsule guiding system according to the present invention comprises a capsule medical device inserted into a subject; a magnetic field generator that guides the capsule medical device by applying a magnetic field to the capsule medical device; an operating device by which an operator inputs a physical value; and a control device that controls the magnetic field generator according to the physical value, wherein the operating device comprises a casing held by the operator to input at least 3 degrees-of-freedom physical value; and a detecting unit that detects the at least 3 degrees-of-freedom physical value input to the casing by the operator.

In the capsule guiding system according to the present invention, the control device controls the magnetic field generator based on the physical value so that a posture of the capsule medical device is changed.

In the capsule guiding system according to the present invention, the control device controls the magnetic field generator based on the physical value so that a position of the capsule medical device is changed.

In the capsule guiding system according to the present invention, the physical value indicates an amount of change in a position or posture of the casing with respect to the operating device, and the detecting unit is a change-amount detecting device that detects the amount of change.

In the capsule guiding system according to the present invention, the operating device comprises the casing, which is not connected with the operating device, and the change-amount detecting device detects a position or posture of the casing with respect to the operating device.

In the capsule guiding system according to the present invention, the change-amount detecting device that detects the amount of change is provided in the casing, the casing comprises a transmitting unit that wirelessly transmits the amount of change to the operating device, and the operating device comprises a receiving unit that receives the amount of change transmitted by the transmitting unit.

In the capsule guiding system according to the present invention, the operating device comprises the casing connected to the operating device only by a flexible cable, the cable electrically couples the operating device and the casing with each other, and the change-amount detecting device detects a position or posture of the casing.

In the capsule guiding system according to the present invention, the change-amount detecting unit comprises a magnetic field generator for detecting the amount of change, provided in the casing and generates the magnetic field for detecting a position or posture in the operating device; and a plurality of magnetic-field detection sensors that are provided outside of the casing and detect the magnetic field for detecting the position or posture, and wherein the change-amount detecting unit detects the position or posture of the casing based on the magnetic field detected by the magnetic-field detection sensors.

In the capsule guiding system according to the present invention, the change-amount detecting unit comprises an acceleration sensor provided in the casing, and the change-amount detecting unit detects the amount of change based on the acceleration detected by the acceleration sensor.

In the capsule guiding system according to the present invention, the operating device comprises a switching unit that switches whether the control device controls the magnetic field generator based on the amount of change of the casing.

In the capsule guiding system according to the present invention, the switching unit switches whether to perform specific degree-of-freedom control.

In the capsule guiding system according to the present invention, the physical value indicates a force loaded on the casing, and the detecting unit is a force sensor that detects the loaded force.

In the capsule guiding system according to the present invention, the detecting unit detects 6 degrees-of-freedom physical value input by an operator, and the control device controls the magnetic field generator based on the detected physical value so that 3 degrees-of-freedom position and 3 degrees-of-freedom posture of the capsule medical device are controlled.

In the capsule guiding system according to the present invention, the detecting unit detects 5 degrees-of-freedom physical value input by an operator, and the control device controls the magnetic field generator based on the detected physical value so that 3 degrees-of-freedom position of the capsule medical device and 2 degrees-of-freedom posture of the capsule medical device excluding a rotation around a longitudinal axis of the capsule medical device are controlled.

In the capsule guiding system according to the present invention, the control device associates a coordinate system of the casing with a coordinate system of the capsule medical device, and controls the magnetic field generator based on the physical value input to the casing to control a position or posture of the capsule medical device.

In the capsule guiding system according to the present invention, the casing is a three-dimensional shape having directionality.

In the capsule guiding system according to the present invention, the three-dimensional shape is substantially identical to the capsule medical device.

In the capsule guiding system according to the present invention, the three-dimensional shape has a display unit that displays a direction matched with a specific direction of the capsule medical device on the three-dimensional shape.

In the capsule guiding system according to the present invention, the control device associates a coordinate system of the operating device with a coordinate system of the magnetic field generator, and controls the magnetic field generator based on the physical value input to the casing to control a position or posture of the capsule medical device.

The capsule guiding system according to the present invention comprises a movable unit that changes the position or posture of the casing.

In the capsule guiding system according to the present invention, the operating device comprises a driving unit that drives the movable unit; and a position and posture detecting unit that detects a position or posture of the capsule medical device, and the driving unit controls a position or posture of the casing.

In the capsule guiding system according to the present invention, a drive-control switching unit that switches whether the operating unit controls the position or posture of the casing is provided in the operating unit.

In the capsule guiding system according to the present invention, a holding unit that holds a position of the movable unit is provided in the operating unit.

In the capsule guiding system according to the present invention, the casing is a three-dimensional shape having directionality.

A capsule guiding system according to the present invention magnetically guides a capsule medical device inserted into a subject, and comprises any one of the operating devices according to the present invention; a magnetic field generator that generates a magnetic field with respect to the capsule medical device; and a control device that generates the magnetic field for causing the capsule medical device to perform desired at least 3 degrees-of-freedom motion, based on respective physical values of at least 3 degrees-of-freedom motion input by the operating device.

The capsule guiding system according to the present invention further comprises a monitor device that displays a current position of the capsule medical device in the subject.

In the capsule guiding system according to the present invention, the monitor device displays the current position of the capsule medical device with reference to a three-axis rectangular coordinate system defined with respect to the capsule medical device.

In the capsule guiding system according to the present invention, the monitor device displays an image of the subject superposed on an image of the capsule medical device, and updates the image of the subject while fixing the image of the capsule medical device.

In the capsule guiding system according to the present invention, the monitor device further displays a predicted posture taken by the capsule medical device that performs at least 3 degrees-of-freedom motion after a predetermined time.

In the capsule guiding system according to the present invention, the monitor device provides a vector display of a force generated in the capsule medical device that performs at least 3 degrees-of-freedom motion.

In the capsule guiding system according to the present invention, the monitor device displays the current position of the capsule medical device with reference to a three-axis rectangular coordinate system defined with respect to the magnetic field generator.

In the capsule guiding system according to the present invention, the monitor device further displays the current posture of the capsule medical device in the subject.

The capsule guiding system according to the present invention further comprises a position and posture detecting device that detects a current position and a current posture of the capsule medical device in the subject, wherein the control device displays the current position and the current posture of the capsule medical device detected by the position and posture detecting device on the monitor device.

In the capsule guiding system according to the present invention, the monitor device displays an image of the capsule medical device on a substantially central part of a display screen.

In the capsule guiding system according to the present invention, the monitor device displays the image of the subject added with an image of a digestive tract in which the capsule medical device moves.

In the capsule guiding system according to the present invention, the force generated in the capsule medical device is a driving force and a turning force of the capsule medical device, and the monitor device provides a vector display of the driving force and the turning force of the capsule medical device.

In the capsule guiding system according to the present invention, the monitor device displays the predicted posture, which is prediction information of a rotational position of the capsule medical device, which changes when the capsule medical device performs a rotary motion.

In the capsule guiding system according to the present invention, the capsule medical device comprises a magnet that follows a magnetic field generated by the magnetic field generator to contribute to a motion of the capsule medical device, and the monitor device displays a polar direction of the magnet as the prediction information of the rotational position of the capsule medical device.

In the capsule guiding system according to the present invention, the monitor device displays an image of the subject in a state with a relative direction with respect to a display screen being fixed, and displays an image of the capsule medical device so that a display position of the image of the capsule medical device in the displayed image of the subject match a current position of the capsule medical device.

In the capsule guiding system according to the present invention, the monitor device displays an image of the subject added with an image of a digestive tract in which the capsule medical device moves, and displays a current position of the capsule medical device in the digestive tract.

In the capsule guiding system according to the present invention, the monitor device displays the current posture of the capsule medical device in the subject under the control of the control device.

In the capsule guiding system according to the present invention, the monitor device displays a current posture of the capsule medical device in the subject with reference to a three-axis rectangular coordinate system defined with respect to the magnetic field generator.

A monitor device according to the present invention is for a capsule guiding system that guides a capsule medical device inserted into a subject by a magnetic field generated by a magnetic field generator. The monitor device comprises a position and posture display unit that displays a current position and a current posture in the subject of the capsule medical device guided by the magnetic field generated by the magnetic field generator; and a magnetic-action display unit that displays a magnitude and a direction of an acting force acting on the capsule medical device due to the magnetic field generated by the magnetic field generator, and a magnitude of a direction-changing speed of the capsule medical device.

In the monitor device according to the present invention, the magnetic-action display unit displays the magnitude and the direction of the acting force and the magnitude of the direction-changing speed superposed on a capsule image indicating the current posture of the capsule medical device in the subject.

In the monitor device according to the present invention, the magnetic-action display unit provides a vector display of the magnitude and the direction of the acting force and the magnitude of the direction-changing speed.

The monitor device according to the present invention further comprises an input-amount display unit that displays an input amount of an operating device in the capsule guiding system that guides the capsule medical device by the magnetic field generated by the magnetic field generator.

In the monitor device according to the present invention, the input-amount display unit displays the input amount of the operating device according to a shape of the operating device identical to the capsule medical device.

The monitor device according to the present invention further comprises an acquisition-information display unit that displays acquisition information acquired by the capsule medical device.

In the monitor device according to the present invention, the acquisition-information display unit is an image display unit that displays an in-vivo image of the subject captured by the capsule medical device.

In the monitor device according to the present invention, the image display unit displays the magnitude of the direction-changing speed and a change direction when the capsule medical device changes a direction, superposed on the in-vivo image of the subject.

### EFFECT OF THE INVENTION

The operating device and the capsule guiding system using the same according to the present invention are substantially identical to a capsule medical device, and includes a casing having a three-dimensional shape with a holdable size as an operating unit. When the entirety or a part of the operating unit is partly or continuously operated to provide 6 degrees-of-freedom motion, respective physical values of 6 degrees-of-freedom motion of the operating unit are detected, and detection results of the respective physical values are output as instruction information for instructing desired 6 degrees-of-freedom motion of the capsule medical device. Therefore, desired 6 degrees-of-freedom motion can be performed by the capsule medical device in a subject by instructing the operating unit to perform one operation or continuous operations with 6 degrees-of-freedom motion. As a result, at least 3 degrees-of-freedom motion (and further 6 degrees-of-freedom motion) to be performed by the capsule medical device in the subject can be easily controlled by one operation or continuous operations of the operating unit.

### BRIEF DESCRIPTION OF DRAWINGS

FIG. 1 is a schematic block diagram of a configuration example of a capsule guiding system according to a first embodiment of the present invention.
FIG. 2 is a schematic diagram of a configuration example of a capsule endoscope in the capsule guiding system according to the first embodiment of the present invention.
FIG. 3 is a schematic diagram for explaining 6 degrees-of-freedom motion of the capsule endoscope.
FIG. 4 is a schematic outline view of a configuration example of an operating device according to the first embodiment of the present invention.
FIG. 5 is a schematic sectional view of a longitudinal cross sectional structure of the operating device according to the first embodiment of the present invention.
FIG. 6 is a schematic sectional view along a line A-A of the operating device shown in FIG. 5.
FIG. 7 is a schematic diagram of a display mode example of a monitor in the capsule guiding system according to the first embodiment of the present invention.
FIG. 8 is a schematic block diagram of a configuration example of a capsule guiding system according to a second embodiment of the present invention.
FIG. 9 is a schematic outline view of a configuration example of an operating device in the capsule guiding system according to the second embodiment of the present invention.
FIG. 10 is a schematic diagram of a display mode example of a monitor in the capsule guiding system according to the second embodiment of the present invention.
FIG. 11 is a schematic block diagram of a configuration example of a capsule guiding system according to a third embodiment of the present invention.
FIG. 12 is a schematic outline view of a configuration example of an operating device in the capsule guiding system according to the third embodiment of the present invention.
FIG. 13 is a schematic block diagram of a configuration example of a capsule guiding system according to a fourth embodiment of the present invention.
FIG. 14 is a schematic outline view of a configuration example of an operating device in the capsule guiding system according to the fourth embodiment of the present invention.
FIG. 15 is a schematic diagram of an outline of the operating unit of the operating device according to the fourth embodiment of the present invention.
FIG. 16 is a schematic block diagram of a configuration example of a capsule guiding system according to a fifth embodiment of the present invention.
FIG. 17 is a schematic outline view of a configuration example of an operating device in the capsule guiding system according to the fifth embodiment of the present invention.
FIG. 18 is a schematic block diagram of a configuration example of a capsule guiding system according to a sixth embodiment of the present invention.
FIG. 19 is a schematic diagram of a display mode example of a monitor in the capsule guiding system according to the sixth embodiment of the present invention.
FIG. 20 is a schematic diagram of a display mode example of a magnetic-action display unit.
FIG. 21 is a schematic diagram of a display mode example of a position and posture display unit.
FIG. 22 is a schematic diagram of a display mode example of an input-amount display unit that displays an input amount of an operating device that operates magnetic guidance for a capsule endoscope.
FIG. 23 is a schematic diagram of a display mode example of an image display unit that displays an in-vivo image group of a subject captured by the capsule endoscope.
FIG. 24 is a schematic sectional view of a modification example of the operating device according to the first embodiment of the present invention.

### EXPLANATIONS OF LETTERS OR NUMERALS

1, 41, 61, 71, 81, 91 Capsule guiding system
2 Capsule endoscope
3 Magnetic field generator
4 Coil power supply
5, 43, 63, 73, 83 Operating device
6 Receiving antenna
7 Receiving device
8 Magnetic-field generating unit
9 Magnetic field detector
10 Position and posture detecting device
11 Input unit
12, 42, 92 Monitor
12a to 12c Position and posture display unit
12d to 12f Predicted-posture display unit
12g, 42g Image display unit
13 Storage unit
14, 44, 64, 74, 94 Control device
20b Dome casing
21 Illuminating unit
22 Condenser lens
23 Imaging device
24 Signal processor
25 Battery
26 Transmitting unit
27 Optical switch
28 Magnet
29 Magnetic-field generating unit
30, 50, 75, 85 Operating unit
31 Fixed unit
32, 32a Movable unit
32b Turning unit
33, 56, 68 Support base
33a Support column
34, 56d, 76a, 87a Cable
35, 67 Force sensor
36 Shaft
37a to 37c Stick member
38a to 38f Distortion gauge
49, 65 Supporting unit
42a to 42c Position display unit
42d to 42f Posture display unit
50a Body
50b Turning unit
51 Turning support column
52 Movable support column
53 Z-stage
54 Y-stage
55 X-stage
56a Initial setting button
56b Return button
56c, 79a, 88a Enable button
57a to 57c, 95 Rotary encoder
58a to 58c Linear encoder
59a to 59f Drive motor
66 Support column
66a Movable unit
66b Fixed unit
76, 87 Operating amount detector
77 Magnetic-field generating stage
78a, 78b Sense coil
79b, 88b Hold button
85a Battery
85b Acceleration sensor
85c Transmitting unit
86 Receiving unit
96 Stopper
100 Magnetic-action display unit
101, 111 Z-point-of-view display unit
102, 112 X-point-of-view display unit
103, 113 Y-point-of-view display unit
101a, 101b, 102a, 102b, 103a, 103b Arrow
110 Position and posture display unit
114, 140 Status display unit
120 Input-amount display unit
121a to 126a Arrow
121b to 126b Input-amount display area
127 Operating device image
130 Image display unit
131 Arrow
BL Bed image
D1 to D6 Capsule image
K1 to K3 Subject image
K4 to K6 Digestive tract image
L1 to L3 Locus
P In-vivo image
SP Thumbnail image

### BEST MODE(S) FOR CARRYING OUT THE INVENTION

Exemplary embodiments of an operating device, a monitor device, and a capsule guiding system according to the present invention will be explained below in detail with reference to the accompanying drawings. In the following embodiments, a capsule endoscope that captures images of inside of digestive organs of a subject (hereinafter, "in-vivo images") is exemplified as an example of a capsule medical device in the capsule guiding system according to the present invention. However, the present invention is not limited to these embodiments.

### First embodiment

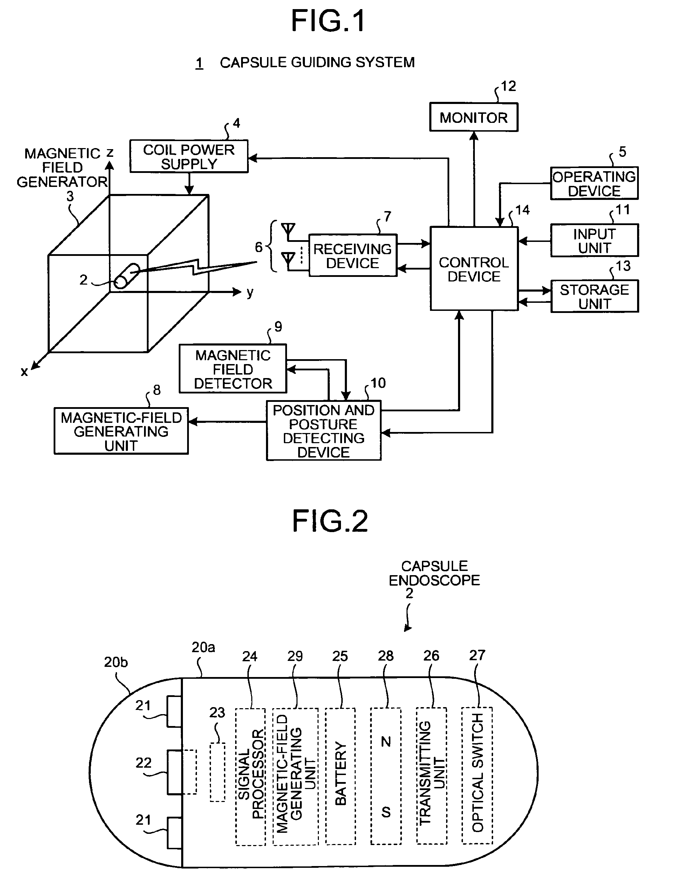

FIG. 1 is a schematic block diagram of a configuration example of a capsule guiding system according to a first embodiment of the present invention. As shown in FIG. 1, a capsule guiding system 1 according to the first embodiment includes a capsule endoscope 2 inserted into digestive organs of a subject such as a patient, a magnetic field generator 3 that generates a magnetic field for guiding the capsule endoscope 2 in the subject, a coil power supply 4 that supplies an electric current to a coil (electromagnet) in the magnetic field generator 3, and an operating device 5 for operating the capsule endoscope 2 with 6 degrees-of-freedom motion. The capsule guiding system 1 further includes a plurality of receiving antennas 6 arranged on a body surface of the subject, a receiving device 7 that receives a radio signal from the capsule endoscope 2 via these receiving antennas 6, a magnetic-field generating unit 8 that generates a magnetic field for detecting the position and posture of the capsule endoscope 2 in the subject, a magnetic field detector 9 that detects an induction field generated from the capsule endoscope 2 by the magnetic field generated by the magnetic-field generating unit 8, and a position and posture detecting device 10 that detects the current position and posture of the capsule endoscope 2 in the subject based on a magnetic field detection result acquired by the magnetic field detector 9. Further, the capsule guiding system 1 includes an input unit 11 that inputs various pieces of information, a monitor 12 that displays various pieces of information such as the current position and current posture of the capsule endoscope 2 in the subject, a storage unit 13 that stores various pieces of information such as in-vivo images of the subject, and a control device 14 that controls respective components in the capsule guiding system 1.

The capsule endoscope 2 is a capsule medical device that acquires an in-vivo image of a subject (an example of in-vivo information), and includes an imaging function and a wireless communication function. The capsule endoscope 2 is inserted into a digestive tract of the subject such as a patient (not shown), and sequentially captures the in-vivo images while moving in the digestive tract of the subject. The capsule endoscope 2 sequentially and wirelessly transmits image signals including the in-vivo images of the subject to the receiving device 7 outside the subject. The capsule endoscope 2 has a built-in magnetic substance or electromagnet such as a permanent magnet (hereinafter, simply "magnet"), and is guided while operating with 6 degrees-of-freedom motion due to a magnetic field generated by the magnetic field generator 3.

The magnetic field generator 3 is realized by combining a plurality of electromagnets such as a Helmholtz coil, and generates the magnetic field capable of guiding the capsule endoscope 2 in the subject. Specifically, a three-axis rectangular coordinate system (hereinafter, "absolute coordinate system") by three axes (x-axis, y-axis, and z-axis) orthogonal to each other is defined, and the magnetic field generator 3 respectively generates magnetic fields of a desired strength with respect to respective axial directions (x-axis direction, y-axis direction, and z-axis direction) of the absolute coordinate system. The magnetic field generator 3 locates a subject (not shown) lying on a bed in a space of the absolute coordinate system (that is, in a space surrounded by electromagnets in the magnetic field generator 3), and applies a three-dimensional rotating magnetic field or three-dimensional gradient field formed by the magnetic field in the respective axial directions of the absolute coordinate system to the capsule endoscope 2 in the subject, thereby operating the capsule endoscope 2 with 6 degrees-of-freedom motion and magnetically guide the capsule endoscope 2. The magnetic field in the respective axial directions of the absolute coordinate system generated by the magnetic field generator 3 (that is, rotating magnetic field and gradient field) is controlled by an alternating current supplied from the coil power supply 4 (amount of current from the coil power supply 4).

As described above, the absolute coordinate system can be the three-axis rectangular coordinate system defined with respect to the magnetic field generator 3 (that is, fixed to the magnetic field generator 3). However, it can be a three-axis rectangular coordinate system fixed with respect to a subject (not shown) who holds the capsule endoscope 2 in the digestive tract thereof, or it can be a three-axis rectangular coordinate system fixed to a bed (not shown) for placing the subject thereon.

The coil power supply 4 supplies electric current for generating the magnetic field applied to the capsule endoscope 2 in the subject to the magnetic field generator 3. The coil power supply 4 supplies the alternating current to the electromagnets in the magnetic field generator 3, to generate the magnetic field in the respective axial directions of the absolute coordinate system.

The operating device 5 functions as an operating device that uses the magnetic field generator 3 with respect to the capsule endoscope 2 inserted into the subject to operate the capsule endoscope 2 in the subject with 6 degrees-of-freedom motion. The operating device 5 inputs instruction information for instructing desired 6 degrees-of-freedom motion to be performed by the capsule endoscope 2 in the subject to the control device 14 based on one operation or continuous operations by a user such as a doctor or nurse. Details of the operating device 5 will be described later.

The receiving antennas 6 capture a radio signal from the capsule endoscope 2 inserted into the subject. Specifically, the receiving antennas 6 are distributed and arranged on a body surface of the subject who holds the capsule endoscope 2 introduced into the digestive tract, to capture the radio signal from the capsule endoscope 2 that moves along the digestive tract. The receiving antennas 6 transmit the radio signal from the capsule endoscope 2 to the receiving device 7. The radio signal from the capsule endoscope 2 corresponds to an image signal including the in-vivo image of the subject captured by the capsule endoscope 2.

The receiving device 7 is connected to the receiving antennas 6, and receives the radio signal from the capsule endoscope 2 via the receiving antennas 6. In this case, the receiving device 7 selects the receiving antenna having highest received field strength of the receiving antennas 6 and acquires the radio signal from the capsule endoscope 2 via the selected receiving antenna. The receiving device 7 performs a demodulation process to the acquired radio signal from the capsule endoscope 2. The receiving device 7 transmits a demodulated image signal to the control device 14. The image signal demodulated by the receiving device 7 includes the in-vivo image of the subject captured by the capsule endoscope 2.

The magnetic-field generating unit 8 generates a magnetic field for detecting the position and posture of the capsule endoscope 2 in the subject. Specifically, the magnetic-field generating unit 8 generates the magnetic fields with respect to two axial directions of three axial directions of the absolute coordinate system based on the instruction from the position and posture detecting device 10, and applies the generated magnetic fields in the two axial directions to the capsule endoscope 2 in the subject. The magnetic-field generating unit 8 generates an induction field from the capsule endoscope 2 in the subject by an action of the respective magnetic fields in the two axial directions.

The magnetic field detector 9 detects the induction field output from the capsule endoscope 2 in the subject by the action of the magnetic field formed by the magnetic-field generating unit 8. Specifically, the magnetic field detector 9 detects the induction field from the capsule endoscope 2 in the subject based on the instruction from the position and posture detecting device 10. In this case, the magnetic field detector 9 detects a magnetic field strength and a direction of the magnetic field of the induction field for the two axial directions of the absolute coordinate system. The magnetic field detector 9 transmits a detection result of the induction field to the position and posture detecting device 10.

The position and posture detecting device 10 three-dimensionally detects the position and posture of the capsule endoscope 2 in the subject. Specifically, every time the detection result of the induction field from the capsule endoscope 2 is acquired from the magnetic field detector 9, the position and posture detecting device 10 calculates spatial coordinates and direction vectors (direction vectors in a longitudinal axis direction and a radial direction of the capsule endoscope 2) of the capsule endoscope 2 in the absolute coordinate system based on the acquired induction field. The position and posture detecting device 10 three-dimensionally detects the current position and current posture of the capsule endoscope 2 in the subject based on the spatial coordinates and the direction vectors of the capsule endoscope 2 in the absolute coordinate system. The position and posture detecting device 10 transmits the thus detected current position information and current posture information of the capsule endoscope 2 in the subject to the control device 14.

The posture of the capsule endoscope 2 is determined based on a rotational state centering on the longitudinal axis of the capsule endoscope 2 defined by the longitudinal axis direction of a capsule casing included in the capsule endoscope 2 and the radial direction of the capsule casing (direction of two axes at right angles to each other perpendicular to the longitudinal axis direction of capsule casing).

The input unit 11 is realized by using an input device such as a keyboard and a mouse, and inputs various pieces of information to the control device 14 corresponding to an input operation by a user such as a doctor or nurse. The various pieces of information input to the control device 14 from the input unit 11 include, for example, instruction information instructed to the control device 14, patient information of the subject, and examination information of the subject. The patient information of the subject is specific information for specifying the subject, and includes information such as a patient name of the subject, patient ID, date of birth, gender, and age. The examination information of the subject is specific information for specifying a capsule endoscope examination performed with respect to the subject (an examination for observing the inside of the digestive tract by inserting the capsule endoscope 2 into the digestive tract), and includes information such as an examination ID and the date of examination.

The monitor 12 is a monitor device realized by using various displays such as a CRT display or a liquid crystal display, and displays various pieces of information instructed to be displayed by the control device 14. Specifically, the monitor 12 displays information useful for the capsule endoscope examination such as an in-vivo image group of the subject captured by the capsule endoscope 2, patient information of the subject, and examination information of the subject. The monitor 12 also displays the information useful for magnetic guidance for the capsule endoscope 2 such as current position information and current posture information of the capsule endoscope 2 in the subject.

The storage unit 13 is realized by using various storage media that rewritably stores information, such as a RAM, EEPROM, flash memory, or hard disk. The storage unit 13 stores various pieces of information instructed to be stored by the control device 14, and transmits information instructed to be read by the control device 14 from the stored various pieces of information to the control device 14. The storage unit 13 stores the in-vivo image group of the subject, the patient information and examination information of the subject, and the current position information and current posture information of the capsule endoscope 2 in the subject under control of the control device 14.

The control device 14 controls the motion of respective components (the magnetic field generator 3, the coil power supply 4, the operating device 5, the receiving device 7, the position and posture detecting device 10, the input unit 11, the monitor 12, and the storage unit 13) in the capsule guiding system 1, and controls input and output of signals between the respective components. Specifically, the control device 14 controls the motion of the receiving device 7, the position and posture detecting device 10, the monitor 12, and the storage unit 13 based on the instruction information input by the input unit 11. The control device 14 controls the amount of current of the coil power supply 4 with respect to the magnetic field generator 3 based on the instruction information input by the operating device 5, and controls a magnetic field generating motion of the magnetic field generator 3 through the control of the coil power supply 4. Accordingly, the control device 14 controls the 6 degrees-of-freedom motion of the capsule endoscope 2 in the subject. The control device 14 controls an operation timing of the magnetic field generator 3, an operation timing of the receiving device 7, and an operation timing of the position and posture detecting device 10 so that the timing at which the magnetic field generator 3 generates the magnetic field with respect to the capsule endoscope 2, a timing at which the receiving device 7 receives the radio signal from the capsule endoscope 2, and a timing at which the position and posture detecting device 10 detects the current position and current posture of the capsule endoscope 2 by using the magnetic-field generating unit 8 and the magnetic field detector 9 do not overlap on each other.

The control device 14 acquires the current position information and current posture information of the capsule endoscope 2 from the position and posture detecting device 10, and displays the acquired current position information and current posture information on the monitor 12. Every time the control device 14 acquires the current position information and current posture information of the capsule endoscope 2 from the position and posture detecting device 10, the control device 14 controls the monitor 12 to update the current position information and current posture information of the capsule endoscope 2 in the subject to the latest information.

Further, the control device 14 has an image processing function for generating (restructuring) the in-vivo image of the subject based on the image signal demodulated by the receiving device 7. Specifically, the control device 14 acquires an image signal from the receiving device 7, and performs predetermined image processing with respect to the acquired image signal to generate image information (that is, in-vivo images of the subject captured by the capsule endoscope 2). The control device 14 sequentially causes the storage unit 13 to store the generated in-vivo images of the subject, and displays the in-vivo image group of the subject on the monitor 12 based on the instruction information from the input unit 11.

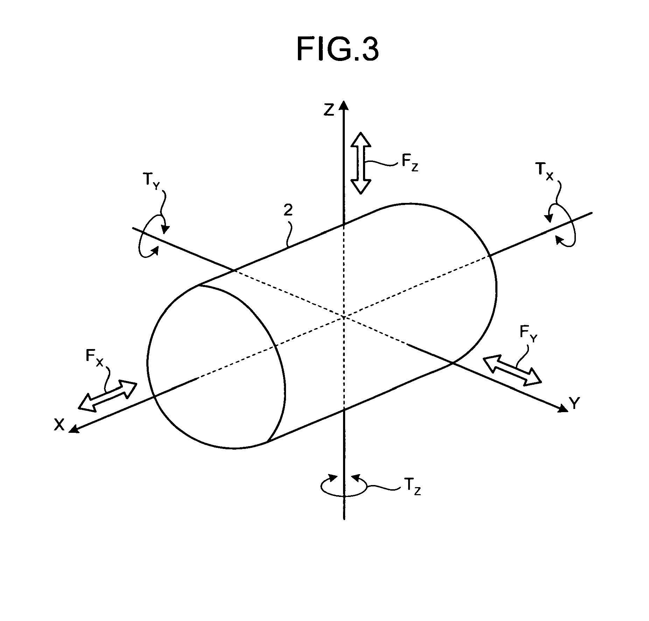

The capsule endoscope 2 described above is explained next in detail. FIG. 2 is a schematic diagram of a configuration example of the capsule endoscope 2 in the capsule guiding system 1 according to the first embodiment of the present invention. FIG. 3 is a schematic diagram for explaining 6 degrees-of-freedom motion of the capsule endoscope 2. As shown in FIGS. 2 and 3, the capsule endoscope 2 has a capsule casing including a substantially opaque cylindrical casing 20a, at least a part thereof being capable of transmitting light in a predetermined wavelength band (for example, infrared rays) and a transparent dome casing 20b. The capsule casing is formed by covering one end (opening end) of the cylindrical casing 20a with the other end having a dome shape by the dome casing 20b.

In the capsule casing formed of the cylindrical casing 20a and the dome casing 20b, an illuminating unit 21 realized by an LED or the like, a condenser lens 22, and an imaging device 23 are provided on the dome casing 20b side to capture a subject around the dome casing 20b. An image signal output from the imaging device 23 is processed by a signal processor 24, and is wirelessly transmitted to the receiving device 7 from a transmitting unit 26 as the image signal including an in-vivo image of the subject.

An optical switch 27 having a sensitivity to the light in the predetermined wavelength band such as the infrared rays and a battery 25 are arranged on the cylindrical casing 20a side of the capsule casing. When having received the infrared rays transmitted through the dome part of the cylindrical casing 20a, the optical switch 27 is changed over to a power-on state and starts supplying force to the respective components in the capsule endoscope 2 from the battery 25. Upon reception of the infrared rays, the optical switch 27 maintains the power-on state. When having received the infrared rays again in the power-on state, the optical switch 27 can be changed over to a power-off state where power supply is stopped.

Further, a magnetic-field generating unit 29 that generates the induction field by an action of the magnetic field generated by the magnetic-field generating unit 8 is arranged on the cylindrical casing 20a side in the capsule casing. The magnetic-field generating unit 29 is realized by using, for example, two coils with an opening direction of the coil being arranged in an orthogonal two axis directions. The magnetic-field generating unit 29 generates the induction field by the action of the magnetic field generated by the magnetic-field generating unit 8 for detecting the current position and current posture of the capsule endoscope 2, and outputs the generated induction field to the magnetic field detector 9.

A magnet 28 is arranged on the cylindrical casing 20a side in the capsule casing (for example, near the central part of the capsule endoscope 2). As shown in FIG. 2, a magnetic pole of the magnet 28 is arranged in a direction perpendicular to the longitudinal axis direction of the capsule endoscope 2, that is, in a radial direction of the capsule casing. When the rotating magnetic field is applied to the capsule endoscope 2, the magnet 28 rotates like a rotor of a motor, attracting on the rotating magnetic field. The capsule endoscope 2 rotates three-dimensionally, centering on the longitudinal axis or a radial axis perpendicular to the center of the longitudinal axial due to a rotary motion of the magnet 28. When the gradient field is applied to the capsule endoscope 2, the magnet 28 moves three-dimensionally, attracting on the gradient field. The capsule endoscope 2 moves three-dimensionally in a coordinate space of the absolute coordinate system due to such a displacing motion of the magnet 28.

As shown in FIG. 3, a three-axis rectangular coordinates system (hereinafter, "capsule coordinate system") by three axes (XYZ) orthogonal to each other is defined with respect to the capsule endoscope 2 having such a configuration. The capsule coordinate system defines the position and posture of the capsule endoscope 2 in the absolute coordinate system, and freely moves in a spatial coordinate of the absolute coordinate system. The spatial coordinate and a direction vector of the capsule coordinate system can be converted to components of the absolute coordinate system (spatial coordinate, direction vector, and the like) by performing a predetermined coordinate conversion process. The respective axial directions of the three axes (X-axis, Y-axis, and Z-axis) of the capsule coordinate system are specific axial directions of the capsule endoscope 2. For example, the X-axis direction of the capsule coordinate system is a longitudinal axis direction of the capsule endoscope 2 and is an imaging direction of the capsule endoscope 2.

Specifically, the X-axis of the capsule coordinate system is matched with a central axis of the capsule endoscope 2 in the longitudinal axis direction. The Z-axis of the capsule coordinate system is a radial axis perpendicular to the longitudinal axis direction of the capsule endoscope 2 and in a magnetizing direction of the magnet 28 shown in FIG. 2 (a direction connecting the north pole and the south pole). The Y-axis of the capsule coordinate system is a radial axis of the capsule endoscope 2, and in a direction perpendicular to the Z-axis. In this case, the front of the capsule endoscope 2 (that is, on the dome casing 20b side of the capsule casing) is designated as a positive direction of the X-axis, a direction from the south pole to the north pole of the magnet 28 is designated as the positive direction of the Z-axis, and the right as viewed from the front of the capsule endoscope 2 is designated as the positive direction of the Y-axis.

At least one of a driving force F_{X} in the X-axis direction, a driving force F_{Y} in the Y-axis direction, a driving force F_{Z} in the Z-axis direction, a turning force T_{X} around the X-axis, a turning force T_{Y} around the Y-axis, and a turning force T_{Z} around the Z-axis is generated in the capsule endoscope 2, in which the capsule coordinate system is defined, due to an action of the magnet 28 that three-dimensionally rotates or moves due to the rotating magnetic field or gradient field generated by the magnetic field generator 3. The capsule endoscope 2 operates three-dimensionally with 6 degrees-of-freedom motion by at least one of the driving forces F_{X}, F_{Y}, and F_{Z}, and turning forces T_{X}, T_{Y}, and T_{Z}, or a resultant force thereof. Specifically, the capsule endoscope 2 performs a forward and backward motion for being displaced in the positive or negative direction of the X-axis by the driving force F_{X}, performs a shifting motion for being displaced (translating) in the positive or negative direction of the Y-axis by the driving force F_{Y}, and performs a shifting motion for being displaced (translating) in the positive or negative direction of the Z-axis by the driving force F_{Z}. The capsule endoscope 2 also performs the rotary motion for rotating around the X-axis by the turning force T_{X}, performs a direction changing motion for changing the direction by rotating around the Y-axis by the turning force T_{Y}, and performs a direction changing motion for changing the direction by rotating around the Z-axis by the turning force T_{Z}. The capsule endoscope 2 three-dimensionally performs desired 6 degrees-of-freedom motion by appropriately combining the forward and backward motion, the direction changing motion, the rotary motion, and the shifting motion in presence of the magnetic field generated by the magnetic field generator 3.

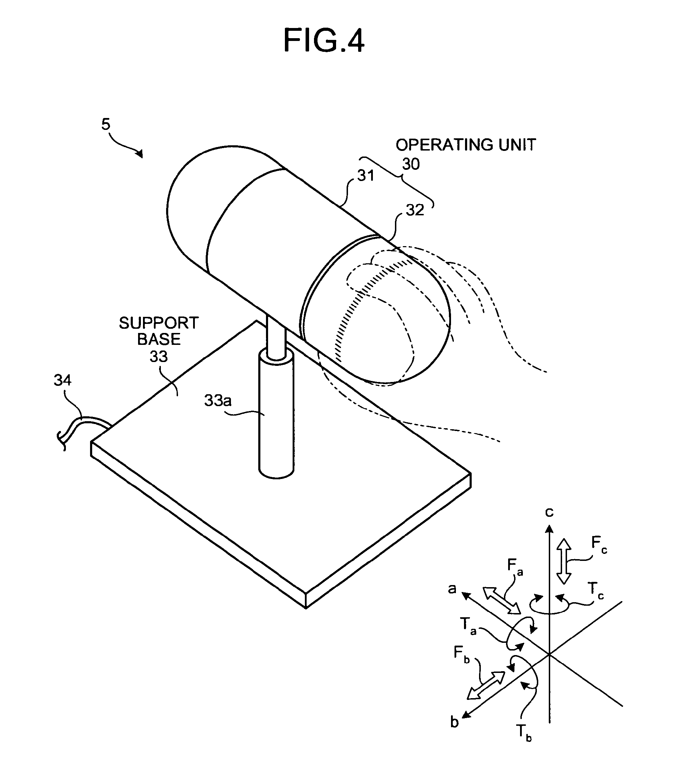

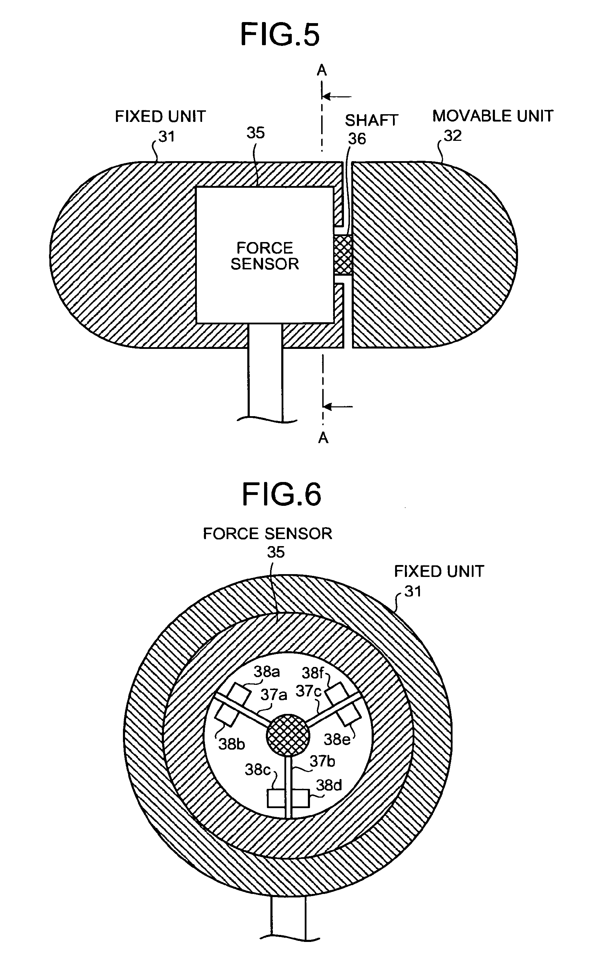

The operating device 5 in the capsule guiding system 1 according to the first embodiment of the present invention is explained next in detail. FIG. 4 is a schematic outline view of a configuration example of the operating device 5 according to the first embodiment of the present invention. FIG. 5 is a schematic sectional view of a longitudinal cross sectional structure of the operating device 5 according to the first embodiment of the present invention. FIG. 6 is a schematic sectional view along a line A-A of the operating device 5 shown in FIG. 5. As shown in FIGS. 4 and 5, the operating device 5 according to the first embodiment includes an operating unit 30 for performing one operation or continuous operations corresponding to the desired 6 degrees-of-freedom motion of the capsule endoscope 2, a support base 33 that supports the operating unit 30, and a force sensor 35 that detects information of force applied to the operating unit 30 by one operation or continuous operations.

The operating unit 30 is a three-dimensional casing having directionality such as an elliptical or capsule shape, and is operated by a user such as a doctor or nurse when the capsule endoscope 2 in the subject performs desired 6 degrees-of-freedom motion. Specifically, the operating unit 30 is a three-dimensional casing substantially identical to the capsule endoscope 2 and is a size holdable by the user. The operating unit 30 includes a movable unit 32 that receives one operation or continuous operations in response to the desired 6 degrees-of-freedom motion of the capsule endoscope 2 and a fixed unit 31 that supports the movable unit 32.

The casing forming the operating unit 30 is not limited to the capsule shape so long as it has a three-dimensional shape visually indicating a specific axial direction of the capsule endoscope 2, for example, the longitudinal axis direction (and further, the imaging direction) of the capsule endoscope 2, and it can be a three-dimensional shape having directionality such as a hexahedron or octagonal prism. An axis display (for example, a mark such as an arrow) for indicating a specific axial direction of the capsule endoscope 2 by marking or the like can be equipped in the operating unit 30, and the operating unit 30 can have the directionality by the axis display. In this case, the three-dimensional shape of the operating unit 30 including the axis display can be the one which does not have the directionality such as a spherical shape. Further, the operating unit 30 can have the axis display and the three-dimensional shape having the directionality.

The fixed unit 31 is fixed and supported by a supporting column 33a of the support base 33 and includes the force sensor 35 incorporated therein. The fixed unit 31 is connected to the movable unit 32 via a shaft 36 of the force sensor 35, and supports the movable unit 32 by the shaft 36 so that the 6 degrees-of-freedom motion can be realized. The fixed unit 31 is fixed with respect to the 6 degrees-of-freedom motion of the movable unit 32. That is, the fixed unit 31 hardly moves even if the movable unit 32 moves with 6 degrees-of-freedom motion and maintains the fixed state with respect to the support base 33.

The movable unit 32 is a moving unit of the operating unit 30, and is held by the user at the time of operating the capsule endoscope 2 with the desired 6 degrees-of-freedom motion. The movable unit 32 operates with 6 degrees-of-freedom motion corresponding to the desired 6 degrees-of-freedom motion of the capsule endoscope 2 in response to one operation or continuous operations corresponding to the desired 6 degrees-of-freedom motion to be performed by the capsule endoscope 2.

As shown in FIG. 4, a three-axis rectangular coordinate system (hereinafter, "operation coordinate system") by three axes (abc) orthogonal to each other is defined with respect to the operating unit 30 including the fixed unit 31 and the movable unit 32. An a-axis, a b-axis, and a c-axis of the operation coordinate system respectively correspond to the X-axis, Y-axis, and Z-axis of the capsule coordinate system. That is, the a-axis of the operation coordinate system is matched with the central axis of the capsular operating unit 30 in the longitudinal axis direction substantially identical to the capsule endoscope 2. The c-axis of the operation coordinate system is a radial axis perpendicular to the longitudinal axis direction of the operating unit 30 and substantially parallel to the supporting column 33a of the support base 33 (that is, a direction substantially perpendicular to a surface of the support base 33), and the b-axis of the operation coordinate system is a radial axis of the operating unit 30 and perpendicular to the c-axis. In this case, the front of the operating unit 30 (that is, a direction from the movable unit 32 toward the fixed unit 31) is the positive direction of the a-axis, the direction from the support base 33 toward the fixed unit 31 is the positive direction of the c-axis, and the right as viewed from the front of the operating unit 30 is the positive direction of the b-axis.

The movable unit 32 of the operating unit 30, in which the operation coordinate system is defined, operates with 6 degrees-of-freedom motion corresponding to the desired 6 degrees-of-freedom motion of the capsule endoscope 2 by one operation or continuous operations for operating the capsule endoscope 2 with the desired 6 degrees-of-freedom motion. In this case, at least one of a force Fₐ in the a-axis direction, a force F_{b} in the b-axis direction, a force F_{c} in the c -axis direction, a turning force Tₐ around the a-axis, a turning force T_{b} around the b-axis, and a turning force T_{c} around the c-axis is applied to the movable unit 32. The forces Fₐ in the positive and negative directions of the a-axis respectively correspond to the driving forces F_{X} in the positive and negative directions of the X-axis in the capsule coordinate system, the forces F_{b} in the positive and negative directions of the b-axis respectively correspond to the driving forces F_{Y} in the positive and negative directions of the Y-axis in the capsule coordinate system, and the forces F_{c} in the positive and negative directions of the c-axis respectively correspond to the driving forces F_{Z} in the positive and negative directions of the Z-axis in the capsule coordinate system. The turning forces Tₐ in clockwise and counterclockwise directions of the a-axis respectively correspond to the turning forces T_{X} in the clockwise and counterclockwise directions of the X-axis in the capsule coordinate system, the turning forces T_{b} in the clockwise and counterclockwise directions of the b-axis respectively correspond to the turning forces T_{Y} in the clockwise and counterclockwise directions of the Y-axis in the capsule coordinate system, and the turning forces T_{c} in the clockwise and counterclockwise directions of the c-axis respectively correspond to the turning forces T_{z} in the clockwise and counterclockwise directions of the Z-axis in the capsule coordinate system.

The force sensor 35 is a 6-axis force sensor, and functions as a detecting unit that detects respective pieces of force information (an example of physical values) of the 6 degrees-of-freedom motion of the movable unit 32. Specifically, the force sensor 35 is connected to the movable unit 32 via the shaft 36, and receives an external force applied to the movable unit 32 by one operation or continuous operations of the movable unit 32 via the shaft 36. The force sensor 35 detects the force information such as the magnitude and direction of the external force applied to the movable unit 32 transmitted through the shaft 36. The external force applied to the movable unit 32 is at least one of the forces Fₐ, F_{b}, and F_{c} and the turning forces Tₐ, T_{b}, and T_{c} or a resultant force thereof.

In more detail, as shown in FIG. 6, the force sensor 35 includes stick members 37a to 37c that support the shaft 36 at three points with respect to the casing of the force sensor 35, and distortion gauges 38a to 38f that measure respective distortions of the stick members 37a to 37c. The stick members 37a to 37c support the shaft 36 connected to the movable unit 32, and generate distortions due to the external force applied to the movable unit 32 transmitted via the shaft 36. The distortion gauges 38a and 38b measure the distortion generated in the stick member 37a, the distortion gauges 38c and 38d measure the distortion generated in the stick member 37b, and the distortion gauges 38e and 38f measure the distortion generated in the stick member 37c.

The force sensor 35 having such a configuration detects 6-axial components of the external force of the movable unit 32, that is, respective force components in the a-axis direction, b-axis direction, and c-axis direction of the external force applied to the movable unit 32 and respective moment components around the a-axis, the b-axis, and the c-axis based on respective distortion measurement results by the distortion gauges 38a to 38f. As a result, the force sensor 35 detects at least one piece of force information of the forces Fₐ, F_{b}, and F_{c} and the turning forces Tₐ, T_{b}, and T_{c} applied to the movable unit 32. The force sensor 35 is connected to the control device 14 via a cable 34 shown in FIG. 4, and transmits the detected force information of the external force applied to the movable unit 32 to the control device 14. In this case, the force information detected by the force sensor 35 is input to the control device 14 as instruction information for instructing the desired 6 degrees-of-freedom motion of the capsule endoscope 2.

The operating device 5 having such a configuration can provide the desired 6 degrees-of-freedom motion of the capsule endoscope 2 in the subject (that is, the operating device 5 can cause the capsule endoscope 2 in the subject to operate with desired 6 degrees-of-freedom motion) by providing one operation or continuous operations to the movable unit 32. Specifically, when the operating device 5 causes the capsule endoscope 2 to perform a forward motion, it only needs to receive one operation for moving (pressing) the movable unit 32 by applying the force Fₐ in the positive direction of the a-axis corresponding to the X-axis, and when the operating device 5 causes the capsule endoscope 2 to perform a backward motion, it only needs to receive one operation for moving (pulling) the movable unit 32 by applying the force Fₐ in the negative direction of the a-axis corresponding to the X-axis. The control device 14 controls the direction of the forward and backward motion of the capsule endoscope 2 corresponding to the direction of the force Fₐ acquired from the operating device 5, and controls the magnitude of the driving force F_{X} corresponding to the force Fₐ.

When the operating device 5 causes the capsule endoscope 2 to perform a shifting motion in the positive direction of the Y-axis, it only needs to receive one operation for moving the movable unit 32 by applying the force F_{b} in the positive direction of the b-axis corresponding to the Y-axis, and when the operating device 5 causes the capsule endoscope 2 to perform the shifting motion in the negative direction of the Y-axis, it only needs to receive one motion for moving the movable unit 32 by applying the force F_{b} in the negative direction of the b-axis corresponding to the Y-axis. The control device 14 controls the Y-axis direction of the shifting motion of the capsule endoscope 2 corresponding to the direction of the force F_{b} acquired from the operating device 5, and controls the magnitude of the driving force F_{Y} corresponding to the magnitude of the force F_{b}.

Further, when the operating device 5 causes the capsule endoscope 2 to perform the shifting motion in the positive direction of the Z-axis, it only needs to receive one motion for moving the movable unit 32 by applying the force F_{c} in the positive direction of the c-axis corresponding to the Z-axis, and when the operating device 5 causes the capsule endoscope 2 to perform the shifting motion in the negative direction of the Z-axis, it only needs to receive one motion for moving the movable unit 32 by applying the force F_{c} in the negative direction of the c-axis corresponding to the Z-axis. The control device 14 controls the Z-axis direction of the shifting motion of the capsule endoscope 2 corresponding to the direction of the force F_{c} acquired from the operating device 5, and controls the magnitude of the driving force F_{z} corresponding to the magnitude of the force F_{c}.

When the operating device 5 causes the capsule endoscope 2 to perform a clockwise rotary motion, it only needs to receive one motion for turning the movable unit 32 clockwise by applying the turning force Tₐ clockwise around the a-axis corresponding to the X-axis, and when the operating device 5 causes the capsule endoscope 2 to perform a counterclockwise rotary motion, it only needs to receive one motion for rotating the movable unit 32 by applying the turning force Tₐ counterclockwise around the a-axis corresponding to the X-axis. The control device 14 controls the direction of the rotary motion of the capsule endoscope 2 corresponding to the direction of the turning force Tₐ acquired from the operating device 5, and controls the magnitude of the turning force T_{X} corresponding to the magnitude of the turning force Tₐ. The turning force includes a rotation torque.

Further, when the operating device 5 causes the capsule endoscope 2 to perform the direction changing motion around the Y-axis, it only needs to receive one motion for turning the movable unit 32 by applying the turning force T_{b} around the b-axis corresponding to the Y-axis, and when the operating device 5 causes the capsule endoscope 2 to perform the direction changing motion around the Z-axis, it only needs to receive one motion for turning the movable unit 32 by applying the turning force T_{c} around the c-axis corresponding to the Z-axis. The control device 14 controls the rotation direction in the direction changing motion around the Y-axis of the capsule endoscope 2 corresponding to the direction of the turning force T_{b} acquired from the operating device 5, and controls the magnitude of the turning force T_{Y} corresponding to the magnitude of the force T_{b}. Further, the control device 14 controls the rotation direction in the direction changing motion around the Z-axis of the capsule endoscope 2 corresponding to the direction of the turning force T_{c} acquired from the operating device 5, and controls the magnitude of the turning force T_{Z} corresponding to the magnitude of the force T_{c}.

The operating device 5 can cause the capsule endoscope 2 to perform the three-dimensional 6 degrees-of-freedom motion combining at least two of the forward and backward motion, shifting motion, rotary motion, and direction changing motion by receiving continuous operations for moving the movable unit 32 by applying a resultant force acquired by combining at least two of the forces Fₐ, F_{b}, and F_{c} and the turning forces Tₐ, T_{b}, and T_{c}. In this case, the control device 14 controls the direction of the three-dimensional 6 degrees-of-freedom motion (such as a driving direction or rotation direction) of the capsule endoscope 2 corresponding to the direction of the resultant force acquired from the operating device 5, and controls the magnitude of the force of the three-dimensional 6 degrees-of-freedom motion (such as a promotion direction or rotation direction) corresponding to the magnitude of the resultant force.

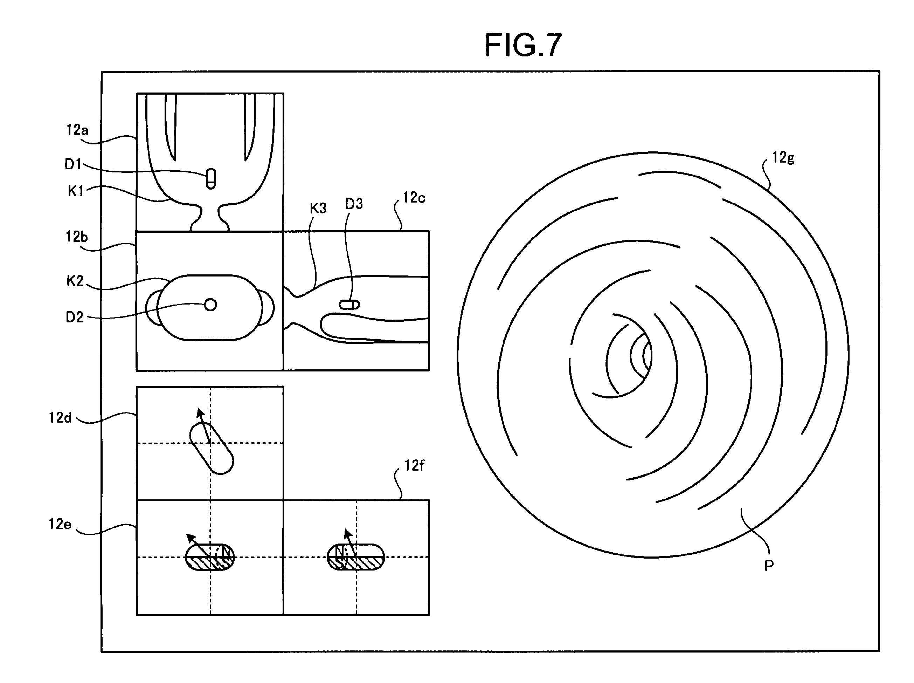

The monitor 12 that displays various pieces of information such as the current position information and current posture information of the capsule endoscope 2 in the subject is explained next in detail. FIG. 7 is a schematic diagram of a display mode example of the monitor 12 in the capsule guiding system 1 according to the first embodiment of the present invention. The monitor 12 displays in-vivo images of the subject captured by the capsule endoscope 2, current position information of the capsule endoscope 2 in the subject, and current posture information of the capsule endoscope 2 under control of the control device 14.

Specifically, as shown in FIG. 7, the monitor 12 includes position and posture display units 12a to 12c that display the current position information and current posture information of the capsule endoscope 2 in the subject, predicted-posture display units 12d to 12f that display predicted posture information of the capsule endoscope 2 after operating by the operating device 5, and an image display unit 12g that displays in-vivo images P of the subject captured by the capsule endoscope 2.

The position and posture display units 12a to 12c display the current position information and current posture information of the capsule endoscope 2 in the subject with reference to the capsule coordinate system under control of the control device 14. Specifically, the position and posture display unit 12a superposes a pattern image (hereinafter, "capsule image") D1 of the capsule endoscope 2 as viewed from the Z-axis direction of the capsule coordinate system and a pattern image (hereinafter, "subject image") K1 of the subject as viewed from the Z-axis direction of the capsule coordinate system on each other and displays a superposed image. In this case, the position and posture display unit 12a displays the capsule image D1 on a substantially central part of a display screen in a state with a relative direction with respect to the display screen being fixed at all times, and displays the subject image K1 while changing (updating) the position and direction of the subject image K1 so that the display position of the capsule image D1 in the subject image K1 and a relative posture of the capsule image D1 with respect to the subject image K1 are respectively matched with the current position and current posture of the capsule endoscope 2 in an XY plane.

The position and posture display unit 12b superposes a capsule image D2 as viewed from the X-axis direction of the capsule coordinate system and a subject image K2 as viewed from the X-axis direction of the capsule coordinate system on each other and displays a superposed image. In this case, the position and posture display unit 12b displays the capsule image D2 on a substantially central part of the display screen in a state with the relative direction with respect to the display screen being fixed at all times, and displays the subject image K2 while changing (updating) the position and direction of the subject image K2 so that the display position of the capsule image D2 in the subject image K2 and the relative posture of the capsule image D2 with respect to the subject image K2 are respectively matched with the current position and current posture of the capsule endoscope 2 in a YZ plane.

The position and posture display unit 12c superposes a capsule image D3 as viewed from the Y-axis direction of the capsule coordinate system and a subject image K3 as viewed from the Y-axis direction of the capsule coordinate system on each other and displays a superposed image. In this case, the position and posture display unit 12c displays the capsule image D3 on a substantially central part of the display screen in a state with the relative direction with respect to the display screen being fixed at all times, and displays the subject image K3 while changing (updating) the position and direction of the subject image K3 so that the display position of the capsule image D3 in the subject image K3 and the relative posture of the capsule image D3 with respect to the subject image K3 are respectively matched with the current position and current posture of the capsule endoscope 2 in an XZ plane.

The subject images K1 to K3 displayed by the position and posture display units 12a to 12c can be a pattern image added with a pattern image of the digestive tract, in which the capsule endoscope 2 in the subject moves, although not particularly shown in FIG. 7. Accordingly, the position and posture display units 12a to 12c can display the current position information and current posture information of the capsule endoscope 2 in the subject more comprehensively.

The predicted-posture display units 12d to 12f display prediction information of the posture (that is, predicted posture information), which is taken by the capsule endoscope 2 in the subject after a predetermined time in response to one operation or continuous operations of the operating device 5 that operates the capsule endoscope 2 with 6 degrees-of-freedom motion. Specifically, the predicted-posture display unit 12d displays predicted posture information of the capsule endoscope 2 as viewed from the Z-axis direction of the capsule coordinate system. The predicted-posture display unit 12d provides a vector display of at least one of the forces (the driving force and the turning force of the capsule endoscope 2) generated for the capsule endoscope 2 in the subject to perform the 6 degrees-of-freedom motion in response to one operation or continuous operations by the operating device 5 that operates the capsule endoscope 2 in the subject with 6 degrees-of-freedom motion. For example, as shown in FIG. 7, the predicted-posture display unit 12d provides a vector display of a resultant force of the driving forces F_{X} and F_{Y} generated for operating the capsule endoscope 2 in the subject with 6 degrees-of-freedom motion.

The predicted-posture display unit 12e displays predicted posture information of the capsule endoscope 2 as viewed from the X-axis direction of the capsule coordinate system. The predicted-posture display unit 12e provides a vector display of at least one of the forces generated for the capsule endoscope 2 in the subject to perform the 6 degrees-of-freedom motion in response to one operation or continuous operations by the operating device 5 that operates the capsule endoscope 2 in the subject with 6 degrees-of-freedom motion. For example, as shown in FIG. 7, the predicted-posture display unit 12e provides a vector display of a resultant force of the driving forces F_{Y} and F_{Z} generated for operating the capsule endoscope 2 in the subject with 6 degrees-of-freedom motion.

The predicted-posture display unit 12f displays predicted posture information of the capsule endoscope 2 as viewed from the Y-axis direction of the capsule coordinate system. The predicted-posture display unit 12f provides a vector display of at least one of the forces generated for the capsule endoscope 2 in the subject to perform the 6 degrees-of-freedom motion in response to one operation or continuous operations by the operating device 5 that operates the capsule endoscope 2 in the subject with 6 degrees-of-freedom motion. For example, as shown in FIG. 7, the predicted-posture display unit 12f provides a vector display of a resultant force of the driving forces F_{X} and F_{Z} generated for operating the capsule endoscope 2 in the subject with 6 degrees-of-freedom motion.

When the capsule endoscope 2 performs the rotary motion in response to one operation or continuous operations of the operating device 5, the predicted-posture display units 12e and 12f display the prediction information of a rotational position of the capsule endoscope 2 changing due to the rotary motion (rotational position at the time of rotating around the X-axis). Specifically, as shown in FIG. 7, the predicted-posture display units 12e and 12f display a polar orientation of the magnet 28 in the capsule endoscope 2 (rectilinear direction connecting the north pole and the south pole) as the prediction information of the rotational position of the capsule endoscope 2.

The image display unit 12g displays in-vivo images P of the subject captured by the capsule endoscope 2 in the subject under control of the control device 14. The image display unit 12g displays the in-vivo images P of the subject, sequentially changing over the in-vivo image P to a desired in-vivo image, according to an instruction of the control device 14 based on the instruction information input by the input unit 11.

As described above, in the first embodiment of the present invention, the casing having the same directionality as that of the capsule endoscope is provided as the operating unit by having an axis display unit that indicates a specific axial direction of the capsule endoscope by a three-dimensional shape or marking having the three-axis rectangular coordinate system (operation coordinate system) corresponding to the capsule coordinate system defined with respect to the capsule endoscope. A unit of the operating unit is defined as the moving unit capable of performing the 6 degrees-of-freedom motion. When the moving unit is operated with 6 degrees-of-freedom motion by performing one operation or continuous operations corresponding to the desired 6 degrees-of-freedom motion of the capsule endoscope, the external force applied to the moving unit is detected by the detecting unit (force sensor), and a detection result of the detecting unit is output as the instruction information for instructing the desired 6 degrees-of-freedom motion of the capsule endoscope. Accordingly, by providing one operation or continuous operations for causing the moving unit to perform the 6 degrees-of-freedom motion in the operation coordinate system to the moving unit, the capsule endoscope in the subject can perform the desired 6 degrees-of-freedom motion. As a result, the operating device that can easily operate the capsule endoscope in the subject with at least 6 degrees-of-freedom motion by one operation or continuous operations of the moving unit and the capsule guiding system using the same can be realized.

Because the operating unit has a three-dimensional shape substantially identical to the capsule endoscope and is a holdable size, one operation or continuous operations of the moving unit for causing the capsule endoscope 2 in the subject to perform the desired 6 degrees-of-freedom motion can be easily imaged, by assuming the three-dimensional operating unit as the capsule endoscope in the subject. As a result, one operation or continuous operations of the moving unit for causing the capsule endoscope to perform the desired 6 degrees-of-freedom motion can be easily performed.

Further, because the current position information and current posture information of the capsule endoscope in the subject is displayed on the monitor device with reference to the capsule coordinate system, the relative posture of the capsule endoscope with respect to the subject can be easily operated and the capsule endoscope can be easily magnetically guided to a desired position in the subject by performing one operation or continuous operations of the moving unit while visually checking the current position information and current posture information.

Because the prediction information of the posture to be taken by the capsule endoscope is displayed on the monitor device based on one operation or continuous operations of the moving unit, the 6 degrees-of-freedom motion of the capsule endoscope in the subject can be operated more easily by performing one operation or continuous operations of the moving unit, while visually checking the prediction information. Further, a torque (turning force) generated in the capsule endoscope can be visually checked by displaying prediction information on the monitor device.

Further, because the force (such as the driving force and the turning force) generated in the capsule endoscope is displayed by a vector on the monitor device so that the capsule endoscope can perform desired 6 degrees-of-freedom motion by one operation or continuous operations of the moving unit, the magnitude and direction of the force for operating the capsule endoscope in the subject with 6 degrees-of-freedom motion can be easily changed to a desired magnitude and direction.

Because the detecting unit is configured by the force sensor, when it is desired to stop the operation, the shaft of the force sensor returns to an original position and an input by the operating unit is suspended only by releasing a hand from the operating device (that is, by releasing grip of the operating unit). As a result, operability of the operating unit is improved and one operation or continuous operations of the operating unit is facilitated.

### Second embodiment

A second embodiment of the present invention is explained next. In the first embodiment, the respective physical values (force information) of the 6 degrees-of-freedom motion of the movable unit 32 according to one operation or continuous operations are detected by the force sensor 35. However, in the second embodiment, the respective physical values (force information) of the 6 degrees-of-freedom motion of the movable unit 32 according to one operation or continuous operations are detected by a plurality of rotary encoders and linear encoders.

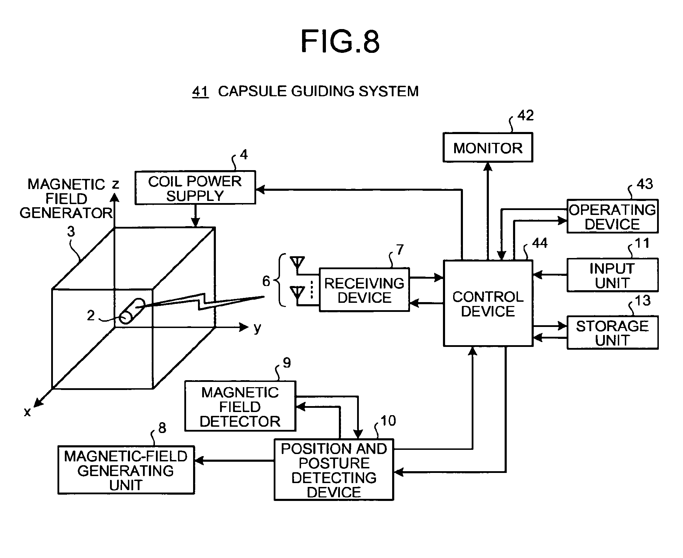

FIG. 8 is a schematic block diagram of a configuration example of a capsule guiding system according to the second embodiment of the present invention. As shown in FIG. 8, a capsule guiding system 41 according to the second embodiment includes an operating device 43 instead of the operating device 5, a monitor 42 instead of the monitor 12, and a control device 44 instead of the control device 14 in the capsule guiding system 1 according to the first embodiment. Other configurations of the second embodiment are identical to those of the first embodiment, and like constituent elements are denoted by like reference numerals or letters.

The monitor 42 is realized by using various displays such as a CRT display or liquid crystal display, and displays various pieces of information instructed to be displayed by the control device 44. Specifically, the monitor 42 displays information useful for a capsule endoscope examination such as an in-vivo image group of a subject captured by the capsule endoscope 2, patient information of the subject, and examination information of the subject, as in the monitor 12 according to the first embodiment. The monitor 42 displays the information useful for magnetic guidance for the capsule endoscope 2 such as current position information and current posture information of the capsule endoscope 2 in the subject, with reference to the absolute coordinate system.

The operating device 43 functions as an operating device that operates the capsule endoscope 2 in the subject with 6 degrees-of-freedom motion, using the magnetic field generator 3 with respect to the capsule endoscope 2 inserted into the subject. The operating device 43 inputs instruction information for instructing desired 6 degrees-of-freedom motion to be performed by the capsule endoscope 2 in the subject to the control device 44, based on one operation or continuous operations by a user such as a doctor or nurse. In this case, the operating device 43 detects respective physical values of motions in three axial directions (forward and backward motion and shifting motion of the 6 degrees-of-freedom motion) of the absolute coordinate system by the linear encoders instead of the force sensor 35, and detects respective physical values of motions around the three axes (rotary motion and direction changing motion of 6 degrees-of-freedom motion) in the absolute coordinate system or operation coordinate system by the rotary encoders. The operating device 43 inputs the respective physical values detected by the linear encoders or rotary encoders to the control device 44 as the instruction information for instructing the desired 6 degrees-of-freedom motion.

The control device 44 displays on the monitor 42 the current position information and current posture information of the capsule endoscope 2 in the subject with reference to the absolute coordinate system, based on the current position information and current posture information of the capsule endoscope 2 acquired from the position and posture detecting device 10. The control device 44 controls the rotary encoders and the linear encoders in the operating device 43 to control drive of a plurality of drive motors (described later) incorporated in the operating device 43. The control device 44 controls an amount of current of the coil power supply 4 with respect to the magnetic field generator 3 based on the instruction information input by the operating device 43, and controls a magnetic field generating motion of the magnetic field generator 3 through the control of the coil power supply 4. Accordingly, the control device 44 controls the 6 degrees-of-freedom motion of the capsule endoscope 2 in the subject. Other functions of the control device 44 are the same as those of the control device 14 according to the first embodiment described above.

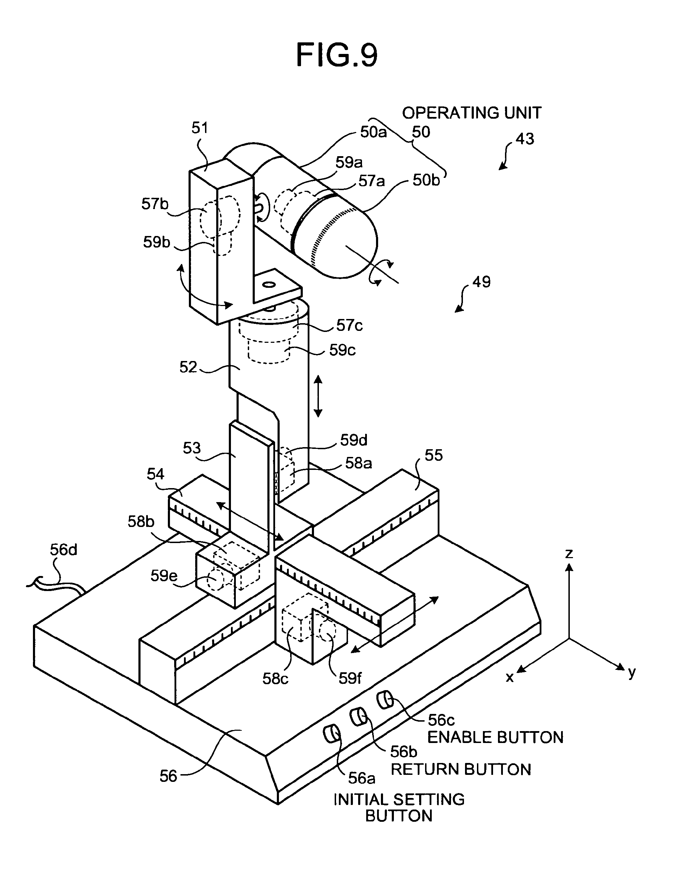

The operating device 43 in the capsule guiding system 41 according to the second embodiment of the present invention is explained next in detail. FIG. 9 is a schematic outline view of a configuration example of the operating device in the capsule guiding system according to the second embodiment of the present invention. As shown in FIG. 9, the operating device 43 according to the second embodiment includes an operating unit 50 for performing one operation or continuous operations corresponding to the desired 6 degrees-of-freedom motion of the capsule endoscope 2, a supporting unit 49 that supports the operating unit 50 so that the 6 degrees-of-freedom motion can be performed, a plurality of rotary encoders 57a to 57c and a plurality of linear encoders 58a to 58c that detect the respective physical values of the desired 6 degrees-of-freedom motion performed by the operating unit 50 based on one operation or continuous operations, and a plurality of drive motors 59a to 59f.

The operating unit 50 is a three-dimensional casing having directionality such as an elliptical or capsule shape, and it is held and operated by a user such as a doctor or nurse at the time of operating the capsule endoscope 2 in the subject with desired 6 degrees-of-freedom motion. Specifically, the operating unit 50 is substantially identical to the capsule endoscope 2 and is a three-dimensional casing with a size holdable by the user. The entirety or a part of the operating unit 30 receives one operation or continuous operations corresponding to the desired 6 degrees-of-freedom motion of the capsule endoscope 2. A casing structure of the operating unit 30 is realized by a body 50a operably supported by the supporting unit 49 and a turning unit 50b rotatably supported by the body.

The body 50a is connected to a shaft of the rotary encoder 57b incorporated in the supporting unit 49. As a result, the body 50a is rotatably supported by the supporting unit 49. The body 50a forms a front-end casing of the capsule operating unit 50, and includes the rotary encoder 57a and the drive motor 59a incorporated therein. On the other hand, the turning unit 50b forms a rear-end casing of the capsule operating unit 50, and is connected to the shaft of the rotary encoder 57a. The turning unit 50b is connected to a shaft of the rotary encoder 57a. The body 50a rotatably supports the turning unit 50b.

An operation coordinate system (see FIG. 4) is defined with respect to the operating unit 50 formed of the body 50a and the turning unit 50b as in the operating unit 30 according to the first embodiment. In this case, the body 50a rotates around a b-axis of the operation coordinate system with respect to a turning support column 51 (described later) of the supporting unit 49. On the other hand, the turning unit 50b rotates around an a-axis of the operation coordinate system with respect to the body 50a.

The supporting unit 49 supports the operating unit 50 so that the operating unit can be operated with 6 degrees-of-freedom motion. Specifically, the absolute coordinate system (see FIG. 1) is defined with respect to the supporting unit 49, and the supporting unit 49 supports the operating unit 50 so that the operating unit 50 can be operated with 6 degrees-of-freedom motion in the defined absolute coordinate system. As shown in FIG. 9, the supporting unit 49 includes the turning support column 51 that rotatably supports the operating unit 50, a movable support column 52 that rotatably supports the turning support column 51, a z-stage 53 that slidably supports the movable support column 52 in a z-axis direction of the absolute coordinate system, a y-stage 54 that slidably supports the z-stage 53 in a y-axis direction of the absolute coordinate system, an x-stage 55 that slidably supports the y-stage 54 in an x-axis direction of the absolute coordinate system, and a support base 56 that fixes and supports the x-stage 55.

The turning support column 51 includes the rotary encoder 57b and drive motor 59b incorporated therein, and rotatably supports the operating unit 50 through a connection between the shaft of the rotary encoder 57b and the body 50a. The turning support column 51 is connected to a shaft of the rotary encoder 57c incorporated in the movable support column 52, and rotates around the z-axis of the absolute coordinate system, assuming the shaft as an axis of rotation.

The movable support column 52 includes the rotary encoder 57c, the linear encoder 58a, and the drive motors 59c and 59d incorporated therein, and rotatably supports the turning support column 51 due to the connection between the shaft of the rotary encoder 57c and the turning support column 51. The movable support column 52 is slidably connected to the z-stage 53, and moves in the z-axis direction of the absolute coordinate system along the z-stage 53.

The z-stage 53 includes the linear encoder 58b and the drive motor 59e, and slidably supports the movable support column 52. The z-stage 53 is slidably connected to the y-stage 54, and moves in the y-axis direction of the absolute coordinate system along the y-stage 54. The y-stage 54 includes the linear encoder 58c and the drive motor 59f, and slidably supports the z-stage 53. The y-stage 54 is slidably connected to the x-stage 55, and moves in the x-axis direction of the absolute coordinate system along the x-stage 55. The x-stage 55 slidably supports the y-stage 54, and is fixed and supported by the support base 56.

A scale indicating a displacement amount of the movable support column 52 in the z-axis direction is added to the z-stage 53, and a scale indicating a displacement amount of the z-stage 53 in the y-axis direction is added to the y-stage 54. A scale indicating a displacement amount of the y-stage 54 in the x-axis direction is added to the x-stage 55.

The support base 56 fixes and supports the x-stage 55, and supports the operating unit 50, the turning support column 51, the movable support column 52, the z-stage 53, and the y-stage 54 via the x-stage 55. The support base 56 includes a predetermined circuit incorporated therein, and includes an initial setting button 56a, a return button 56b, and an enable button 56c.

The initial setting button 56a is an input button for inputting instruction information for setting the operating unit 50, the turning support column 51, the movable support column 52, the z-stage 53, and the y-stage 54 to an initial state (a state corresponding to an initial position and an initial posture of the capsule endoscope 2 at the time of being inserted into a subject) to the control device 44. The return button 56b is an input button for inputting instruction information for returning the operating unit 50, the turning support column 51, the movable support column 52, the z-stage 53, and the y-stage 54 to a state corresponding to a current position and a current posture of the capsule endoscope 2 in the subject to the control device 44. The enable button 56c is an input button for inputting instruction information for switching "valid" and "invalid" of respective detecting processes by the rotary encoders 57a to 57c and the linear encoders 58a to 58c that detect respective physical values of the 6 degrees-of-freedom motion of the operating unit 50 to the control device 44. The pieces of instruction information respectively corresponding to the initial setting button 56a, the return button 56b, and the enable button 56c are input to the control device 44 via a cable 56d.

The operating unit 50 supported by the supporting unit 49 can perform the 6 degrees-of-freedom motion in the absolute coordinate system of the operating device 43 by receiving one operation or continuous operations with respect to the entirety or a part of the operating unit 50 (the body 50a or the turning unit 50b). In this case, the desired 6 degrees-of-freedom motion of the operating unit 50 can be realized by appropriately combining at least one of the rotation of the turning unit 50b, the rotation of the body 50a, the rotation of the turning support column 51, the movement of the movable support column 52, the movement of the z-stage 53, and the movement of the y-stage 54.

The rotary encoder 57a is included in the body 50a, and connected to the turning unit 50b. The rotary encoder 57a detects an amount of rotation and direction of rotation of the turning unit 50b as physical values of a rotary motion around the a-axis. The rotary encoder 57a outputs the detected amount of rotation and direction of rotation of the turning unit 50b to the control device 44 as instruction information of the rotary motion around the X-axis to be performed by the capsule endoscope 2 in the subject. A detection result of the rotary encoder 57a is input to the control device 44 via the cable 56d or the like.

The rotary encoder 57b is included in the turning support column 51, and connected to the body 50a. The rotary encoder 57b detects an amount of rotation and direction of rotation of the body 50a as a physical value of the rotary motion around the b-axis. The rotary encoder 57b outputs the detected amount of rotation and direction of rotation of the body 50a to the control device 44 as instruction information of a direction changing motion around the Y-axis to be performed by the capsule endoscope 2 in the subject. A detection result of the rotary encoder 57b is input to the control device 44 via the cable 56d or the like.

The rotary encoder 57c is included in the movable support column 52, and connected to the turning support column 51. The rotary encoder 57c detects an amount of rotation and direction of rotation of the turning support column 51 as a physical value of the rotary motion around the z-axis. The rotary encoder 57c outputs the detected amount of rotation and direction of rotation of the turning support column 51 to the control device 44 as instruction information of the direction changing motion around the z-axis to be performed by the capsule endoscope 2 in the subject. A detection result of the rotary encoder 57c is input to the control device 44 via the cable 56d or the like.

The linear encoder 58a is included in the movable support column 52, and connected to the z-stage 53. The linear encoder 58a detects a shift amount and shift direction of the movable support column 52 along the z-stage 53 as a physical value of a displacing motion in the z-axis direction. The linear encoder 58a outputs the detected shift amount and shift direction of the movable support column 52 to the control device 44 as instruction information of the shifting motion in the z-axis direction to be performed by the capsule endoscope 2 in the subject. A detection result of the linear encoder 58a is input to the control device 44 via the cable 56d or the like.

The linear encoder 58b is included in the z-stage 53, and connected to the y-stage 54. The linear encoder 58b detects a shift amount and shift direction of the z-stage 53 along the y-stage 54 as a physical value of the displacing motion in the y-axis direction. The linear encoder 58b outputs the detected shift amount and shift direction of the z-stage 53 to the control device 44 as instruction information of the shifting motion in the y-axis direction to be performed by the capsule endoscope 2 in the subject. A detection result of the linear encoder 58b is input to the control device 44 via the cable 56d or the like.

The linear encoder 58c is included in the y-stage 54, and connected to the x-stage 55. The linear encoder 58c detects a shift amount and shift direction of the y-stage 54 along the x-stage 55 as a physical value of the displacing motion in the x-axis direction. The linear encoder 58b outputs the detected shift amount and shift direction of the z-stage 53 to the control device 44 as instruction information of the shifting motion in the x-axis direction to be performed by the capsule endoscope 2 in the subject. A detection result of the linear encoder 58b is input to the control device 44 via the cable 56d or the like.

The drive motors 59a, 59b, and 59c respectively rotate the turning unit 50b, the body 50a, and the turning support column 51 under control of the control device 44. The drive motors 59d, 59e, and 59f linearly drive the movable support column 52, the z-stage 53, and the y-stage 54, respectively, under control of the control device 44. The drive motors 59a, 59b, 59c, 59d, 59e, and 59f can generate a retaining force for maintaining the position and posture of the operating unit 50 when an operator releases the operating unit 50. As a method for generating the retaining force, friction or the like of the respective movable parts can be used.

The control device 44 controls the 6 degrees-of-freedom motion of the capsule endoscope 2 in the subject based on detection results respectively acquired from the rotary encoders 57a to 57c and the linear encoders 58a to 58c (respective physical values of the 6 degrees-of-freedom motion of the operating unit 50). Specifically, the control device 44 performs arithmetic processing for converting an amount of rotation and direction of rotation of the turning unit 50b acquired from the rotary encoder 57a to an amount of rotation and direction of rotation around the X-axis of the capsule coordinate system, and controls the rotary motion (around the X-axis) of the capsule endoscope 2 based on the arithmetic processing result. The control device 44 also performs arithmetic processing for converting an amount of rotation and direction of rotation of the body 50a acquired from the rotary encoder 57b to an amount of rotation and direction of rotation around the Y-axis of the capsule coordinate system, and controls the direction changing motion around the Y-axis of the capsule endoscope 2 based on this arithmetic processing result. Further, the control device 44 performs arithmetic processing for converting an amount of rotation and direction of rotation of the turning support column 51 acquired from the rotary encoder 57c to an amount of rotation and direction of rotation of the capsule endoscope 2 around the z-axis in the absolute coordinate system, and controls the rotary motion around the z-axis of the capsule endoscope 2 based on the arithmetic processing result.

The control device 44 performs arithmetic processing for converting a shift amount and shift direction of the movable support column 52 acquired from the linear encoder 58a to a shift amount and shift direction of the capsule endoscope 2 along the z-axis of the absolute coordinate system, and controls the shifting motion of the capsule endoscope 2 in the z-axis direction based on the arithmetic processing result. The control device 44 also performs arithmetic processing for converting a shift amount and shift direction of the z-stage 53 acquired from the linear encoder 58b to a shift amount and shift direction of the capsule endoscope 2 along the y-axis of the absolute coordinate system, and controls the shifting motion of the capsule endoscope 2 in the y-axis direction based on the arithmetic processing result. Further, the control device 44 performs arithmetic processing for converting a shift amount and shift direction of the y-stage 54 acquired from the linear encoder 58c to a shift amount and shift direction of the capsule endoscope 2 along the X-axis of the absolute coordinate system, and controls the shifting motion of the capsule endoscope 2 in the x-axis direction based on the arithmetic processing result.

The control device 44 can control the desired 6 degrees-of-freedom motion of the capsule endoscope 2 in the absolute coordinate system by appropriately combining the rotary motion and the shifting motion of the capsule endoscope 2 in the absolute coordinate system.

On the other hand, the control device 44 acquires the current position information and posture position information of the capsule endoscope 2 in the absolute coordinate system based on the instruction information input by pressing the initial setting button 56a, and controls drive of the drive motors 59a to 59f so that the acquired current position information and current posture information substantially match or resemble the position and posture of the operating unit 50 in the absolute coordinate system of the operating device 43. As a result, the posture of the operating unit 50 substantially matches the current posture of the capsule endoscope 2 in the subject.

The control device 44 acquires the current position information and posture position information of the capsule endoscope 2 in the absolute coordinate system based on the instruction information input by pressing the return button 56b, and controls the drive of the drive motors 59a to 59f so that the acquired current position information and current posture information substantially match or resemble the position and posture of the operating unit 50 in the absolute coordinate system of the operating device 43, thereby returning the position and posture of the operating unit 50 to the previous position and posture (that is, position and posture respectively match or resemble the current position and current posture of the capsule endoscope 2). In this case, the control device 44 invalidates the physical values respectively detected by the rotary encoders 57a to 57c and the linear encoders 58a to 58c in a process of returning the position and posture of the operating unit 50 to the previous position and posture. As a result, a deviation of the current position and current posture between the capsule endoscope 2 and the operating unit 50 generated when one operation or continuous operations of the operating unit 50 is continued, although the capsule endoscope 2 is stagnating in a digestive tract can be corrected.

The control device 44 validates the physical values respectively detected by the rotary encoders 57a to 57c and the linear encoders 58a to 58c based on the instruction information input by pressing the enable button 56c. The control device 44 then invalidates the physical values respectively detected by the rotary encoders 57a to 57c and the linear encoders 58a to 58c based on the instruction information input by pressing the enable button 56c again. That is, when the enable button 56c is pressed once, the control device 44 validates respective detecting processes of the rotary encoders 57a to 57c and the linear encoders 58a to 58c, and when the enable button 56c is pressed again, the control device 44 invalidates the respective detecting processes of the rotary encoders 57a to 57c and the linear encoders 58a to 58b. As a result, in a process of one operation or continuous operations of the operating unit 50, a relative position between the movable support column 52 and the z-stage 53, a relative position between the z-stage 53 and the y-stage 54, and a relative position between the y-stage 54 and the x-stage 55 can be adjusted to desired relative positions. Accordingly, in the process of one operation or continuous operations of the operating unit 50, a situation where the movable support column 52, the z-stage 53, and the y-stage 54 exceed respective movable ranges (that is, a situation in which one operation or continuous operations of the operating unit 50 cannot be continued) can be prevented.

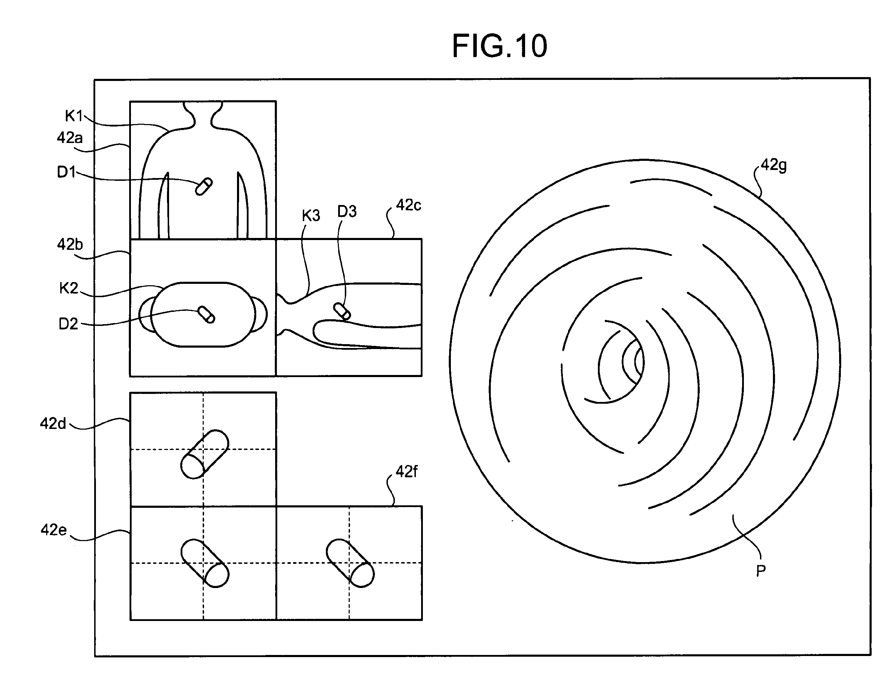

The monitor 42 that displays various pieces of information such as the current position information and current posture information of the capsule endoscope 2 in the subject is explained next in detail. FIG. 10 is a schematic diagram of an example of a display mode of the monitor 42 in the capsule guiding system according to the second embodiment of the present invention. The monitor 42 displays in-vivo images of the subject captured by the capsule endoscope 2, the current position information of the capsule endoscope 2 in the subject, and the current posture information of the capsule endoscope 2 under control of the control device 44.

Specifically, as shown in FIG. 10, the monitor 42 includes position display units 42a to 42c that display the current position information of the capsule endoscope 2 in the subject with reference to the absolute coordinate system, posture display units 42d to 42f that displays the current posture information of the capsule endoscope 2 with reference to the absolute coordinate system, and an image display unit 42g that displays the in-vivo images P of the subject captured by the capsule endoscope 2.

The position display units 42a to 42c display the current position information of the capsule endoscope 2 in the subject with reference to the absolute coordinate system under control of the control device 44. Specifically, the position display unit 42a superposes a capsule image D1 as viewed from the z-axis direction of the absolute coordinate system and the subject image K1 as viewed from the z-axis direction of the absolute coordinate system on each other and displays a superposed image. In this case, the position display unit 42a displays the subject image K1 in a state with a relative direction with respect to a display screen being fixed at all times, and displays the capsule image D1 while changing (updating) the position and direction of the capsule image D1 so that a display position of the capsule image D1 in the subject image K1 is matched with the current position of the capsule endoscope 2 in an xy plane.

The position display unit 42b superposes a capsule image D2 as viewed from the x-axis direction of the absolute coordinate system and the subject image K2 as viewed from the x-axis direction of the absolute coordinate system on each other and displays a superposed image. In this case, the position display unit 42b displays the subject image K2 in a state with a relative direction with respect to the display screen being fixed at all times, and displays the capsule image D2 while changing (updating) the position and direction of the capsule image D2 so that a display position of the capsule image D2 in the subject image K2 is matched with the current position of the capsule endoscope 2 in a yz plane.

The position display unit 42c superposes a capsule image D3 as viewed from the y-axis direction of the absolute coordinate system and the subject image K3 as viewed from the y-axis direction of the absolute coordinate system on each other and displays a superposed image. In this case, the position display unit 42c displays the subject image K3 in a state with a relative direction with respect to the display screen being fixed at all times, and displays the capsule image D3 while changing (updating) the position and direction of the capsule image D3 so that a display position of the capsule image D3 in the subject image K3 is matched with the current position of the capsule endoscope 2 in an xz plane.

The subject images K1 to K3 displayed by the position display units 42a to 42c can be pattern images added with a pattern image of the digestive tract, in which the capsule endoscope 2 in the subject moves, though not specifically shown in FIG. 7. Accordingly, the position display units 42a to 42c can display the current position information of the capsule endoscope 2 in the subject more comprehensively.

The posture display units 42d to 42f display the current posture information of the capsule endoscope 2 in the subject with reference to the absolute coordinate system, under control of the control device 44. Specifically, the posture display unit 42d displays the current posture information of the capsule endoscope 2 as viewed from the z-axis direction of the absolute coordinate system. The posture display unit 42e displays the current posture information of the capsule endoscope 2 as viewed from the x-axis direction of the absolute coordinate system. The posture display unit 42f displays the current posture information of the capsule endoscope 2 as viewed from the y-axis direction of the absolute coordinate system. The posture display units 42d to 42f sequentially update the current posture information of the capsule endoscope 2 to the latest information under control of the control device 44.

The image display unit 42g displays in-vivo images P of the subject captured by the capsule endoscope 2 in the subject under control of the control device 44. The image display unit 42g displays the in-vivo images P of the subject, sequentially changing over the in-vivo image P to a desired in-vivo image, according to an instruction of the control device 44 based on the instruction information input by the input unit 11.

As described above, in the second embodiment of the present invention, the casing having the same directionality as that of the capsule endoscope is provided as the operating unit by having an axis display unit that indicates a specific axial direction of the capsule endoscope by a three-dimensional shape or marking having a three-axis rectangular coordinate system (operation coordinate system) corresponding to the capsule coordinate system defined with respect to the capsule endoscope. The operating unit is supported so that the 6 degrees-of-freedom motion can be realized by the supporting unit movable in the respective axial directions and around the respective axes of the absolute coordinate system. When one operation or continuous operations of the operating unit corresponding to the desired 6 degrees-of-freedom motion of he capsule endoscope is performed to operate the entirety or a part of the operating unit with 6 degrees-of-freedom motion, the three-dimensional amount of rotation and direction of rotation of the operating unit in the absolute coordinate system are detected by the rotary encoders, and the three-dimensional shift amount and shift direction of the operating unit in the absolute coordinate system are detected by the linear encoders. Respective detection results of the rotary encoders and the linear encoders are output as instruction information for instructing the desired 6 degrees-of-freedom motion of the capsule endoscope in the absolute coordinate system. Accordingly, by providing one operation or continuous operations for causing the operating unit to perform the 6 degrees-of-freedom motion in the absolute coordinate system to the operating unit, the capsule endoscope in the subject can perform the desired 6 degrees-of-freedom motion in the absolute coordinate system. As a result, the operating device that can easily operate the capsule endoscope in the subject with at least 3 degrees-of-freedom motion by one operation or continuous operations of the operating unit and the capsule guiding system using the same can be realized.

Because the operating unit has a three-dimensional shape substantially identical to the capsule endoscope and is a holdable size, one operation or continuous operations of the operating unit for causing the capsule endoscope 2 in the subject to perform the desired 6 degrees-of-freedom motion can be easily imaged, by assuming the three-dimensional operating unit as the capsule endoscope in the subject. As a result, one operation or continuous operations of the operating unit for causing the capsule endoscope to perform the desired 6 degrees-of-freedom motion can be easily performed.

Further, because the current position information and current posture information of the capsule endoscope in the subject is displayed on the monitor device with reference to the absolute coordinate system, the relative posture of the capsule endoscope with respect to the subject can be easily operated and the capsule endoscope can be easily magnetically guided to a desired position in the subject by performing one operation or continuous operations of the operating unit while visually checking the current position information and current posture information.

In the second embodiment described above, the rotary encoder is used as a rotation-amount detecting device and the linear encoder is used as a displacement-amount detecting device. However, a displacement measuring device such as a potentiometer can be used instead of the rotary encoder and the linear encoder, or the rotary encoder, the linear encoder, and the potentiometer can be appropriately combined and used.

### Third embodiment

A third embodiment of the present invention is explained next. In the second embodiment, the shift amount and shift direction of the operating unit 50 along the respective axes of the absolute coordinate system are detected by the linear encoders 58a to 58c. However, in the third embodiment, force information (the direction and magnitude of the force) of the operating unit 50 applied along the respective axes of the absolute coordinate system is detected by a three-axis force sensor.

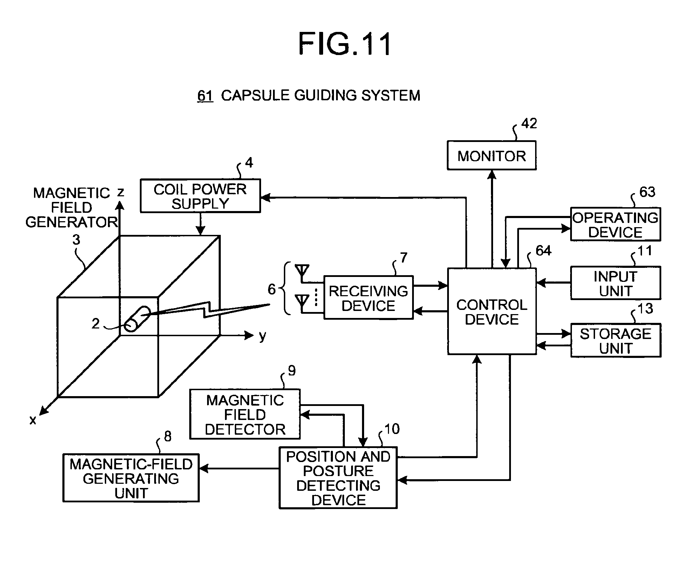

FIG. 11 is a schematic block diagram of a configuration example of a capsule guiding system according to the third embodiment of the present invention. As shown in FIG. 11, a capsule guiding system 61 according to the third embodiment includes an operating device 63 instead of the operating device 43 and a control device 64 instead of the control device 44 in the capsule guiding system 41 according to the second embodiment. Other configurations of the third embodiment are identical to those of the second embodiment, and like constituent elements are denoted by like reference numerals or letters.

The operating device 63 functions as an operating device that operates the capsule endoscope 2 in the subject with 6 degrees-of-freedom motion, using the magnetic field generator 3 with respect to the capsule endoscope 2 inserted into the subject. The operating device 63 inputs instruction information for instructing desired 6 degrees-of-freedom motion to be performed by the capsule endoscope 2 in the subject to the control device 64, based on one operation or continuous operations by a user such as a doctor or nurse. In this case, the operating device 63 detects respective physical values (force information) of motions in three axial directions (forward and backward motion and shifting motion of the 6 degrees-of-freedom motion) of the absolute coordinate system by the three-axis force sensor instead of the linear encoders 58a to 58c. The operating device 63 inputs the respective physical values detected by the three-axis force sensor as instruction information for instructing a motion in the three axial directions of the absolute coordinate system of the 6 degrees-of-freedom motion. Other functions of the operating device 63 are substantially the same as those of the operating device 43 according to the second embodiment.

The control device 64 controls the three-axis force sensor in the operating device 63 based on the instruction information input by pressing the enable button 56c. Because the operating device 63 does not include the drive motors 59d to 59f for respectively driving the movable support column 52, the z-stage 53, and the y-stage 54, the control device 64 does not have a drive control function of the drive motors 59d to 59f. The control device 64 controls the amount of current of the coil power supply 4 to the magnetic field generator 3 based on the instruction information (force information) input by the three-axis force sensor in the operating device 63, and controls the magnetic field generating motion of the magnetic field generator 3 through the control of the coil power supply 4. Accordingly, the control device 64 controls the respective shifting motion and forward and backward motion of the capsule endoscope 2 along the three axial directions (the x-axis direction, y-axis direction, and z-axis direction) in the absolute coordinate system. Other functions of the control device 64 are the same as those of the control device 44 in the second embodiment.

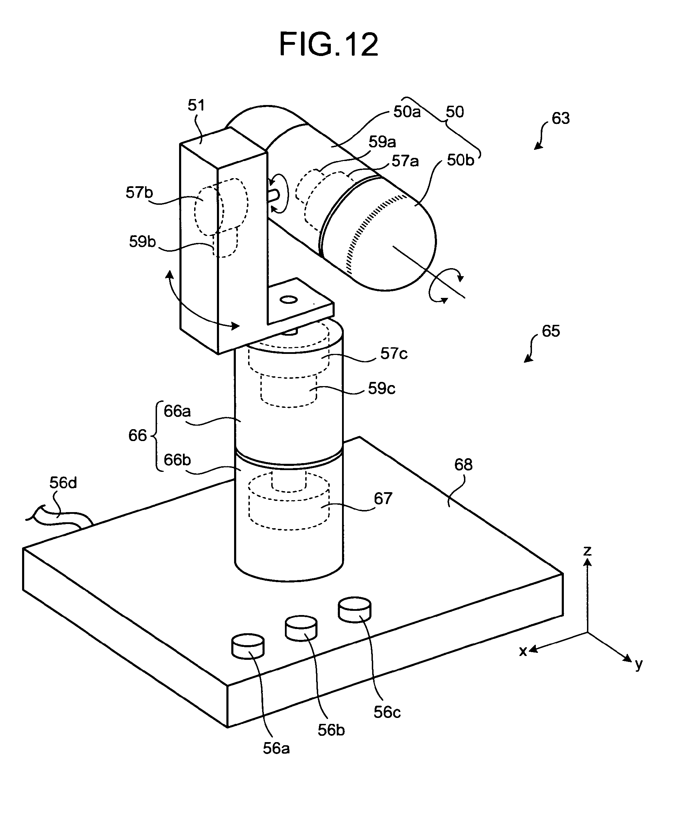

The operating device 63 in the capsule guiding system 61 according to the third embodiment of the present invention is explained next in detail. FIG. 12 is a schematic outline view of a configuration example of the operating device in the capsule guiding system according to the third embodiment of the present invention. As shown in FIG. 12, the operating device 63 according to the third embodiment includes a supporting unit 65 instead of the supporting unit 49 and a force sensor 67 instead of the linear encoders 58a to 58c of the operating device 43 (see FIG. 9) according to the second embodiment. In this case, the operating device 63 does not include the drive motors 59d to 59f. The supporting unit 65 includes a support column 66 and a support base 68 instead of the movable support column 52, the z-stage 53, the y-stage 54, the x-stage 55, and the support base 56 of the operating device 43 according to the second embodiment.

The support column 66 operably supports the operating unit 50 along the three axial directions (the x-axis direction, y-axis direction, and z-axis direction) of the absolute coordinate system. Specifically, one end of the support column 66 is fixed and supported by the support base 68, and the turning support column 51 is rotatably connected to the other end. The support column 66 supports the operating unit 50 via the turning support column 51. The support column 66 includes a movable unit 66a capable of being displaced in the absolute coordinate system, and a fixed unit 66b that operably supports the movable unit 66a.

The movable unit 66a includes the rotary encoder 57c and the drive motor 59c incorporated therein, and rotatably supports the turning support column 51 by a connection between the rotary encoder 57c and the turning support column 51. The movable unit 66a is connected to the force sensor 67 (described later) incorporated in the fixed unit 66b, and can be displaced in a desired direction in the absolute coordinate system by one operation or continuous operations of the operating unit 50.

One end of the fixed unit 66b is fixed and supported by the support base, and the force sensor 67 is incorporated in the fixed unit 66b near the other end thereof. The fixed unit 66b operably supports the movable unit 66a by a connection between a shaft (not shown) of the force sensor 67 and the movable unit 66a. The fixed unit 66b is fixed with respect to a displacing motion of the movable unit 66a in the absolute coordinate system. That is, even if the movable unit 66a is displaced, the fixed unit 66b hardly moves and maintains the fixed state with respect to the support base 68.

The force sensor 67 is a three-axis force sensor, and detects respective pieces of force information (an example of physical values) of the displacing motion of the movable unit 66a as respective axial components of the absolute coordinate system. Specifically, the force sensor 67 is connected to the movable unit 66a via the shaft thereof (not shown), and receives an external force applied to the movable unit 66a by one operation or continuous operations of the operating unit 50 via the shaft. The force sensor 67 is also connected to the control device 64 via the cable 56d. The force sensor 67 detects force information such as a magnitude and direction of the external force of the movable unit 66a transmitted via the shaft. In this case, the force sensor 67 detects respective force components in the x-axis direction, y-axis direction, and z-axis direction of the external force applied to the movable unit 66a. The force sensor 67 transmits the thus detected force information of the external force applied to the movable unit 66a to the control device 64. The force information detected by the force sensor 67 is input to the control device 64 as instruction information for instructing the shifting motion or forward and backward motion of the capsule endoscope 2 in the absolute coordinate system.

The support base 68 fixes and supports the fixed unit 66b, and supports the operating unit 50, the turning support column 51, and the movable unit 66a via the fixed unit 66b. The support base 68 includes a built-in predetermined circuit as in the support base 56 of the operating device 43 according to the second embodiment, and includes the initial setting button 56a, the return button 56b, and the enable button 56c.

The operating unit 50 supported by the supporting unit 65 can perform the 6 degrees-of-freedom motion in the absolute coordinate system of the operating device 63 by receiving one operation or continuous operations with respect to the entirety or a part of the operating unit 50 (the body 50a or the turning unit 50b). In this case, the desired 6 degrees-of-freedom motion of the operating unit 50 can be realized by appropriately combining at least one of the rotation of the turning unit 50b, the rotation of the body 50a, the rotation of the turning support column 51, and the displacement of the movable unit 66a.

The control device 64 performs arithmetic processing for converting the force information (the magnitude and direction of the force) of the movable unit 66a acquired from the force sensor 67 to a driving force and a driving direction (a shift direction or a forward and backward direction) of the capsule endoscope 2 in the absolute coordinate system, and controls the shifting motion and the forward and backward motion of the capsule endoscope 2 in the absolute coordinate system based on an arithmetic processing result. The control device 64 can control the desired 6 degrees-of-freedom motion of the capsule endoscope 2 in the absolute coordinate system by appropriately combining the rotary motion and the shifting motion of the capsule endoscope 2 in the absolute coordinate system.

The control device 64 validates the physical values respectively detected by the rotary encoders 57a to 57c and the force sensor 67 based on the instruction information input by pressing the enable button 56c. The control device 64 then invalidates the physical values respectively detected by the rotary encoders 57a to 57c and the force sensor 67 based on the instruction information input by pressing the enable button 56c again. That is, when the enable button 56c is pressed once, the control device 64 validates the detecting process of the rotary encoders 57a to 57c and the force sensor 67, and when the enable button 56c is pressed again, the control device 64 invalidates the detecting process of the rotary encoders 57a to 57c and the force sensor 67.

As described above, in the third embodiment, the three-axis force sensor is provided instead of the linear encoders of the operating device according to the second embodiment. The three-axis force sensor detects the force information of the external force applied by the displacing motion of the operating unit in the absolute coordinate system, and the force information detected by the three-axis force sensor is output as the instruction information instruction the shifting motion or the forward and backward motion of the capsule endoscope 2 in the absolute coordinate system, and other parts of the configuration are substantially the same as those of the second embodiment. Accordingly, the operating device that can achieve the same operations and effects as those of the second embodiment and can promote downsizing of the apparatus without requiring the x-stage, the y-stage, and the z-stage corresponding to the respective axes of the absolute coordinate system, and the capsule guiding system using the same can be realized with a simple configuration.

### Fourth embodiment

A fourth embodiment of the present invention is explained next. In the first embodiment, the movable unit 32, which is a part of the operating unit 30 supported by the support base 33, is operated once or continuously to operate the capsule endoscope 2 in the subject with 6 degrees-of-freedom motion. However, in the fourth embodiment, the entire operating unit holdable by the user is moved to perform one operation or continuous operations for operating the capsule endoscope 2 with 6 degrees-of-freedom motion.

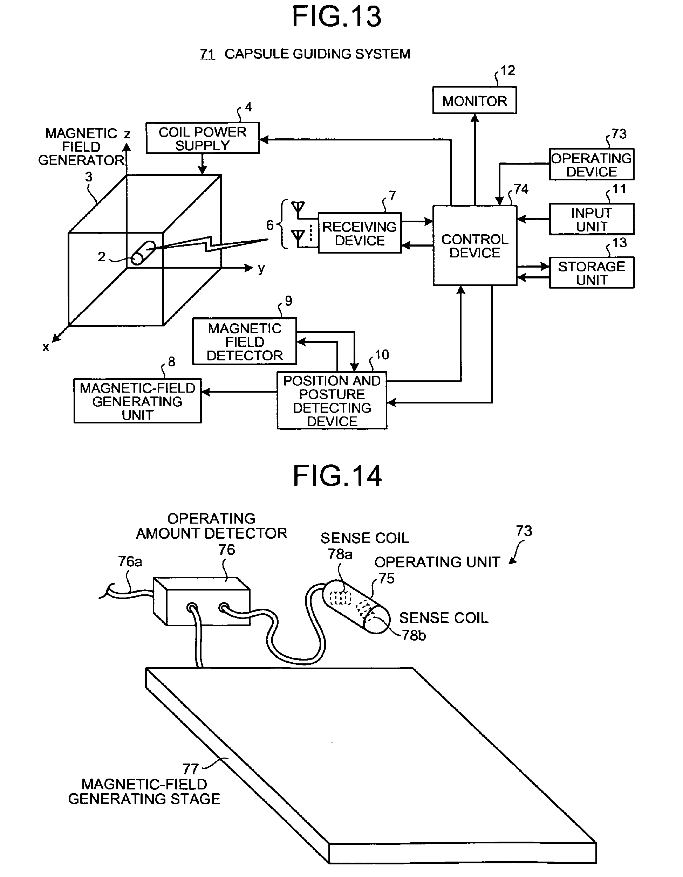

FIG. 13 is a schematic block diagram of a configuration example of a capsule guiding system according to the fourth embodiment of the present invention. As shown in FIG. 13, a capsule guiding system 71 according to the fourth embodiment includes an operating device 73 instead of the operating device 5 and a control device 74 instead of the control device 14 in the capsule guiding system 1 according to the first embodiment. Other configurations of the fourth embodiment are identical to those of the first embodiment, and like constituent elements are denoted by like reference numerals or letters.

The operating device 73 functions as an operating device that uses the magnetic field generator 3 with respect to the capsule endoscope 2 inserted into the subject to operate the capsule endoscope 2 in the subject with 6 degrees-of-freedom motion. The operating device 73 inputs instruction information for instructing desired 6 degrees-of-freedom motion to be performed by the capsule endoscope 2 in the subject to the control device 74 based on one operation or continuous operations by a user such as a doctor or nurse. Details of the operating device 73 will be described later.

The control device 74 controls an amount of current of the coil power supply 4 with respect to the magnetic field generator 3 based on the instruction information input by the operating device 73, that is, the respective physical values of the 6 degrees-of-freedom motion to be performed by an operating unit 75 according to one operation or continuous operations using the entire operating unit 75 of the operating device 73, and controls a magnetic field generating motion of the magnetic field generator 3 through the control of the coil power supply 4. Accordingly, the control device 74 controls the 6 degrees-of-freedom motion of the capsule endoscope 2 in the subject. Other functions of the control device 74 are the same as those of the control device 14 according to the first embodiment.

The 6 degrees-of-freedom motion of the capsule endoscope 2 controlled by the control device 74 is a forward and backward motion in the X-axis direction, a shifting motion in the Y-axis direction, a shifting motion in the Z-axis direction, a rotary motion around the X-axis, a direction changing motion around the Y-axis, and a direction changing motion around the Z-axis of the capsule coordinate system. The control device 74 can control the three-dimensional 6 degrees-of-freedom motion of the capsule endoscope 2 in the subject by appropriately combining these motions.

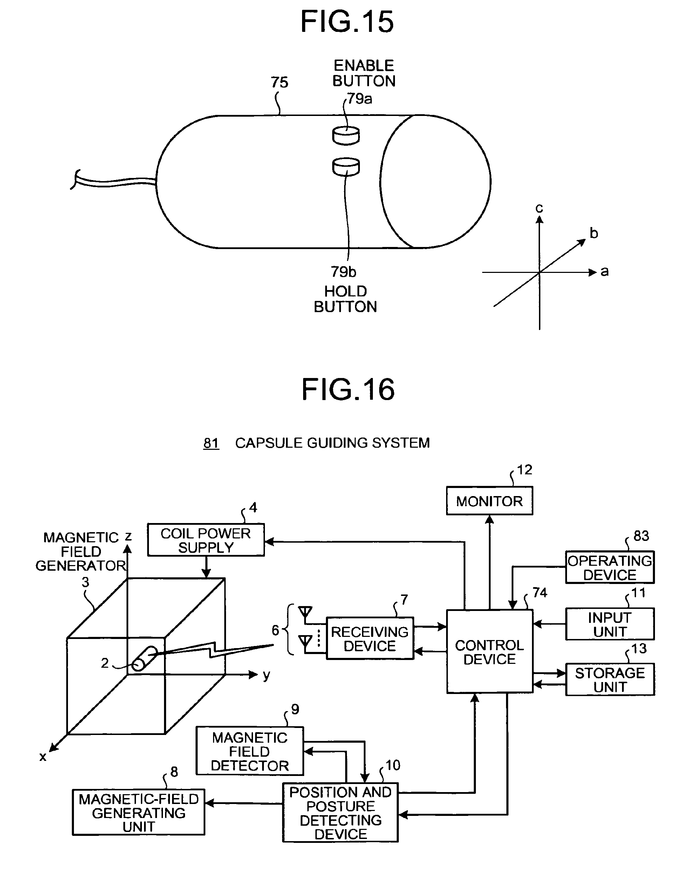

The operating device 73 in the capsule guiding system 71 according to the fourth embodiment of the present invention is explained next in detail. FIG. 14 is a schematic outline view of a configuration example of the operating device in the capsule guiding system according to the fourth embodiment of the present invention. FIG. 15 is a schematic diagram of an outline of the operating unit of the operating device according to the fourth embodiment of the present invention. As shown in FIGS. 14 and 15, the operating device 73 according to the fourth embodiment includes the operating unit 75 for performing one operation or continuous operations corresponding to the desired 6 degrees-of-freedom motion of the capsule endoscope 2, an operating amount detector 76 that detects an operating amount (an example of the physical value) of the operating unit 75 operated with 6 degrees-of-freedom motion according to such one operation or continuous operations, and a magnetic-field generating stage 77 that generates the magnetic field in a space domain in which the operating unit 75 is operated with 6 degrees-of-freedom motion.

The operating unit 75 is a three dimensional casing having directionality such as an elliptical or capsule shape, and is operated by a user such as a doctor or nurse when the capsule endoscope 2 in the subject performs desired 6 degrees-of-freedom motion. Specifically, the operating unit 30 is substantially identical to the capsule endoscope 2 and is a three-dimensional casing with a size holdable by the user. The operating unit 75 held by the user is operated once or continuously corresponding to the desired 6 degrees-of-freedom motion of the capsule endoscope 2 in the presence of the magnetic field generated by the magnetic-field generating stage 77 described later, to move the entire casing with 6 degrees-of-freedom motion. The operating unit 75 includes a plurality of sense coils 78a and 78b incorporated therein, and an enable button 79a for enabling or disabling a magnetic field detecting process by the sense coils 78a and 78b and a hold button 79b for holding the operating amount of the operating unit 75 on an external wall thereof. The operation coordinate system is defined with respect to the operating unit (see FIG. 15) as in the operating unit 30 of the operating device 5 according to the first embodiment. The operating unit 75 includes a predetermined circuit (not shown), and is connected to the operating amount detector 76 via a cable.

The sense coils 78a and 78b detect the magnetic field generated by the magnetic-field generating stage 77. Specifically, the sense coils 78a and 78b are fixed and arranged in the operating unit 75 so that each coil shaft thereof is arranged, slanted with respect to each other, to form an inverted V-shape. The sense coils 78a and 78b arranged in this manner can detect the magnetic field (a magnetic field generated by the magnetic-field generating stage 77) from an arbitrary direction in the operation coordinate system defined with respect to the operating unit 75. A magnetic field detection result acquired by the sense coils 78a and 78b is transmitted to the operating amount detector 76 via a cable or the like in a state with the enable button 79a being pressed. That is, when the enable button 79a is not pressed, the magnetic field detection result of the sense coils 78a and 78b are not transmitted to the operating amount detector 76.

The number of sense coils incorporated in the operating unit 75 is not limited to two, and can be three or more so long as the number of sense coils is sufficient for detecting the magnetic field generated by the magnetic-field generating stage 77. The arrangement of the sense coil is not limited to an inverted V-shape, and can be a state where the respective coil shafts are not parallel to each other. For example, the arrangement of the sense coils can be such that projection lines of the coil shafts are orthogonal to each other, or the coil shafts are in a twisted position.

The operating amount detector 76 acquires the magnetic field detection result of the sense coils 78a and 78b from the operating unit 75, to detect the respective operating amounts of the 6 degrees-of-freedom motion of the operating unit 75 based on the acquired magnetic field detection result. In this case, the operating amount detector 76 detects the three-dimensional operating amount of the operating unit 75 in the operation coordinate system defined with respect to the operating unit 75. The operating amount detector 76 outputs the respective axial components of the detected operating amounts of the operating unit 75, that is, a shift amount in the a-axis direction, a shift amount in the b-axis direction, a shift amount in the c-axis direction, an amount of rotation around the a-axis, an amount of rotation around the b-axis, and an amount of rotation around the c-axis as the instruction information of the 6 degrees-of-freedom motion of the capsule endoscope 2. The operating amount detector 76 is connected to the control device 74 via a cable 76a, and the respective axial components (instruction information) of the operating amounts detected by the operating amount detector 76 are input to the control device 74 via the cable 76a.

In a state with the enable button 79a being pressed, the operating amount detector 76 acquires the magnetic field detection result of the sense coils 78a and 78b, and in a state with the enable button 79a not being pressed, the operating amount detector 76 does not acquire the magnetic field detection result. That is, the operating amount detector 76 detects the respective operating amounts of the operating unit 75 only in the state with the enable button 79a being pressed, and transmits the detected respective operating amounts to the control device 74.

When the instruction information is input by pressing the hold button 79b, the operating amount detector 76 stores and holds the respective operating amounts of the operating unit 75 detected immediately before, based on the instruction information corresponding to the hold button 79b. In this case, the operating amount detector 76 continuously transmits the respective operating amounts of the operating unit 75 held therein to the control device 74. The control device 74 causes the capsule endoscope 2 to continuously perform the 6 degrees-of-freedom motion corresponding to the operating amounts continuously held by the operating amount detector 76. The control by the control device 74 to cause the capsule endoscope 2 to continuously perform the 6 degrees-of-freedom motion is continued until the operating amount detector 76 stops holding the respective operating amounts of the operating unit 75, that is, the hold button 79b is pressed again, or pressing of the hold button 79b is released.

The control device 74 processes the shift amount in the a-axis direction as the shift amount in the X-axis direction of the capsule coordinate system (the shift amount of the forward and backward motion of the capsule endoscope 2), processes the shift amount in the b-axis direction as the shift amount in the Y-axis direction of the capsule coordinate system (the shift amount of the shifting motion of the capsule endoscope 2 in the Y-axis direction), and processes the shift amount in the c-axis direction as the shift amount in the Z-axis direction of the capsule coordinate system (the shift amount of the shifting motion of the capsule endoscope 2 in the Z-axis direction), of the operating amounts of the operating unit 75 acquired from the operating amount detector 76. Further, the control device 74 processes the amount of rotation around the a-axis as the amount of rotation around the X-axis of the capsule coordinate system (the amount of rotation of the rotary motion of the capsule endoscope 2), processes the amount of rotation around the b-axis as the amount of rotation around the Y-axis of the capsule coordinate system (the amount of rotation of the direction changing motion of the capsule endoscope 2 around the Y-axis), and processes the amount of rotation around the c-axis as the amount of rotation around the Z-axis of the capsule coordinate system (the amount of rotation of the direction changing motion of the capsule endoscope 2 around the Z-axis).

The magnetic-field generating stage 77 generates the magnetic field in a space where one operation or continuous operations of the operating unit 75 for operating the capsule endoscope 2 in the subject with desired 6 degrees-of-freedom motion is performed. Specifically, the magnetic-field generating stage 77 is connected to the operating amount detector 76 via the cable, and generates the magnetic field by an alternating current supplied by the operating amount detector 76. The magnetic field generated by the magnetic-field generating stage 77 is formed in the space on the magnetic-field generating stage 77, and is detected by the sense coils 78a and 78b in the operating unit 75 operated once or continuously. The magnetic field generation timing of the magnetic-field generating stage 77 is controlled by the operating amount detector 76. For example, the operating amount detector 76 acquires the instruction information when the enable button 79a is pressed, and supplies the alternating current to the magnetic-field generating stage 77 to generate the magnetic field based on the acquired instruction information.

The operating device 73 having such a configuration can provide the desired 6 degrees-of-freedom motion of the capsule endoscope 2 in the subject (that is, the operating device 73 can cause the capsule endoscope 2 in the subject to operate with desired 6 degrees-of-freedom motion) by providing one operation or continuous operations to the entire operating unit 75. Specifically, when the enable button 79a is pressed and the operating unit 75 is held by a user, the operating device 73 can input the respective operating amounts (that is, the instruction information of 6 degrees-of-freedom motion) of the operating unit 75 corresponding to the desired 6 degrees-of-freedom motion of the capsule endoscope 2 to the control device 74, by receiving one operation or continuous operations for operating the operating unit 75 with 6 degrees-of-freedom motion. In this case, one operation or continuous operations of the operating unit 75 held by the user is performed in the presence of the magnetic field generated by the magnetic-field generating stage 77, assuming the operation coordinate system of the operating unit 75 as the capsule coordinate system of the capsule endoscope 2.

As described above, in the fourth embodiment of the present invention, because the three-axis rectangular coordinate system (operation coordinate system) corresponding to the capsule coordinate system defined with respect to the capsule endoscope is provided, the three-dimensional casing having the same directionality as the capsule endoscope is provided as the operating unit, and the entire operating unit is operated with 6 degrees-of-freedom motion according to one operation or continuous operations. When the operating unit is operated with 6 degrees-of-freedom motion by performing one operation or continuous operations of the operating unit corresponding to desired 6 degrees-of-freedom motion of the capsule endoscope in the presence of the magnetic field, the magnetic field applied to the operating unit is detected by the sense coils in the operating unit, and the respective operating amounts of the 6 degrees-of-freedom motion of the operating unit are detected based on a magnetic field detection result of the sense coils. The detected respective operating amounts are output as the instruction information for instructing the desired 6 degrees-of-freedom motion of the capsule endoscope. Other parts of the configuration are the same as those in the first embodiment. Accordingly, the same operations and effects as those of the first embodiment can be achieved, and by providing one operation or continuous operations for causing the operating unit to perform the 6 degrees-of-freedom motion in the operation coordinate system to the operating unit, the capsule endoscope in the subject can perform the desired 6 degrees-of-freedom motion. As a result, the operating device that can easily operate the capsule endoscope in the subject with 6 degrees-of-freedom motion by one operation or continuous operations of the operating unit and the capsule guiding system using the same can be realized.

Because the operating unit has a three-dimensional shape substantially identical to the capsule endoscope and is a holdable size, one operation or continuous operations of the operating unit for causing the capsule endoscope 2 in the subject to perform the desired 6 degrees-of-freedom motion can be easily imaged, by assuming the three-dimensional operating unit as the capsule endoscope in the subject. As a result, one operation or continuous operations of the operating unit for causing the capsule endoscope to perform the desired 6 degrees-of-freedom motion can be easily performed.

### Fifth embodiment

A fifth embodiment of the present invention is explained next. In the fourth embodiment, one operation or continuous operations of the operating unit 75 is performed in the presence of the magnetic field, the magnetic field to be applied to the operating unit 75 is detected by the sense coils 78a and 78b at the time of one operation or continuous operations, and the respective operating amounts of the operating unit 75 are detected based on the magnetic field detection result. However, in the fifth embodiment, an acceleration sensor is incorporated in the operating unit to detect an acceleration of the operating unit generated by one operation or continuous operations of the operating unit by the acceleration sensor, and the respective operating amounts of the operating unit are detected based on the detected acceleration of the operating unit.

FIG. 16 is a schematic block diagram of a configuration example of a capsule guiding system according to the fifth embodiment of the present invention. As shown in FIG. 16, a capsule guiding system 81 according to the fifth embodiment includes an operating device 83 instead of the operating device 73 in the capsule guiding system 71 according to the fourth embodiment. Other configurations of the fifth embodiment are identical to those of the fourth embodiment, and like constituent elements are denoted by like reference numerals or letters.

The operating device 83 functions as an operating device that operates the capsule endoscope 2 in the subject with 6 degrees-of-freedom motion, using the magnetic field generator 3 with respect to the capsule endoscope 2 inserted into the subject. The operating device 83 inputs instruction information for instructing desired 6 degrees-of-freedom motion to be performed by the capsule endoscope 2 in the subject to the control device 74, based on one operation or continuous operations by a user such as a doctor or nurse.

In the fifth embodiment, the control device 74 controls the 6 degrees-of-freedom motion of the capsule endoscope 2 in the subject based on the instruction information input by the operating device 83, that is, respective physical values of the 6 degrees-of-freedom motion performed by an operating unit 85 according to one operation or continuous operations using the entire operating unit 85 of the operating device 83 described later.

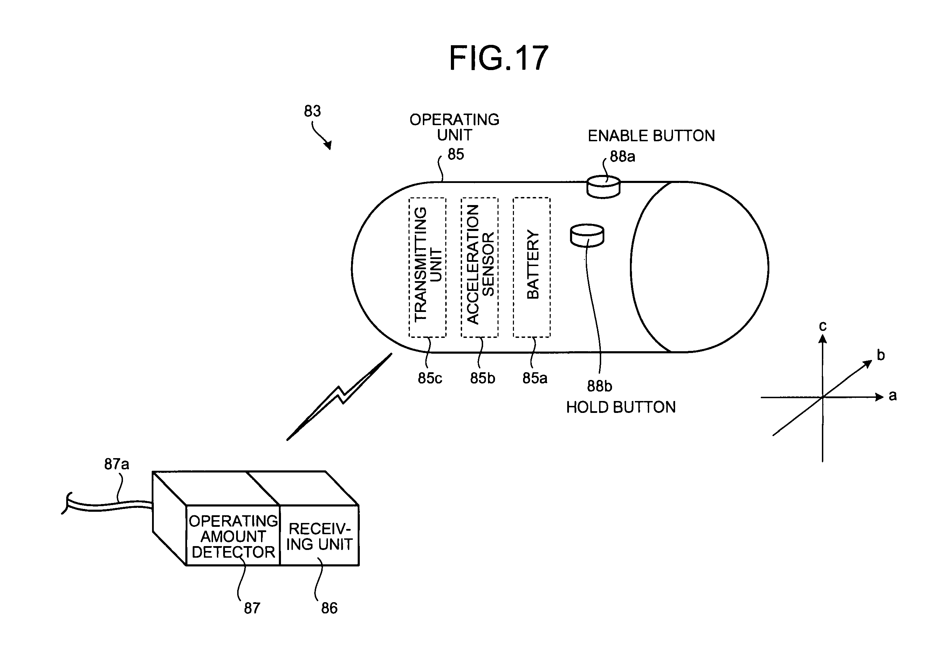

The operating device 83 in the capsule guiding system 81 according to the fifth embodiment of the present invention is explained next in detail. FIG. 17 is a schematic outline view of a configuration example of the operating device in the capsule guiding system according to the fifth embodiment of the present invention. As shown in FIG. 17, the operating device 83 according to the fifth embodiment includes the operating unit 85 for performing one operation or continuous operations corresponding to the desired 6 degrees-of-freedom motion of the capsule endoscope 2, a receiving unit 86 that receives information wirelessly transmitted from the operating unit 85, and an operating amount detector 87 that detects the respective operating amounts (an example of physical values) of the operating unit 85 based on the information (acceleration detection result of an acceleration sensor 85b described later) acquired from the operating unit 85 via the receiving unit 86.

The operating unit 85 is a three-dimensional casing having directionality such as an elliptical or capsule shape, and it is operated by a user such as a doctor or nurse at the time of operating the capsule endoscope 2 in the subject with 6 degrees-of-freedom motion. Specifically, the operating unit 85 is substantially identical to the capsule endoscope 2 and is a three-dimensional casing with a size holdable by the user. The operating unit 85 held by the user is operated once or continuously corresponding to the desired 6 degrees-of-freedom motion of the capsule endoscope 2. The operating unit 85 includes therein a battery 85a that supplies electric power to the acceleration sensor 85b and a transmitting unit 85c, the acceleration sensor 85b that detects the acceleration of the operating unit 85, and the transmitting unit 85c that wirelessly transmits an acceleration detection result of the acceleration sensor 85b to the receiving unit 86. The operating unit 85 also includes an enable button 88a for enabling or disabling an acceleration detecting process by the acceleration sensor 85b, and a hold button 88b for holding the operating amount of the operating unit 85 on an external wall thereof. Further, the operation coordinate system is defined with respect to the operating unit 85 similarly to the operating unit 75 of the operating device 73 according to the fourth embodiment.

The acceleration sensor 85b detects the acceleration of the operating unit 85 operated once or continuously for operating the capsule endoscope 2 with desired 6 degrees-of-freedom motion. In this case, the acceleration sensor 85b detects an acceleration vector in a desired displacement direction or rotation direction in the operation coordinate system defined with respect to the operating unit 85. The acceleration sensor 85b detects the acceleration vector of the operating unit 85, in a state with the enable button 88a being pressed, and transmits the acceleration detection result to the transmitting unit 85c. On the other hand, when the enable button 88a is not pressed, the acceleration sensor 85b does not detect the acceleration vector of the operating unit 85. That is, the acceleration detecting process by the acceleration sensor 85b is valid in a state with the enable button 79a being pressed, and not valid in a state with the enable button 79a not being pressed.

The transmitting unit 85c wirelessly transmits the acceleration detection result of the acceleration sensor 85b to the receiving unit 86. Specifically, the transmitting unit 85c acquires the information including the acceleration vector of the operating unit 85 detected by the acceleration sensor 85b from the acceleration sensor 85b, and performs a predetermined modulation process or the like with respect to the acquired information to generate a radio signal including the information (acceleration vector of the operating unit 85). The transmitting unit 85c transmits the generated radio signal to the receiving unit 86. When the instruction information is input to the transmitting unit 85c by pressing the hold button 88b, the transmitting unit 85c generates the radio signal including the instruction information, and transmits the generated radio signal to the receiving unit 86.

The receiving unit 86 receives the radio signal transmitted from the transmitting unit 85c, and performs a predetermined demodulation process or the like with respect to the received radio signal to extract the acceleration detection result from the radio signal. The receiving unit 86 transmits the extracted acceleration detection result to the operating amount detector 87. The acceleration detection result extracting on the receiving unit 86 indicates the acceleration vector of the operating unit 85 detected by the acceleration sensor 85b. The receiving unit 86 extracts the instruction information corresponding to the hold button 88b based on the radio signal received from the transmitting unit 85c, and transmits the extracted instruction information to the operating amount detector 87.

The operating amount detector 87 functions as a detecting unit that detects the respective operating amounts (an example of physical values of 6 degrees-of-freedom motion) of the operating unit in the operation coordinate system based on the acceleration vector of the operating unit 85 detected by the acceleration sensor 85b. Specifically, the operating amount detector 87 acquires the acceleration detection result extracting on the receiving unit 86, and detects the respective operating amounts of the 6 degrees-of-freedom motion of the operating unit 85 based on the acceleration vector (that is, the acceleration vector detected by the acceleration sensor 85b) of the operating unit 85 corresponding to the acquired acceleration detection result. In this case, the operating amount detector 87 detects the three-dimensional operating amounts of the operating unit 85 in the operation coordinate system defined with respect to the operating unit 85. The operating amount detector 87 outputs the respective axial components of the detected operating amounts of the operating unit 85, that is, a shift amount in the a-axis direction, a shift amount in the b-axis direction, a shift amount in the c-axis direction, an amount of rotation around the a-axis, an amount of rotation around the b-axis, and an amount of rotation around the c-axis as the instruction information of the 6 degrees-of-freedom motion of the capsule endoscope 2. The operating amount detector 87 is connected to the control device 74 via a cable 87a, and the respective axial components (instruction information) of the operating amounts detected by the operating amount detector 87 are input to the control device 74 via the cable 87a.

In a state with the enable button 88a being pressed, the operating amount detector 87 acquires the acceleration detection result of the acceleration sensor 85b, and in a state with the enable button 88a not being pressed, the operating amount detector 87 does not acquire the acceleration detection result. That is, the operating amount detector 87 detects the respective operating amounts of the operating unit 85 only in the state with the enable button 88a being pressed, and transmits the detected respective operating amounts to the control device 74.

The operating amount detector 87 acquires the instruction information corresponding to the hold button 88b via the receiving unit 86, and stores and holds the respective operating amounts of the operating unit 85 detected immediately before, based on the instruction information corresponding to the hold button 88b. In this case, the operating amount detector 87 continuously transmits the held operating amounts of the operating unit 85 to the control device 74. The control device 74 causes the capsule endoscope 2 to continuously perform the 6 degrees-of-freedom motion corresponding to the operating amounts continuously held by the operating amount detector 87. The control by the control device 74 to cause the capsule endoscope 2 to continuously perform the 6 degrees-of-freedom motion is continued until the operating amount detector 87 stops holding the respective operating amounts of the operating unit 75, that is, the hold button 88b is pressed again, or pressing of the hold button 88b is released.

The control device 74 processes the shift amount in the a-axis direction as the shift amount in the X-axis direction of the capsule coordinate system (the shift amount of the forward and backward motion of the capsule endoscope 2), processes the shift amount in the b-axis direction as the shift amount in the Y-axis direction of the capsule coordinate system (the shift amount of the shifting motion of the capsule endoscope 2 in the Y-axis direction), and processes the shift amount in the c-axis direction as the shift amount in the Z-axis direction of the capsule coordinate system (the shift amount of the shifting motion of the capsule endoscope 2 in the Z-axis direction), of the operating amounts of the operating unit 85 acquired from the operating amount detector 87. Further, the control device 74 processes the amount of rotation around the a-axis as the amount of rotation around the X-axis of the capsule coordinate system (the amount of rotation of the rotary motion of the capsule endoscope 2), processes the amount of rotation around the b-axis as the amount of rotation around the Y-axis of the capsule coordinate system (the amount of rotation of the direction changing motion of the capsule endoscope 2 around the Y-axis), and processes the amount of rotation around the c-axis as the amount of rotation around the Z-axis of the capsule coordinate system (the amount of rotation of the direction changing motion of the capsule endoscope 2 around the Z-axis).

The operating device 83 having such a configuration can provide the desired 6 degrees-of-freedom motion of the capsule endoscope 2 in the subject (that is, the operating device 83 can cause the capsule endoscope 2 in the subject to operate with desired 6 degrees-of-freedom motion) by providing one operation or continuous operations to the entire operating unit 75. Specifically, when the enable button 88a is pressed and the operating unit 85 is held by the user, the operating device 83 can input the respective operating amounts (that is, the instruction information of 6 degrees-of-freedom motion) of the operating unit 75 corresponding to the desired 6 degrees-of-freedom motion of the capsule endoscope 2 to the control device 74, by receiving one operation or continuous operations for operating the operating unit 85 with 6 degrees-of-freedom motion. In this case, one operation or continuous operations of the operating unit 85 held by the user is performed, assuming the operation coordinate system of the operating unit 85 as the capsule coordinate system of the capsule endoscope 2.

As described above, in the fifth embodiment of the present invention, the acceleration sensor is incorporated in the operating unit to be operated with 6 degrees-of-freedom motion by one operation or continuous operations, to detect the acceleration vector of the operating unit at the time of performing the 6 degrees-of-freedom motion, the respective operating amounts of the 6 degrees-of-freedom motion of the operating unit are detected based on the detected acceleration vector of the operating unit, and the detected respective operating amounts are output as the instruction information for instructing the desired 6 degrees-of-freedom motion of the capsule endoscope. Other parts of the configuration are substantially the same as those in the fourth embodiment. Accordingly, the fifth embodiment can achieve the same operations and effects as those of the fourth embodiment, and the operating device can detect the respective operating amounts of the 6 degrees-of-freedom motion of the operating unit without performing one operation or continuous operations for causing the operating unit to perform the 6 degrees-of-freedom motion in the presence of the magnetic field. As a result, an operating device that can promote downsizing of the entire device and a capsule guiding system using the operating device can be realized with a simpler configuration.

Because the acceleration detection result of the operating unit by the acceleration sensor is wirelessly transmitted to the outside of the operating unit, the operating amount detector that detects the respective operating amounts of the operating unit need not be connected to the operating unit. As a result, one operation or continuous operations using the operating unit can be performed more easily without being blocked by a cable or the like.

### Sixth embodiment

A sixth embodiment of the present invention is explained next. In the first embodiment, the current position information and current posture information of the capsule endoscope 2 in the subject are displayed on the monitor by superposing the capsule images D1 to D3 on the subject images K1 to K3, respectively. However, in the sixth embodiment, an input amount of the operating device 5 at the time of magnetically guiding the capsule endoscope 2 in a subject, and an operating state of magnetic guidance for the capsule endoscope 2 such as actions and effects of the magnetic field to be applied from the magnetic field generator 3 to the capsule endoscope 2 corresponding to the input amount are displayed on a monitor.

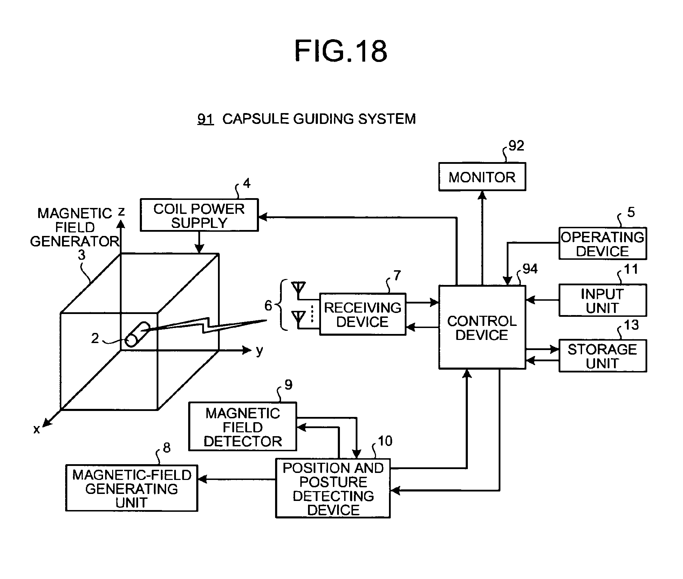

FIG. 18 is a schematic block diagram of a configuration example of a capsule guiding system according to the sixth embodiment of the present invention. As shown in FIG. 18, a capsule guiding system 91 according to the sixth embodiment includes a monitor 92 instead of the monitor 12 and a control device 94 instead of the control device 14 in the capsule guiding system 1 according to the first embodiment. Other configurations of the sixth embodiment are identical to those of the first embodiment, and like constituent elements are denoted by like reference numerals or letters.

The monitor 92 is a monitor device realized by using various displays such as a CRT display or a liquid crystal display, and displays various pieces of information instructed to be displayed by the control device 94. Specifically, the monitor 92 displays information useful for a capsule endoscope examination such as an in-vivo image group of the subject captured by the capsule endoscope 2, patient information of the subject, and examination information of the subject. The monitor 92 also displays the information useful for magnetic guidance for the capsule endoscope 2 such as the current position information and current posture information of the capsule endoscope 2 in the subject. Further, the monitor 92 displays the input amount of the operating device 5 at the time of causing the capsule endoscope 2 in the subject to perform the 6 degrees-of-freedom motion (that is, magnetically guiding the capsule endoscope 2) and the operating state of the magnetic guidance for the capsule endoscope 2 such as actions and effects of the magnetic field to be applied from the magnetic field generator 3 to the capsule endoscope 2 corresponding to the input amount.

The control device 94 controls the motion of respective components in the capsule guiding system 91 and controls input and output of a signal between the respective components. Specifically, the control device 94 calculates an input amount of the operating device 5 based on the instruction information of 6 degrees-of-freedom motion input by the operating device 5 at the time of magnetically guiding the capsule endoscope 2, and displays the calculated input amount on the monitor 92 by an arrow or numerical value. The control device 94 calculates a force and direction of the magnetic field acting on the capsule endoscope 2 in the subject (in detail, a rotating magnetic field or gradient field generated by the magnetic field generator 3) according to the input amount of the operating device 5, and displays the calculated force and direction of the magnetic field on the monitor 92 by an arrow or the like, as a consequence of the action of the magnetic field with respect to the capsule endoscope 2 at the time of magnetic guidance. The control device 94 thus displays on the monitor 92 the input amount of the operating device 5 and the operating state of the magnetic guidance for the capsule endoscope 2 exemplified in the consequence of the action of the magnetic field with respect to the capsule endoscope 2.

The control device 94 stores the current position information and current posture information of the capsule endoscope 2 sequentially acquired from the position and posture detecting device 10 in the storage unit 13, and calculates a locus of the capsule endoscope 2 in the subject based on the current position information and current posture information of the capsule endoscope 2 changing in the subject. In this case, the control device 94 corrects the shift amount and shift direction of the capsule endoscope 2 according to the shift amount and shift direction of a bed (not shown) for placing the subject thereon at the time of moving the bed in an internal space of the magnetic field generator 3 (that is, inside a space of the absolute coordinate system), and calculates the locus of the capsule endoscope 2 with reference to the bed and the subject (that is, an actual locus of the capsule endoscope 2 moved relative to the capsule endoscope 2).
The control device 94 displays the calculated locus of the capsule endoscope 2 on the monitor 92. The control device 94 also displays desired pieces of information such as the in-vivo images captured by the capsule endoscope 2, reduced images (thumbnail images) of the in-vivo images, and the status of the respective devices in the capsule guiding system on the monitor 92. Other functions of the control device 94 are the same as those of the control device 14 in the capsule guiding system 1 according to the first embodiment.

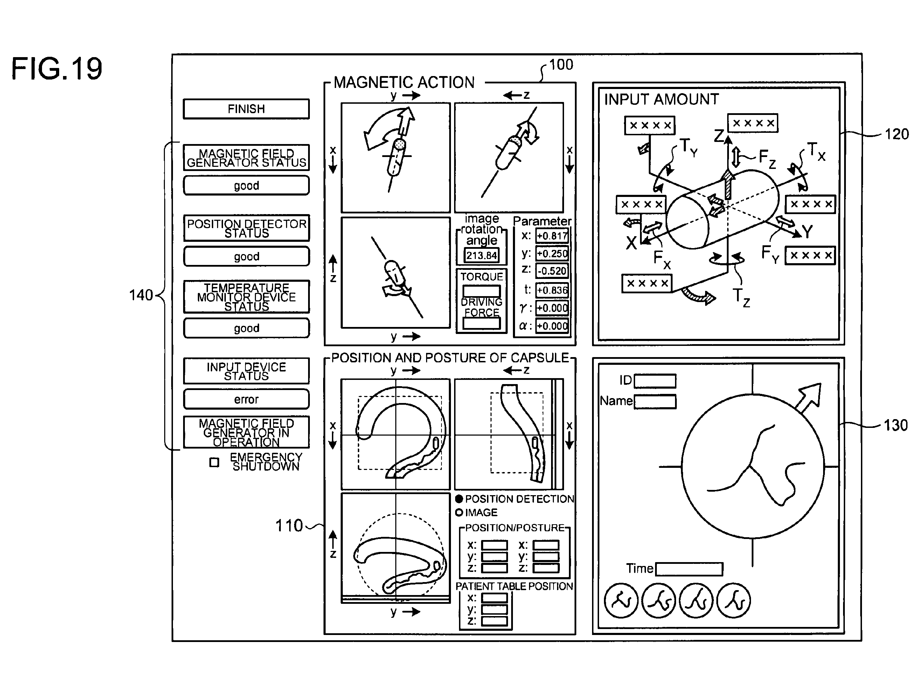

A display mode of the monitor 92 that displays various pieces of information under control of the control device 94 is explained in detail. FIG. 19 is a schematic diagram of a display mode example of the monitor 92 in the capsule guiding system according to the sixth embodiment of the present invention. The monitor 92 displays information useful for the capsule endoscope examination such as the in-vivo image group of the subject, information useful for the magnetic guidance for the capsule endoscope 2 such as the current position information and current posture information of the capsule endoscope 2 in the subject, an operating state of the magnetic guidance for the capsule endoscope 2 such as the input amount of the operating device and the consequence of the action of the magnetic field with respect to the capsule endoscope 2.

In detail, as shown in FIG. 19, the monitor 92 includes a magnetic-action display unit 100, which is a graphical user interface (GUI) that displays the consequence of the action of the magnetic field to be acted on the capsule endoscope 2 at the time of magnetically guiding the capsule endoscope 2 in the subject, a position and posture display unit 110, which is a GUI that displays the position and posture of the capsule endoscope 2 in the subject, an input-amount display unit 120, which is a GUI that displays the input amount of the operating device 5, an image display unit 130, which is a GUI that displays the in-vivo image group of the subject captured by the capsule endoscope 2, and a status display unit 140, which a GUI that displays status of the respective devices in the capsule guiding system 91.

The magnetic-action display unit 100 displays the current posture information of the capsule endoscope 2 as viewed from the respective points of view in the x-axis direction, the y-axis direction, and the z-axis direction of the absolute coordinate system, and displays a consequence of the action of the magnetic field actually acting on the capsule endoscope 2 according to the input operation of the operating device 5 (for example, acting force and acting direction of the magnetic field with respect to the capsule endoscope 2) by an arrow, numerical value, or the like. The position and posture display unit 110 displays the current position information and current posture information of the capsule endoscope 2 as viewed from the respective points of view same as the magnetic-action display unit 100 (in the x-axis direction, the y-axis direction, and the z-axis direction of the absolute coordinate system) and the locus of the capsule endoscope 2 in the subject.

The input-amount display unit 120 displays the input amount of instruction information of the 6 degrees-of-freedom motion input by the operating device 5 at the time of magnetically guiding the capsule endoscope 2 by using an arrow and a numerical value. In this case, the input-amount display unit 120 displays respective input amounts of the driving forces F_{X}, F_{Y}, and F_{Z} in the axial direction or turning forces T_{X}, T_{Y}, and T_{Z} around the axes in the capsule coordinate system, respectively, corresponding to the shape of the operating device 5 (specifically, the shape of the operating unit 30 identical to the capsule endoscope 2).

The image display unit 130 displays an imaging direction of the capsule endoscope 2, which changes due to magnetic guidance, and a changing speed in the imaging direction, together with the information useful for the capsule endoscope examination of the subject such as the in-vivo image group captured by the capsule endoscope 2 in the subject. The status display unit 140 displays the status of respective devices in the capsule guiding system 91. Specifically, the status display unit 140 displays the status of the magnetic field generator 3, "magnetic field generator status", the status of the position and posture detecting device 10, "position detector status", and the status of the operating device 5, "input device status". The status display unit 140 also displays the status of a temperature monitor (not shown in FIG. 18) that monitors the temperature of the magnetic field generator 3 in the capsule guiding system 91, "temperature monitor status". The status display unit 140 displays, for example, a result of device setting including initialization or availability of operation by a message such as "good" or "error" as the status of the respective devices.

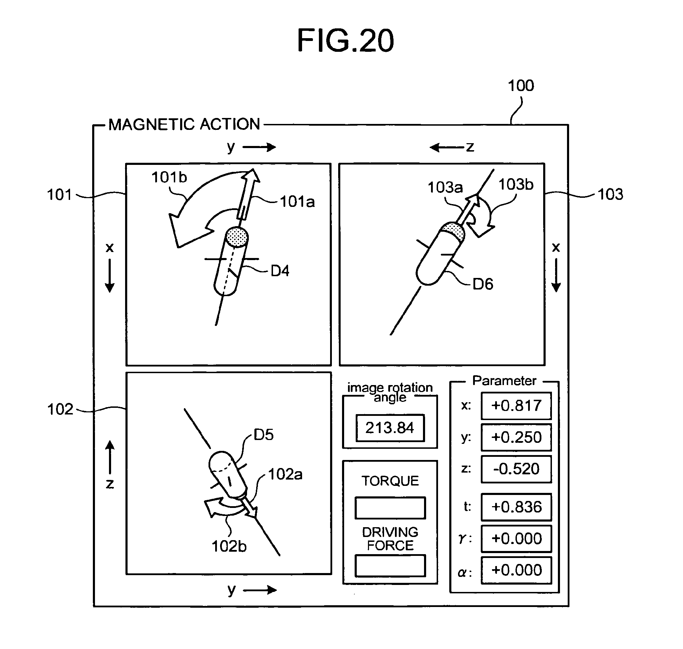

The magnetic-action display unit 100 is explained next in detail with reference to FIG. 20. FIG. 20 is a schematic diagram of a display mode example of the magnetic-action display unit 100. As shown in FIG. 20, the magnetic-action display unit 100 includes a z-point-of-view display unit 101, an x-point-of-view display unit 102, and a y-point-of-view display unit 103 for displaying the current posture information of the capsule endoscope 2 and the consequence of the action of the magnetic field with respect to the capsule endoscope 2, from the points of view in the respective axial directions of the absolute coordinate system.

The z-point-of-view display unit 101 displays the current posture information of the capsule endoscope 2 as viewed from the z-axis direction of the absolute coordinate system and the consequence of the action of the magnetic field with respect to the capsule endoscope 2. Specifically, the z-point-of-view display unit 101 displays a capsule image D4 added with three axes (X-axis, Y-axis, and Z-axis) of the capsule coordinate system, and displays the current posture information of the capsule endoscope 2 as viewed from the z-axis direction based on a three-dimensional display mode of the capsule image D4 or respective axial directions of the capsule coordinate system added to the capsule image D4. The capsule image D4 is a pattern image three-dimensionally indicating the capsule endoscope 2 as viewed from the z-axis direction of the absolute coordinate system.

The z-point-of-view display unit 101 superposes arrows 101a and 101b such as a vector indicating the consequence of the action of the magnetic field with respect to the capsule endoscope 2 on the capsule image D4 and displays a superposed image. The arrow 101a indicates a driving force acting on the capsule endoscope 2 due to the magnetic field applied to the capsule endoscope 2 by the magnetic field generator 3 according to the input amount of the operating device 5. The arrow 101b indicates a turning force such as a torque acting on the capsule endoscope 2 due to the magnetic field applied to the capsule endoscope 2 by the magnetic field generator 3 according to the input amount of the operating device 5. The z-point-of-view display unit 101 displays a direction of the acting force (the driving force or turning force) with respect to the capsule endoscope 2 by the direction of the arrows 101a and 101b, and displays a magnitude of the acting force (the driving force or turning force) with respect to the capsule endoscope 2 by the length of the arrows 101a and 101b, as the consequence of the action of the magnetic field with respect to the capsule endoscope 2 as viewed from the z-axis direction. The z-point-of-view display unit 101 displays the arrows 101a and 101b in different colors according to the type of the acting force (the driving force or turning force).

The x-point-of-view display unit 102 displays the current posture information of the capsule endoscope 2 as viewed from the x-axis direction of the absolute coordinate system and the consequence of the action of the magnetic field with respect to the capsule endoscope 2. Specifically, the x-point-of-view display unit 102 displays a capsule image D5 added with three axes (X-axis, Y-axis, and Z-axis) of the capsule coordinate system, and displays the current posture information of the capsule endoscope 2 as viewed from the x-axis direction according to a three-dimensional display mode of the capsule image D5 or respective axial directions of the capsule coordinate system added to the capsule image D5. The capsule image D5 is a pattern image three-dimensionally indicating the capsule endoscope 2 as viewed from the x-axis direction of the absolute coordinate system.

The x-point-of-view display unit 102 superposes arrows 102a and 102b such as a vector indicating the consequence of the action of the magnetic field with respect to the capsule endoscope 2 on the capsule image D5 and displays a superposed image. The arrow 102a indicates a driving force acting on the capsule endoscope 2 due to the magnetic field applied to the capsule endoscope 2 by the magnetic field generator 3 according to the input amount of the operating device 5. The arrow 102b indicates a turning force such as a torque acting on the capsule endoscope 2 due to the magnetic field applied to the capsule endoscope 2 by the magnetic field generator 3 according to the input amount of the operating device 5. The x-point-of-view display unit 102 displays a direction of the acting force (the driving force or turning force) with respect to the capsule endoscope 2 by the direction of the arrows 101a and 101b, and displays a magnitude of the acting force (the driving force or turning force) with respect to the capsule endoscope 2 by the length of the arrows 102a and 102b, as the consequence of the action of the magnetic field with respect to the capsule endoscope 2 as viewed from the x-axis direction. The x-point-of-view display unit 102 displays the arrows 102a and 102b in different colors according to the type of the acting force (the driving force or turning force).

The y-point-of-view display unit 103 displays the current posture information of the capsule endoscope 2 as viewed from the y-axis direction of the absolute coordinate system and the consequence of the action of the magnetic field with respect to the capsule endoscope 2. Specifically, the y-point-of-view display unit 103 displays a capsule image D6 added with three axes (X-axis, Y-axis, and Z-axis) of the capsule coordinate system, and displays the current posture information of the capsule endoscope 2 as viewed from the y-axis direction based on a three-dimensional display mode of the capsule image D6 or respective axial directions of the capsule coordinate system added to the capsule image D6. The capsule image D6 is a pattern image three-dimensionally indicating the capsule endoscope 2 as viewed from the y-axis direction of the absolute coordinate system.

The y-point-of-view display unit 103 superposes arrows 103a and 103b such as a vector indicating the consequence of the action of the magnetic field with respect to the capsule endoscope 2 on the capsule image D6 and displays a superposed image. The arrow 103a indicates a driving force acting on the capsule endoscope 2 due to the magnetic field applied to the capsule endoscope 2 by the magnetic field generator 3 according to the input amount of the operating device 5. The arrow 103b indicates a turning force such as a torque acting on the capsule endoscope 2 due to the magnetic field applied to the capsule endoscope 2 by the magnetic field generator 3 according to the input amount of the operating device 5. The y-point-of-view display unit 103 displays a direction of the acting force (the driving force or turning force) with respect to the capsule endoscope 2 by the direction of the arrows 101a and 101b, and displays a magnitude of the acting force (the driving force or turning force) with respect to the capsule endoscope 2 by the length of the arrows 103a and 103b, as the consequence of the action of the magnetic field with respect to the capsule endoscope 2 as viewed from the y-axis direction. The y-point-of-view display unit 103 displays the arrows 103a and 103b in different colors according to the type of the acting force (the driving force or turning force).

Although not shown in FIG. 20, the z-point-of-view display unit 101, the x-point-of-view display unit 102, and the y-point-of-view display unit 103 can additionally display information indicating a magnitude of speed of operation (hereinafter, "direction-changing speed") when the capsule endoscope 2 changes a direction (for example, an imaging direction) due to the action of the magnetic field generated by the magnetic field generator 3. That is, the z-point-of-view display unit 101 can display the capsule image D4, the arrows 101a and 101b indicating the magnitude and direction of the acting force acting on the capsule endoscope 2 by the magnetic field generator 3, and the information such as the vector or numerical value indicating the magnitude of the direction-changing speed of the capsule endoscope 2 due to the acting force, by appropriately superposing these on each other. Likewise, the x-point-of-view display unit 102 can display the capsule image D5, the arrows 102a and 102b indicating the magnitude and direction of the acting force t acting on the capsule endoscope 2 by the magnetic field generator 3, and the information such as the vector or numerical value indicating the magnitude of the direction-changing speed of the capsule endoscope 2 due to the acting force, by appropriately superposing these on each other. The y-point-of-view display unit 103 can display the capsule image D6, the arrows 103a and 103b indicating the magnitude and direction of the acting force acting on the capsule endoscope 2 by the magnetic field generator 3, and the information such as the vector or numerical value indicating the magnitude of the direction-changing speed of the capsule endoscope 2 due to the acting force, by appropriately superposing these on each other.

The z-point-of-view display unit 101, the x-point-of-view display unit 102, and the y-point-of-view display unit 103 are formed in a predetermined relative position and coordinate axis in the magnetic-action display unit 100. For example, as shown in FIG. 20, the z-point-of-view display unit 101 is formed on upper left in the magnetic-action display unit 100, the x-point-of-view display unit 102 is formed below the z-point-of-view display unit 101, and the y-point-of-view display unit 103 is formed on the right of the z-point-of-view display unit 101. In this case, the z-point-of-view display unit 101 includes a y-axis designating a rightward direction as a positive direction on an upper side, and an X-axis designating a downward direction as the positive direction on a left side. The x-point-of-view display unit 102 includes a z-axis designating an upward direction as the positive direction on the left side, and a y-axis designating the rightward direction as the positive direction on a lower side. The y-point-of-view display unit 103 includes a z-axis designating the leftward direction as the positive direction on the upper side, and an X-axis designating the downward direction as the positive direction on a right side.

On the other hand, the magnetic-action display unit 100 displays a numerical value indicating the magnitude of the driving force or turning force of the magnetic field acting on the capsule endoscope 2 according to the input amount of the operating device 5 in a predetermined area. The numerical value of the driving force is acquired by digitizing the magnitude of the driving force of the capsule endoscope 2 indicated by a length or the like of the arrows 101a to 103a. The numerical value of the turning force (torque) is acquired by digitizing the magnitude of the turning force of the capsule endoscope 2 indicated by a length or the like of the arrows 101b to 103b. In addition, the magnetic-action display unit 100 digitizes and displays an angle of rotation at the time of performing the rotary motion or direction changing motion due to the action of the magnetic force applied to the capsule endoscope 2, and displays coordinate information indicating a displacement at the time of performing the forward and backward motion or shifting motion due to the action of the magnetic force applied to the capsule endoscope 2.

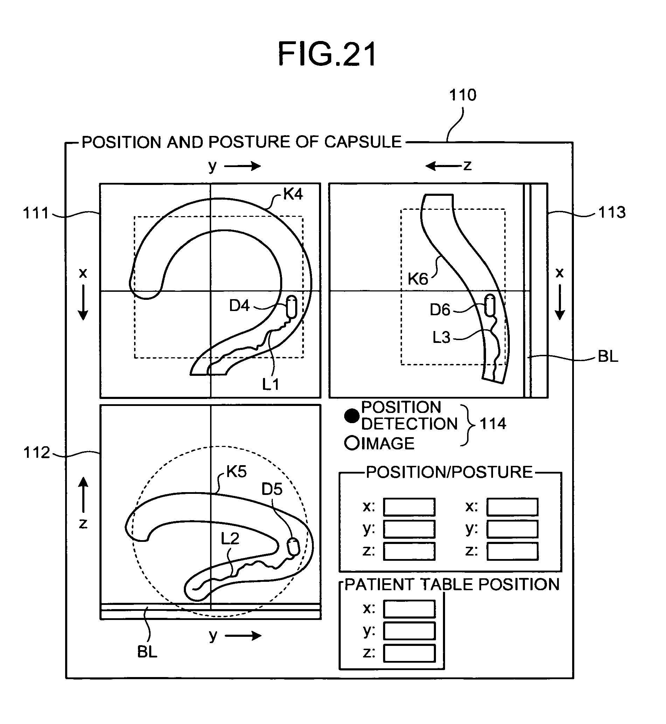

The position and posture display unit 110 is explained next in detail with reference to FIG. 21. FIG. 21 is a schematic diagram of a display mode example of the position and posture display unit 110. As shown in FIG. 21, the position and posture display unit 110 includes a z-point-of-view display unit 111, an x-point-of-view display unit 112, and a y-point-of-view display unit 113 that display the current position information and current posture information of the capsule endoscope 2 in the subject from the points of view in the respective axial directions of the absolute coordinate system.

The z-point-of-view display unit 111 displays the current position information and current posture information of the capsule endoscope 2 in the subject, as viewed from the z-axis direction of the absolute coordinate system. Specifically, the z-point-of-view display unit 111 displays a pattern image of a digestive tract (hereinafter, "digestive tract image") K4 in the subject as viewed from the z-axis direction of the absolute coordinate system, superposed on the capsule image D4. The digestive tract in the subject indicated by the digestive tract image K4 is a migration path of the capsule endoscope 2. The z-point-of-view display unit 111'changes (updates) respective display positions of the digestive tract image K4 and the capsule image D4 so that the relative position between the actual digestive tract in the subject and the capsule endoscope 2 matches the relative position between the digestive tract image K4 and the capsule image D4, and changes (updates) respective display directions of the digestive tract image K4 and the capsule image D4 so that the relative direction between the actual digestive tract in the subject and the capsule endoscope 2 matches the relative direction between the digestive tract image K4 and the capsule image D4. The z-point-of-view display unit 111 displays the relative position between the digestive tract image K4 and the capsule image D4, thereby displaying the current position information of the capsule endoscope 2 in the subject as viewed from the z-axis direction. Simultaneously, the z-point-of-view display unit 111 displays the relative direction between the digestive tract image K4 and the capsule image D4, thereby displaying the current posture information of the capsule endoscope 2 in the subject as viewed from the z-axis direction.

The z-point-of-view display unit 111 displays a locus L1 of the capsule image D4 in the digestive tract image K4, superposed on the digestive tract image K4. When a bed for placing the subject thereon is shifted in the space of the absolute coordinate system, the z-point-of-view display unit 111 shifts the digestive tract image K4, the capsule image D4, and the locus L1, following the shift of the bed. As a result, the z-point-of-view display unit 111 can display the locus of the capsule endoscope 2 with reference to the bed and the subject, that is, the actual locus of the capsule endoscope 2 that relatively moves with respect to the subject by the locus L1, regardless of the shift of the bed.

The x-point-of-view display unit 112 displays the current position information and current posture information of the capsule endoscope 2 in the subject, as viewed from the x-axis direction of the absolute coordinate system. Specifically, the x-point-of-view display unit 112 displays a digestive tract image K5 indicating the digestive tract in the subject as viewed from the x-axis direction of the absolute coordinate system, superposed on the capsule image D5. The digestive tract in the subject indicated by the digestive tract image K5 is a migration path of the capsule endoscope 2. The x-point-of-view display unit 112 changes (updates) respective display positions of the digestive tract image K5 and the capsule image D5 so that the relative position between the actual digestive tract in the subject and the capsule endoscope 2 matches the relative position between the digestive tract image K5 and the capsule image D5, and changes (updates) respective display directions of the digestive tract image K5 and the capsule image D5 so that the relative direction between the actual digestive tract in the subject and the capsule endoscope 2 matches the relative direction between the digestive tract image K5 and the capsule image D5. The x-point-of-view display unit 112 displays the relative position between the digestive tract image K5 and the capsule image D5, thereby displaying the current position information of the capsule endoscope 2 in the subject as viewed from the x-axis direction. Simultaneously, the x-point-of-view display unit 112 displays the relative direction between the digestive tract image K5 and the capsule image D5, thereby displaying the current posture information of the capsule endoscope 2 in the subject as viewed from the x-axis direction.

The x-point-of-view display unit 112 displays a locus L2 of the capsule image D5 in the digestive tract image K5, superposed on the digestive tract image K5, as well as a bed image BL indicating a bed (bed for placing the subject thereon) as viewed from the x-axis direction. When the bed is shifted in the space of the absolute coordinate system, the x-point-of-view display unit 112 shifts the digestive tract image K5, the capsule image D5, the locus L2, and the bed image BL, following the shift of the bed. As a result, the x-point-of-view display unit 112 can display the relative position between the digestive tract and the capsule endoscope 2 in the subject and the bed, and display the locus of the capsule endoscope 2 with reference to the bed and the subject, that is, the actual locus of the capsule endoscope 2 that relatively moves with respect to the subject by the locus L2, regardless of the shift of the bed.

The y-point-of-view display unit 113 displays the current position information and current posture information of the capsule endoscope 2 in the subject, as viewed from the y-axis direction of the absolute coordinate system. Specifically, the y-point-of-view display unit 113 displays a digestive tract image K6 indicating the digestive tract in the subject as viewed from the y-axis direction of the absolute coordinate system, superposed on the capsule image D6. The digestive tract in the subject indicated by the digestive tract image K6 is a migration path of the capsule endoscope 2. The y-point-of-view display unit 113 changes (updates) respective display positions of the digestive tract image K6 and the capsule image D6 so that the relative position between the actual digestive tract in the subject and the capsule endoscope 2 matches the relative position between the digestive tract image K6 and the capsule image D6, and changes (updates) respective display directions of the digestive tract image K6 and the capsule image D6 so that the relative direction between the actual digestive tract in the subject and the capsule endoscope 2 matches the relative direction between the digestive tract image K6 and the capsule image D6. The y-point-of-view display unit 113 displays the relative position between the digestive tract image K6 and the capsule image D6, thereby displaying the current position information of the capsule endoscope 2 in the subject as viewed from six axial directions. Simultaneously, the y-point-of-view display unit 113 displays the relative direction between the digestive tract image K6 and the capsule image D6, thereby displaying the current posture information of the capsule endoscope 2 in the subject as viewed from the y-axis direction.

The y-point-of-view display unit 113 displays a locus L3 of the capsule image D6 in the digestive tract image K6, superposed on the digestive tract image K6, as well as the bed image BL indicating the bed (the bed for placing the subject thereon) as viewed from the y-axis direction. When the bed is shifted in the space of the absolute coordinate system, the y-point-of-view display unit 113 shifts the digestive tract image K6, the capsule image D6, the locus L3, and the bed image BL, following the shift of the bed. As a result, the y-point-of-view display unit 113 can display the relative position between the digestive tract and the capsule endoscope 2 in the subject and the bed, and display the locus of the capsule endoscope 2 with reference to the bed and the subject, that is, the actual locus of the capsule endoscope 2 that relatively moves with respect to the subject by the locus L3, regardless of the shift of the bed.

Although not shown in FIG. 21, the z-point-of-view display unit 111, the x-point-of-view display unit 112, and the y-point-of-view display unit 113 can additionally display information indicating a magnitude of the direction-changing speed of the capsule endoscope 2 due to the action of the magnetic field by the magnetic field generator 3. That is, the z-point-of-view display unit 111 can display the information such as the vector or numerical value indicating the magnitude of the direction-changing speed of the capsule endoscope 2 due to the acting force of the magnetic field, superposing it on the capsule image D4. Likewise, the x-point-of-view display unit 112 can display the information such as the vector or numerical value indicating the magnitude of the direction-changing speed of the capsule endoscope 2 due to the acting force of the magnetic field, superposing it on the capsule image D5. Further, the y-point-of-view display unit 113 can display the information such as the vector or numerical value indicating the magnitude of the direction-changing speed of the capsule endoscope 2 due to the acting force of the magnetic field, superposing it on the capsule image D6.

The z-point-of-view display unit 111, the x-point-of-view display unit 112, and the y-point-of-view display unit 113 are formed in a predetermined relative position and coordinate axis relation in the position and posture display unit 110. Specifically, as shown in FIG. 21, the relative position and the coordinate axis relation of the z-point-of-view display unit 111, the x-point-of-view display unit 112, and the y-point-of-view display unit 113 are the same as those of the z-point-of-view display unit 101, the x-point-of-view display unit 102, and the y-point-of-view display unit 103 in the magnetic-action display unit 100 (see FIG. 20).

On the other hand, the position and posture display unit 110 includes a status display unit 114 that displays an execution state of the position and posture detecting device 10 or a status of a received signal. When the position and posture detecting device 10 is in a state capable of executing detection of the current position information and current posture information of the capsule endoscope 2, the status display unit 114 displays information indicating this state. In this case, the position and posture display unit 110 displays coordinate information indicating a current position and coordinate information indicating a current posture (direction) of the capsule endoscope 2 detected by the position and posture detecting device 10 in a predetermined display area. Further, the position and posture display unit 110 displays coordinate information indicating a current position of the bed for placing the subject thereon (also referred to as "patient table") in a predetermined display area.

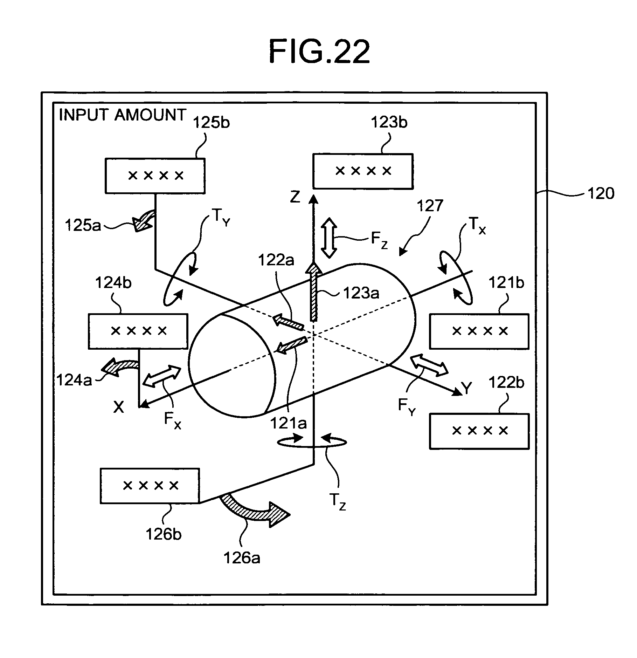

The input-amount display unit 120 is explained next in detail with reference to FIG. 22. FIG. 22 is a schematic diagram of a display mode example of the input-amount display unit 120 that displays an input amount of the operating device 5 that operates magnetic guidance for the capsule endoscope 2. As shown in FIG. 22, the input-amount display unit 120 displays an operating device image 127 schematically indicating the operating unit 30 of the operating device 5 that operates the magnetic guidance for the capsule endoscope 2 in the subject, and displays the respective axes (X-axis, Y-axis, and Z-axis) of the capsule coordinate system corresponding to the operation coordinate system defined with respect to the operating unit 30, superposed on the operating device image 127.

The input-amount display unit 120 displays an input amount of the operating device 5 corresponding to the shape of the operating unit 30 identical to the capsule endoscope 2. In this case, the input-amount display unit 120 displays an input amount of the driving force F_{X} corresponding to the force Fₐ in the a-axis direction of the operation coordinate system by an arrow 121a such as a vector parallel to the X-axis in the operating device image 127. The input-amount display unit 120 also displays the input amount of the driving force F_{X} by a numerical value in a predetermined input-amount display area 121b. The input amount of the driving force F_{X} here is an input amount of instruction information for instructing the forward and backward motion of the capsule endoscope 2. The input-amount display unit 120 displays the direction of the driving force F_{X} by the direction of the arrow 121a, and displays the magnitude of the driving force F_{X} by the length of the arrow 121a. Further, the input-amount display unit 120 displays the magnitude of the driving force Fₓ by the numerical value in the input-amount display area 121b, and displays the direction of the driving force F_{X} by a symbol (positive or negative) of the numerical value.

The input-amount display unit 120 displays an input amount of the driving force Fy corresponding to the force F_{b} in the b-axis direction of the operation coordinate system by an arrow 122a such as a vector parallel to the Y-axis in the operating device image 127. The input-amount display unit 120 also displays the input amount of the driving force F_{Y} by a numerical value in a predetermined input-amount display area 122b. The input amount of the driving force F_{Y} here is an input amount of instruction information for instructing the shifting motion of the capsule endoscope 2 in the Y-axis direction. The input-amount display unit 120 displays the direction of the driving force F_{Y} by the direction of the arrow 122a, and displays the magnitude of the driving force F_{Y} by the length of the arrow 122a. Further, the input-amount display unit 120 displays the magnitude of the driving force F_{Y} by the numerical value in the input-amount display area 122b, and displays the direction of the driving force F_{Y} by the symbol (positive or negative) of the numerical value.

Further, the input-amount display unit 120 displays an input amount of the driving force F_{Z} corresponding to the force F_{c} in the c-axis direction of the operation coordinate system by an arrow 123a such as a vector parallel to the Z-axis in the operating device image 127. The input-amount display unit 120 also displays the input amount of the driving force F_{Y} by a numerical value in a predetermined input-amount display area 123b. The input amount of the driving force F_{Z} here is an input amount of instruction information for instructing the shifting motion of the capsule endoscope 2 in the Z-axis direction. The input-amount display unit 120 displays the direction of the driving force F_{Z} by the direction of the arrow 122a, and displays the magnitude of the driving force F_{Z} by the length of the arrow 123a. Further, the input-amount display unit 120 displays the magnitude of the driving force F_{Z} by the numerical value in the input-amount display area 123b, and displays the direction of the driving force F_{Z} by the symbol (positive or negative) of the numerical value.

On the other hand, the input-amount display unit 120 displays an input amount of the turning force Tₓ corresponding to the turning force Tₐ around the a-axis of the operation coordinate system by a circular-arc arrow 124a around the X-axis in the operating device image 127. The input-amount display unit 120 displays the input amount of the turning force T_{X} by a numerical value in a predetermined input-amount display area 124b. The input amount of the turning force T_{X} is an input amount of instruction information for instructing the rotary motion around the X-axis of the capsule endoscope 2. The input-amount display unit 120 displays the direction of the turning force T_{X} by the direction of the arrow 124a, and displays the magnitude of the turning force T_{X} by the length of the arrow 124a. The input-amount display unit 120 also displays the magnitude of the turning force T_{X} by the numerical value in the input-amount display area 124b, and displays the direction of the turning force T_{X} by the symbol (positive or negative) of the numerical value.

The input-amount display unit 120 displays an input amount of the turning force T_{Y} corresponding to the turning force T_{b} around the b-axis of the operation coordinate system by a circular-arc arrow 125a around the Y-axis in the operating device image 127. The input-amount display unit 120 displays the input amount of the turning force T_{Y} by a numerical value in a predetermined input-amount display area 125b. The input amount of the turning force T_{Y} is an input amount of instruction information for instructing the direction changing motion around the Y-axis of the capsule endoscope 2. The input-amount display unit 120 displays the direction of the turning force T_{Y} by the direction of the arrow 125a, and displays the magnitude of the turning force T_{Y} by the length of the arrow 125a. The input-amount display unit 120 also displays the magnitude of the turning force T_{Y} by the numerical value in the input-amount display area 125b, and displays the direction of the turning force T_{Y} by the symbol (positive or negative) of the numerical value.

Further, the input-amount display unit 120 displays an input amount of the turning force T_{Z} corresponding to the turning force T_{c} around the c-axis of the operation coordinate system by a circular-arc arrow 126a around the Z-axis in the operating device image 127. The input-amount display unit 120 displays the input amount of the turning force T_{Z} by a numerical value in a predetermined input-amount display area 126b. The input amount of the turning force T_{Z} is an input amount of instruction information for instructing the direction changing motion around the Z-axis of the capsule endoscope 2. The input-amount display unit 120 displays the direction of the turning force T_{Z} by the direction of the arrow 126a, and displays the magnitude of the turning force T_{Z} by the length of the arrow 126a. The input-amount display unit 120 also displays the magnitude of the turning force T_{Z} by the numerical value in the input-amount display area 126b, and displays the direction of the turning force T_{Z} by the symbol (positive or negative) of the numerical value.

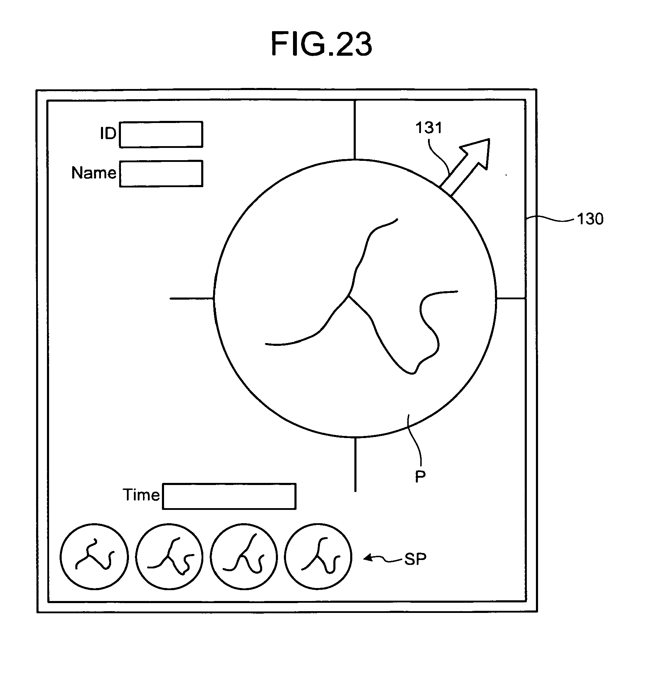

The image display unit 130 is explained next in detail with reference to FIG. 23. FIG. 23 is a schematic diagram of a display mode example of the image display unit 130 that displays an in-vivo image group of the subject captured by the capsule endoscope 2. As shown in FIG. 23, the image display unit 130 sequentially displays the in-vivo images P captured by the capsule endoscope 2 in the subject in a predetermined display area. In this case, the image display unit 130 adds a mark such as a line indicating two axes (for example, the Y-axis and Z-axis) of the capsule coordinate system. The two axes of the capsule coordinate system displayed in the display area as the mark defines respective upward, downward, left, and right directions of the in-vivo image P captured by the imaging device 23 in the capsule endoscope 2.

When the imaging direction of the imaging device 23 changes due to the 6 degrees-of-freedom motion of the capsule endoscope 2, the image display unit 130 displays a direction of change of the imaging direction by an arrow 131 such as a vector. In this case, the image display unit 130 displays the arrow 131 around the in-vivo image P with the direction of change of the imaging direction of the imaging device 23 (for example, a direction of rotation of an optical axis of the imaging device 23) matched with the direction of the arrow 131. The arrow 131 is vector information indicating a change direction (a direction-changing direction) and the magnitude of the direction-changing speed at the time of changing the imaging direction of the imaging device 23, that is, the direction of the capsule endoscope 2 (specifically, a long axis direction of the capsule casing) due to the acting force of the magnetic field. The image display unit 130 can additionally display numerical value information indicating the magnitude of the direction-changing speed of the capsule endoscope 2.

Further, the image display unit 130 displays thumbnail images SP, which are reduced images of a desired in-vivo image selected from the sequentially displayed in-vivo image group in a predetermined display area. Specifically, the input unit 11 inputs information for selecting the desired in-vivo image from the in-vivo image group sequentially displayed by the image display unit 130 to the control device 94. The control device 94 extracts the in-vivo image selected from the in-vivo image group based on the input information from the input unit 11, and stores the extracted in-vivo image in the storage unit 13. The control device 94 additionally displays thumbnail images corresponding to the selected in-vivo image on the monitor 92. The image display unit 130 additionally displays the thumbnail images SP, as shown in FIG. 23, under control of the control device 94. When the input unit 11 inputs information such as a comment with respect to the thumbnail images SP, the control device 94 additionally displays the information such as the comment on the monitor 92. The image display unit 130 adds the information such as the comment to the thumbnail images SP and displays the thumbnail images SP under control of the control device 94.

The image display unit 130 displays patient information of the subject (such as patient ID and patient name) and various pieces of information useful for the capsule endoscope examination such as information indicating an imaging time of the in-vivo image P currently displayed, together with the in-vivo image P.

As explained above, the monitor 92 having the magnetic-action display unit 100, the position and posture display unit 110, the input-amount display unit 120, and the image display unit 130 displays the operating state of the magnetic guidance for the capsule endoscope 2 such as the input amount of the operating device 5 at the time of magnetically guiding the capsule endoscope 2 in the subject, the magnitude and direction of the acting force of the magnetic field acting on the capsule endoscope 2 according to the input amount, and the locus of the capsule endoscope 2 in the subject.

On the other hand, in a conventional capsule guiding system which does not have the operating device 5 having a shape similar to the capsule endoscope 2, the magnetic guidance for the capsule endoscope 2 is operated by using a well-known input device such as a joystick or foot pedal, while visually checking the current position information of the capsule endoscope 2 in the subject or the in-vivo image of the subject displayed on the monitor. However, in the conventional capsule guiding system, the magnetic guidance for the capsule endoscope 2 needs to be operated under a situation where the operating state of the magnetic guidance, that is, how much the magnetic field is acting on the capsule endoscope 2 to be magnetically guided by the operation of the input device, or whether the magnetic field to be acted on the capsule endoscope 2 at the time of magnetic guidance is an appropriate level, cannot be ascertained. Accordingly, it is difficult to operate the capsule endoscope 2 in the digestive tract with desired 6 degrees-of-freedom motion. As a result, not only a load on the subject at the time of magnetically guiding the capsule endoscope 2 increases, but also it becomes difficult to magnetically guide the capsule endoscope 2 in the subject smoothly along the digestive tract.

On the other hand, in the capsule guiding system 91 according to the sixth embodiment of the present invention, at the time of operating the magnetic guidance for the capsule endoscope 2, the monitor 92 displays the operating state of the magnetic guidance for the capsule endoscope 2. Specifically, on the monitor 92, the input amount of the operating device 5 at the time of operating the magnetic guidance is displayed by the input-amount display unit 120, the consequence of the action of the magnetic field with respect to the capsule endoscope 2 according to the input amount of the operating device 5 is displayed by the magnetic-action display unit 100, the current position information, the current posture information, and the locus of the capsule endoscope 2 are displayed by the position and posture display unit 110, and the change direction of the imaging direction is displayed together with the in-vivo images by the image display unit 130.

By performing the magnetic guidance operation while visually checking the operating state of the magnetic guidance for the capsule endoscope 2 displayed on the monitor 92, the magnetic guidance for the capsule endoscope 2 can be operated while ascertaining the input amount of the operating device 5 at the time of the magnetic guidance operation and the consequence of the action of the magnetic field to be acted on the capsule endoscope 2 according to the input amount. As a result, the desired 6 degrees-of-freedom motion can be performed by the capsule endoscope 2 in the digestive tract by causing the magnetic field of an appropriate magnitude and direction to act on the capsule endoscope 2 in the subject, thereby enabling to reduce the load on the subject at the time of the magnetic guidance for the capsule endoscope 2 and magnetically guide the capsule endoscope 2 in the subject smoothly along the digestive tract.

Even when the magnetic guidance for the capsule endoscope 2 is operated by using the well-known input device such as the joystick or foot pedal instead of the operating device 5, actions and effects can be acquired similarly by displaying the input amount of the input device and the consequence of the action of the magnetic field to be acted on the capsule endoscope 2 according to the input amount on the monitor. However, by using the operating device 5 including the operating unit 30 having a shape identical to the capsule endoscope 2, the capsule endoscope 2 in the subject can be easily operated with desired 6 degrees-of-freedom motion more intuitively and the capsule endoscope 2 in the subject can be magnetically guided more easily.

As described above, in the sixth embodiment, the input amount of the operating device that operates the magnetic guidance for the capsule endoscope in the subject and the consequence of the action of the magnetic field to be acted on the capsule endoscope in the subject according to the input amount are displayed on the monitor. Other parts of the configuration are substantially the same as those of the fourth embodiment. Accordingly, the magnitude and direction of the acting force of the magnetic field to be acted on the capsule endoscope according to the input amount of the operating device can be easily ascertained, and the magnetic guidance for the capsule endoscope can be operated in a state with the magnitude and direction of the acting force being ascertained. As a result, the same operations and effects as those of the first embodiment can be acquired, the capsule endoscope in the digestive tract can be easily operated with desired 6 degrees-of-freedom motion, while ascertaining the operating state of the magnetic guidance for the capsule endoscope, and the capsule endoscope in the subject can be magnetically guided smoothly along the digestive tract.

The magnetic field of an appropriate magnitude and direction can be acted on the capsule endoscope in the subject while ascertaining the input amount of the operating device at the time of the magnetic guidance operation and actions and effects of the magnetic field to be acted on the capsule endoscope according to the input amount. As a result, an unnecessary input amount by the operating device and an excessive action of the magnetic field with respect to the capsule endoscope can be suppressed, and the load on the subject at the time of operating the capsule endoscope 2 in the digestive tract with desired 6 degrees-of-freedom motion can be reduced.

Further, in the sixth embodiment, the current position information and current posture information (capsule direction) of the capsule endoscope in the subject detected by the position and posture detecting device are displayed, using A three-dimensional graphic image of the capsule endoscope, and the information such as the arrow or numerical value indicating the magnitude and direction of the acting force (driving force, turning force, and the like) of the magnetic field with respect to the capsule endoscope and the information indicating the direction change amount of the capsule endoscope are superposed on the graphic image and displayed. Accordingly, a position detection result and a magnetic-guidance operating direction of the capsule endoscope can be confirmed simultaneously, and the magnitude and direction of the driving force and the turning force to be acted on the capsule endoscope can be intuitively recognized. As a result, an operator can acquire the operating state of the magnetic guidance for the capsule endoscope more smoothly.

A coordinate axis involved with a display by the magnetic-action display unit that displays the consequence of the action of the magnetic field to be acted on the capsule endoscope together with the current posture information of the capsule endoscope is matched with a coordinate axis involved with a display by the position and posture display unit that simultaneously displays the current position information and the current posture information of the capsule endoscope in the subject. Accordingly, even if the operator moves a line of sight between the magnetic-action display unit and the position and posture display unit, the position and posture of the capsule endoscope in the subject can be recognized intuitively.

Further, a pattern image of the bed for placing the subject thereon (also referred to as "patient table") is displayed together with the digestive tract image of the subject and the capsule image, and the pattern image of the bed, the digestive tract image, and the capsule image are simultaneously shifted, matched with an actual motion of the bed. Therefore, the locus of the capsule endoscope in the digestive tract can be displayed, added with a shift amount and shift direction of the bed. As a result, an actual locus of the capsule endoscope, which has moved relative to the subject, can be displayed regardless of the movement of the bed. In this way, by displaying the locus of the capsule endoscope, the position of the capsule endoscope in a patient's body can be displayed with high accuracy, even if the bed is moved.

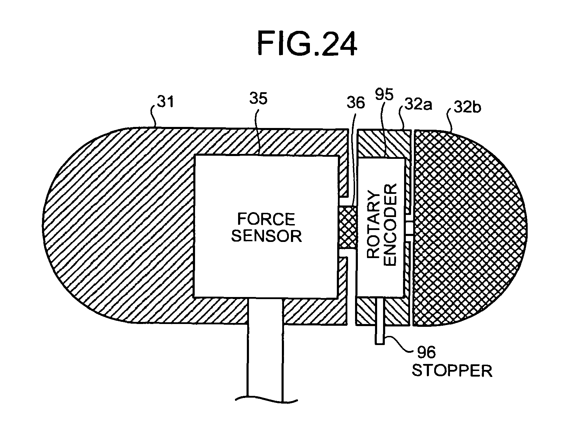

In the first embodiment of the present invention, the turning force Tₐ around the a-axis of the movable unit 32 is detected by the force sensor 35. However, the amount of rotation around the a-axis of the movable unit 32 can be detected by a rotary encoder. Specifically, as shown in FIG. 24, the movable unit 32 is divided into a movable unit 32a and a turning unit 32b, a rotary encoder 95 is incorporated in the movable unit 32a, and the rotary encoder 95 and a shaft 36 of the force sensor 35 are connected with each other, and a shaft of the rotary encoder 95 and the turning unit 32b are connected with each other. The rotary encoder 95 detects a direction of rotation and an amount of rotation (that is, a direction of rotation and an amount of rotation around the a-axis) of the turning unit 32b. A detection result of the rotary encoder 95 needs only to be input to the control device 14 as instruction information for instructing the rotary motion of the capsule endoscope around the X-axis.

Further, a stopper 96 that temporarily stops the rotation of the rotary encoder 95 associated with the rotary motion of the turning unit 32b can be further provided in the operating unit. In this case, the rotary encoder 95 can continuously input the current amount of rotation around the a-axis as the instruction information for instructing the rotary motion of the capsule endoscope around the X-axis, in a state with the rotation being stopped by the stopper 96. Accordingly, the rotary motion around the X-axis of the capsule endoscope in the subject can be continued.

Further, in the first to sixth embodiments of the present invention, the driving force of the forward and backward motion of the capsule endoscope 2 is generated by the gradient field. However, the present invention is not limited thereto, and a spiral protrusion, which forms a spiral shape around a longitudinal axis of a capsule medical device such as a capsule endoscope (the X-axis of the capsule coordinate system) can be provided on an outer wall surface of a cylindrical casing of the capsule medical device, to generate the driving force of the forward and backward motion in the longitudinal axis direction (X-axis direction), by rotating the capsule medical device around the longitudinal axis by the rotating magnetic field.

In the first to fifth embodiments of the present invention, the current position information and current posture information of the capsule endoscope 2 as viewed from three axial directions of the capsule coordinate system defined with respect to the capsule endoscope 2 or three axial directions of the absolute coordinate system defined with respect to the magnetic field generator 3 are displayed on the monitor. However, the present invention is not limited thereto, and the current position information and current posture information of the capsule endoscope 2 as viewed from at least one axial direction of the three axial directions of the capsule coordinate system or the absolute coordinate system (for example, the Z-axis direction of the capsule coordinate system or the z-axis direction of the absolute coordinate system) needs only to be displayed on the monitor.

Further, in the fourth and fifth embodiments of the present invention, only one hold button for holding the operating amount of the operating unit is provided in the operating unit. However, the present invention is not limited thereto, and a plurality of hold buttons can be provided in the operating unit for each operating direction (such as a driving direction or rotation direction) and for each acting force (the driving force and the turning force) of the operating unit by one operation or continuous operations of the 6 degrees-of-freedom motion, so that an operating amount of the operating unit can be held for each operating direction and for each acting force.

In the second to fifth embodiments of the present invention, only one enable button for enabling or disabling a detecting process of respective physical values of the 6 degrees-of-freedom motion is provided in the operating unit. However, the present invention is not limited thereto, and a plurality of enable buttons can be provided in the operating unit for each operating direction (such as a driving direction or rotation direction) and for each acting force (the driving force and the turning force) of the operating unit by one operation or continuous operations of the 6 degrees-of-freedom motion, so that the detecting process of respective physical values can be enabled or disabled for each operating direction and for each acting force.

Further, in the second and third embodiments of the present invention, the amount of rotation and the direction of rotation around the respective axes of the 6 degrees-of-freedom motion are detected by the rotary encoder. However, the present invention is not limited thereto, and the amount of rotation and the direction of rotation around the respective axes of the 6 degrees-of-freedom motion can be detected by a potentiometer instead of the rotary encoder.

In the first to sixth embodiments of the present invention, an induction field is generated from the capsule endoscope 2 due to the action of the magnetic field applied to the capsule endoscope 2, and the current position and current posture of the capsule endoscope 2 in the subject are detected by detecting the induction field. However, the present invention is not limited thereto, and an echo signal from the capsule endoscope 2 can be detected by transmitting or receiving sound waves (desirably, ultrasonic sound waves) to/from the capsule endoscope 2 in the subject, to detect the current position and current posture of the capsule endoscope 2 in the subject based on the detected echo signal. Alternatively, the current position and current posture of the capsule endoscope 2 in the subject can be detected based on X-ray image data of the subject.

Further, in the first to sixth embodiments of the present invention, the capsule guiding system that magnetically guides the capsule endoscope 2 that captures the in-vivo images of the subject is shown. However, the present invention is not limited thereto, and the capsule medical device in the capsule guiding system according to the present invention can be a capsule pH-measuring apparatus that measures pH in a living body, a capsule drug-administration apparatus including a function of spraying or injecting a drug into the living body, or a capsule sampling equipment that samples a substance in the living body, so long as magnetic guidance is possible by applying the rotating magnetic field or gradient field.

In the first to sixth embodiments of the present invention, image information such as the in-vivo images captured by the capsule endoscope 2 in the subject 1 is displayed on the monitor as an example of information acquired by the capsule medical device inserted into the subject. However, the present invention is not limited thereto, and the in-vivo information to be displayed on the monitor according to the present invention needs only to be the information acquired by the capsule medical device in the subject. For example, the in-vivo information can be pH information or temperature information of the living body measured by the capsule medical device, or information of the subject in the body such as a body tissue sampled by the capsule medical device.

Further, in the first to sixth embodiments of the present invention, the operating device including the operating unit (casing) capable of performing the 6 degrees-of-freedom motion by one operation or continuous operations is exemplified and explained. However, the present invention is not limited thereto, and the operating device is not limited thereto, and an operating device that includes an operating unit capable of inputting at least 3 degrees-of-freedom motion by one operation or continuous operations (that is, an operating unit capable of operating with 3 or more degrees-of-freedom motion) and having an axial relation similar to motion axes of the capsule medical device can acquire the same operations and effects as those of at least one of the first to sixth embodiments.

A permanent magnet magnetized in a direction orthogonal to the longitudinal axis of the capsule medical device can be installed in the capsule medical device, and the rotating magnetic field is generated around the permanent magnet to rotate the capsule medical device around the longitudinal axis together with the permanent magnet. As a result, the longitudinal axis direction of the capsule medical device can be maintained in a direction perpendicular to the rotating magnetic field. In this case, the operating device according to the present invention can input 3 degrees-of-freedom position (freedom in respective axial directions of the X-axis, Y-axis, and Z-axis) of the capsule medical device and two degrees of freedom excluding a rotational degree of freedom around the longitudinal axis of the capsule medical device (freedom around the Y-axis and Z-axis), and can guide the capsule medical device. In this case, further, the rotating magnetic field can be automatically generated, and an input amount in a rotation direction around the longitudinal axis of the operating unit can be detected by a sensor that detects rotating torque, to control rotation speed of the rotating magnetic field according to the input amount.

Further, in the sixth embodiment of the present invention, the magnitude of the acting force (driving force or turning force) of the magnetic field to be acted on the capsule endoscope 2 is displayed by the length of the arrow such as the vector. However, the present invention is not limited thereto, and the magnitude of the acting force of the magnetic field with respect to the capsule endoscope 2 can be displayed by a change of color of the arrow. In this case, the color of the arrow displayed on the monitor can be changed according to the driving force, based on a ratio between a migration speed of the capsule endoscope 2 in the subject and the driving force acting on the capsule endoscope. For example, the color of the arrow indicating the driving force can be changed to red as a value obtained by dividing the migration speed of the capsule endoscope 2 by the driving force decreases compared with a predetermined threshold, or the color of the arrow indicating the driving force can be changed to blue as the value increases compared with the predetermined threshold. Information relating to friction between the capsule endoscope 2 and a wall of internal organs in the digestive tract can be displayed by the color change of the arrow.

In the sixth embodiment of the present invention, the digestive tract image and the capsule image are superposed on each other to display the current position information of the capsule endoscope in the subject. However, the present invention is not limited thereto, and a pattern image of the subject (for example, the subject images K1 to K3 in the first embodiment) can be displayed instead of the digestive tract image and the current position information of the capsule endoscope can be displayed by superposing the pattern image of the subject on the capsule image, or the current position information of the capsule endoscope can be displayed by superposing an image obtained by combining the digestive tract image and the pattern image of the subject on the capsule image.

### INDUSTRIAL APPLICABILITY

As explained above, the operating device, the monitor device, and the capsule guiding system according to the present invention are useful for guiding a capsule medical device inserted into a subject, and particularly suitable for an operating device, a monitor device, and a capsule guiding system that can easily operate magnetic guidance for guiding a capsule medical device in a subject by a magnetic field.

## Claims

1. An operating device that uses a magnetic field generator with respect to a capsule medical device inserted into a subject to for operating the capsule medical device with at least 3 degrees-of-freedom motion, the operating device comprising:
a casing having directionality; and
a detecting unit that detects respective physical values of at least 3 degrees-of-freedom motion of an entirety or a part of the casing, wherein
one operation or continuous operations to the entirety or a part of the casing provides the at least 3 degrees-of-freedom motion to the capsule medical device.

2. The operating device according to claim 1, wherein the casing has a three-dimensional shape having the directionality.

3. The operating device according to claim 2, wherein the casing has a three-dimensional shape substantially identical to the capsule medical device and is a holdable size.

4. The operating device according to claim 1, wherein the casing comprises an axis display unit that indicates a specific axial direction of the capsule medical device.

5. The operating device according to claim 1, wherein the casing comprises:
a movable unit that receives one operation or continuous operations of the at least 3 degrees-of-freedom motion; and
a fixed unit that supports the movable unit so that at least 3 degrees-of-freedom motion can be performed, wherein
the detecting unit comprises a force sensor that is encapsulated in the detecting unit and detects respective pieces of force information of the at least 3 degrees-of-freedom motion of the movable unit.

6. The operating device according to claim 5, wherein the detecting unit further comprises a rotation-amount detecting device that detects an amount of rotation around an axis of the at least 3 degrees-of-freedom motion of the movable unit.

7. The operating device according to claim 1, further comprising a supporting unit that supports the casing so that at least 3 degrees-of-freedom motion can be performed, wherein
the detecting unit comprises
a plurality of rotation-amount detecting devices that detect respective amounts of rotation around three axes orthogonal to each other in the at least 3 degrees-of-freedom motion of the casing; and
a plurality of displacement-amount detecting devices that detect respective displacement amounts in three axial directions orthogonal to each other in the at least 3 degrees-of-freedom motion of the casing.

8. The operating device according to claim 1, further comprising a supporting unit that supports the casing so that at least 3 degrees-of-freedom motion can be performed, wherein
the detecting unit comprises
a plurality of rotation-amount detecting devices that detect respective amounts of rotation around three axes orthogonal to each other in the at least 3 degrees-of-freedom motion of the casing; and
a force sensor that is encapsulated in the supporting unit, and detect respective pieces of force information in three axial directions orthogonal to each other in the at least 3 degrees-of-freedom motion of the casing.

9. The operating device according to claim 1, further comprising:
a magnetic-field generating stage that generates a magnetic field in a space where one operation or continuous operations with respect to an entire casing is performed; and
a plurality of sense coils that are fixedly arranged inside the casing, and detect the magnetic field generated by the magnetic-field generating stage, wherein
the detecting unit detects respective operating amounts of the at least 3 degrees-of-freedom motion of the casing based on a magnetic field detection result of the sense coils.

10. The operating device according to claim 1, further comprising:
an acceleration sensor that is arranged in the casing and detects an acceleration of the casing generated by one operation or continuous operations with respect to the entire casing, wherein
the detecting unit detects respective operating amounts of the at least 3 degrees-of-freedom motion of the casing based on an acceleration detection result of the acceleration sensor.

11. The operating device according to claim 10, further comprising:
a transmitting unit that wirelessly transmits the acceleration detection result of the acceleration sensor to outside of the casing; and
a receiving unit that receives the acceleration detection result of the acceleration sensor wirelessly transmitted from the transmitting unit, wherein
the detecting device detects the respective operating amounts of the at least 3 degrees-of-freedom motion of the casing based on the acceleration detection result of the acceleration sensor acquired via the receiving unit.

12. The operating device according to claim 7, further comprising a switching unit for enabling or disabling a detecting process performed by the detecting unit for detecting respective physical values of the at least 3 degrees-of-freedom motion of the casing.

13. The operating device according to claim 9, further comprising an input unit that inputs instruction information for instructing to hold a detection result of the detecting unit, wherein
the detecting unit holds the respective physical values of the at least 3 degrees-of-freedom motion of the casing based on the instruction information input by the input unit.

14. An operating device that uses a magnetic field generator with respect to a capsule medical device inserted into a subject to operate the capsule medical device, the operating device comprising:
a casing having an axis display unit that indicates a specific axial direction of the capsule medical device; and
a detecting unit that detects each physical value of at least 3 degrees-of-freedom motion provided for the casing, wherein
directions of the respective physical values detected by the detecting unit match respective axial directions of a coordinate system set with respect to any one of the capsule medical device, the magnetic field generator, or a bed for placing the subject thereon.

15. The operating device according to claim 14, wherein the casing has a three-dimensional shape having directionality.

16. The operating device according to claim 15, wherein the casing is substantially identical to the capsule medical device.

17. The operating device according to claim 14, wherein the specific axial direction is a longitudinal axis direction of the capsule medical device.

18. The operating device according to claim 14, wherein the specific axial direction is an imaging direction of the capsule medical device.

19. The operating device according to claim 1, wherein the at least 3 degrees-of-freedom motion is 6 degrees-of-freedom motion.

20. A capsule guiding system comprising:
a capsule medical device inserted into a subject;
a magnetic field generator that guides the capsule medical device by applying a magnetic field to the capsule medical device;
an operating device by which an operator inputs a physical value; and
a control device that controls the magnetic field generator according to the physical value, wherein
the operating device comprises
a casing held by the operator to input at least 3 degrees-of-freedom physical value; and
a detecting unit that detects the at least 3 degrees-of-freedom physical value input to the casing by the operator.

21. The capsule guiding system according to claim 20, wherein the control device controls the magnetic field generator based on the physical value so that a posture of the capsule medical device is changed.

22. The capsule guiding system according to claim 20, wherein the control device controls the magnetic field generator based on the physical value so that a position of the capsule medical device is changed.

23. The capsule guiding system according to claim 20, wherein the physical value indicates an amount of change in a position or posture of the casing with respect to the operating device, and
the detecting unit is a change-amount detecting device that detects the amount of change.

24. The capsule guiding system according to claim 23, wherein
the operating device comprises the casing, which is not connected with the operating device, and
the change-amount detecting device detects a position or posture of the casing with respect to the operating device.

25. The capsule guiding system according to claim 24, wherein
the change-amount detecting device that detects the amount of change is provided in the casing,
the casing comprises a transmitting unit that wirelessly transmits the amount of change to the operating device, and
the operating device comprises a receiving unit that receives the amount of change transmitted by the transmitting unit.

26. The capsule guiding system according to claim 23, wherein the operating device comprises the casing connected to the operating device only by a flexible cable,
the cable electrically couples the operating device and the casing with each other, and
the change-amount detecting device detects a position or posture of the casing.

27. The capsule guiding system according to claim 23, wherein the change-amount detecting unit comprises:
a magnetic field generator for detecting the amount of change, provided in the casing and generates the magnetic field for detecting a position or posture in the operating device; and
a plurality of magnetic-field detection sensors that are provided outside of the casing and detect the magnetic field for detecting the position or posture, and wherein the change-amount detecting unit detects the position or posture of the casing based on the magnetic field detected by the magnetic-field detection sensors.

28. The capsule guiding system according to claim 23, wherein the change-amount detecting unit comprises an acceleration sensor provided in the casing, and
the change-amount detecting unit detects the amount of change based on the acceleration detected by the acceleration sensor.

29. The capsule guiding system according to claim 23, wherein the operating device comprises a switching unit that switches whether the control device controls the magnetic field generator based on the amount of change of the casing.

30. The capsule guiding system according to claim 29, wherein the switching unit switches whether to perform specific degree-of-freedom control.

31. The capsule guiding system according to claim 20, wherein
the physical value indicates a force loaded on the casing, and
the detecting unit is a force sensor that detects the loaded force.

32. The capsule guiding system according to claim 20, wherein the detecting unit detects 6 degrees-of-freedom physical value input by an operator, and
the control device controls the magnetic field generator based on the detected physical value so that 3 degrees-of-freedom position and 3 degrees-of-freedom posture of the capsule medical device are controlled.

33. The capsule guiding system according to claim 20, wherein the detecting unit detects 5 degrees-of-freedom physical value input by an operator, and
the control device controls the magnetic field generator based on the detected physical value so that 3 degrees-of-freedom position of the capsule medical device and 2 degrees-of-freedom posture of the capsule medical device excluding a rotation around a longitudinal axis of the capsule medical device are controlled.

34. The capsule guiding system according to claim 20, wherein the control device associates a coordinate system of the casing with a coordinate system of the capsule medical device, and controls the magnetic field generator based on the physical value input to the casing to control a position or posture of the capsule medical device.

35. The capsule guiding system according to claim 34, wherein the casing is a three-dimensional shape having directionality.

36. The capsule guiding system according to claim 35, wherein the three-dimensional shape is substantially identical to the capsule medical device.

37. The capsule guiding system according to claim 35, wherein the three-dimensional shape has a display unit that displays a direction matched with a specific direction of the capsule medical device on the three-dimensional shape.

38. The capsule guiding system according to claim 20, wherein the control device associates a coordinate system of the operating device with a coordinate system of the magnetic field generator, and controls the magnetic field generator based on the physical value input to the casing to control a position or posture of the capsule medical device.

39. The capsule guiding system according to claim 38, comprising a movable unit that changes the position or posture of the casing.

40. The capsule guiding system according to claim 39, wherein the operating device comprises:
a driving unit that drives the movable unit; and
a position and posture detecting unit that detects a position or posture of the capsule medical device, and
the driving unit controls a position or posture of the casing.

41. The capsule guiding system according to claim 40, wherein a drive-control switching unit that switches whether the operating unit controls the position or posture of the casing is provided in the operating unit.

42. The capsule guiding system according to claim 39, wherein a holding unit that holds a position of the movable unit is provided in the operating unit.

43. The capsule guiding system according to claim 38, wherein the casing is a three-dimensional shape having directionality.

44. A capsule guiding system that magnetically guides a capsule medical device inserted into a subject, comprising:
the operating device according to any one of claims 1 to 19;
a magnetic field generator that generates a magnetic field with respect to the capsule medical device; and
a control device that generates the magnetic field for causing the capsule medical device to perform desired at least 3 degrees-of-freedom motion, based on respective physical values of at least 3 degrees-of-freedom motion input by the operating device.

45. The capsule guiding system according to claim 44, further comprising a monitor device that displays a current position of the capsule medical device in the subject.

46. The capsule guiding system according to claim 45, wherein the monitor device displays the current position of the capsule medical device with reference to a three-axis rectangular coordinate system defined with respect to the capsule medical device.

47. The capsule guiding system according to claim 46, wherein the monitor device displays an image of the subject superposed on an image of the capsule medical device, and updates the image of the subject while fixing the image of the capsule medical device.

48. The capsule guiding system according to claim 45, wherein the monitor device further displays a predicted posture taken by the capsule medical device that performs at least 3 degrees-of-freedom motion after a predetermined time.

49. The capsule guiding system according to claim 48, wherein the monitor device provides a vector display of a force generated in the capsule medical device that performs at least 3 degrees-of-freedom motion.

50. The capsule guiding system according to claim 45, wherein the monitor device displays the current position of the capsule medical device with reference to a three-axis rectangular coordinate system defined with respect to the magnetic field generator.

51. The capsule guiding system according to claim 45, wherein the monitor device further displays the current posture of the capsule medical device in the subject.

52. The capsule guiding system according to claim 45, further comprising a position and posture detecting device that detects a current position and a current posture of the capsule medical device in the subject, wherein
the control device displays the current position and the current posture of the capsule medical device detected by the position and posture detecting device on the monitor device.

53. The capsule guiding system according to claim 47, wherein the monitor device displays an image of the capsule medical device on a substantially central part of a display screen.

54. The capsule guiding system according to claim 47, wherein the monitor device displays the image of the subject added with an image of a digestive tract in which the capsule medical device moves.

55. The capsule guiding system according to claim 49, wherein
the force generated in the capsule medical device is a driving force and a turning force of the capsule medical device, and
the monitor device provides a vector display of the driving force and the turning force of the capsule medical device.

56. The capsule guiding system according to claim 48, wherein the monitor device displays the predicted posture, which is prediction information of a rotational position of the capsule medical device, which changes when the capsule medical device performs a rotary motion.

57. The capsule guiding system according to claim 56, wherein the capsule medical device comprises a magnet that follows a magnetic field generated by the magnetic field generator to contribute to a motion of the capsule medical device, and
the monitor device displays a polar direction of the magnet as the prediction information of the rotational position of the capsule medical device.

58. The capsule guiding system according to claim 50, wherein the monitor device displays an image of the subject in a state with a relative direction with respect to a display screen being fixed, and displays an image of the capsule medical device so that a display position of the image of the capsule medical device in the displayed image of the subject match a current position of the capsule medical device.

59. The capsule guiding system according to claim 50, wherein the monitor device displays an image of the subject added with an image of a digestive tract in which the capsule medical device moves, and displays a current position of the capsule medical device in the digestive tract.

60. The capsule guiding system according to claim 45, wherein the monitor device displays the current posture of the capsule medical device in the subject under the control of the control device.

61. The capsule guiding system according to claim 51, wherein the monitor device displays a current posture of the capsule medical device in the subject with reference to a three-axis rectangular coordinate system defined with respect to the magnetic field generator.

62. A monitor device in a capsule guiding system that guides a capsule medical device inserted into a subject by a magnetic field generated by a magnetic field generator, comprising:
a position and posture display unit that displays a current position and a current posture in the subject of the capsule medical device guided by the magnetic field generated by the magnetic field generator; and
a magnetic-action display unit that displays a magnitude and a direction of an acting force acting on the capsule medical device due to the magnetic field generated by the magnetic field generator, and a magnitude of a direction-changing speed of the capsule medical device.

63. The monitor device according to claim 62, wherein the magnetic-action display unit displays the magnitude and the direction of the acting force and the magnitude of the direction-changing speed superposed on a capsule image indicating the current posture of the capsule medical device in the subject.

64. The monitor device according to claim 62, wherein the magnetic-action display unit provides a vector display of the magnitude and the direction of the acting force and the magnitude of the direction-changing speed.

65. The monitor device according to claim 62, further comprising an input-amount display unit that displays an input amount of an operating device in the capsule guiding system that guides the capsule medical device by the magnetic field generated by the magnetic field generator.

66. The monitor device according to claim 65, wherein the input-amount display unit displays the input amount of the operating device according to a shape of the operating device identical to the capsule medical device.

67. The monitor device according to claim 62, further comprising an acquisition-information display unit that displays acquisition information acquired by the capsule medical device.

68. The monitor device according to claim 67, wherein the acquisition-information display unit is an image display unit that displays an in-vivo image of the subject captured by the capsule medical device.

69. The monitor device according to claim 68, wherein the image display unit displays the magnitude of the direction-changing speed and a change direction when the capsule medical device changes a direction, superposed on the in-vivo image of the subject.
